# FASCICULE DE BREVET EUROPEEN

(11) **EP 0 498 722 B1**
(45) Date de publication et mention de la délivrance du brevet: **30.07.1997**
(21) Numéro de dépôt: 92400295.9
(22) Date de dépôt: 05.02.1992
(51) Int. Cl.: C07D 215/12, A61K 31/395, C07D 215/14, C07D 215/233, C07D 237/32, C07D 239/88, C07D 241/38, C07D 237/28, C07D 215/36

(54) **Dérivés bicycliques azotés, leur procédé de préparation, les intermédiaires obtenus, leur application comme médicaments et les compositions pharmaceutiques les renfermant**
Bizyklische Stickstoffverbindungen, ihre Herstellung, erhaltene Zwischenprodukte, ihre Verwendung als Arzneimittel und diese enthaltende pharmazeutische Zusammensetzungen
Bicyclic nitrogen compounds, their preparation, intermediates obtained, their use as pharmaceuticals and pharmaceutical compositions containing them

(30) Priorité: 07.02.1991 FR 9101373; 20.08.1991 FR 9110434
(43) Date de publication de la demande: 12.08.1992
(73) Titulaire: ROUSSEL UCLAF, 93230 Romainville (FR)
(72) Inventeur: Clemence, François, F-75009 Paris (FR); Fortin, Michel, F-75018 Paris (FR); Haesslein, Jean-Luc, F-77181 Courtry (FR)
(74) Mandataire: Vieillefosse, Jean-Claude

(56) Documents cités:
- FR-A- 2 377 400
- GB-A- 1 419 788
- GB-A- 2 143 814
- US-A- 3 936 461
- US-A- 4 956 371
- CHEMICAL ABSTRACTS, vol. 90, no. 23, 1979, page 669, abrégé no. 186992m, Columbus, Ohio, US; & ES-A-453 829 (LABORATORIO FORTUNY S.A.) 01-01-1979
- CHEMICAL ABSTRACTS, vol. 55, no. 10, mai 1961, abrégé no. 9333h, Columbus, Ohio, US; H. MITSUBISHI et al.: "Constituents of Umbelliferae plants. II. Isolation of the active principles of ligusticum root", & CHEM. PHARM. BULL. (TOKYO) 8, 243-5(1960)
- CHEMICAL ABSTRACTS, vol. 85, 1976, page 466, abrégé no. 45850y, Columbus, Ohio, US; S.I. YAKIMOVICH et al.: "Tautomerism in a series of nitrogen derivatives of ethyl alpha-ethoxalylcarboxylates", & ZH. ORG. KHIM. 1976, 12(5), 949-56

## Description

La présente invention concerne de nouveaux dérivés bicycliques azotés substitués par un radical benzyle, leur procédé de préparation, les nouveaux intermédiaires obtenus, leur application à titre de médicaments et les compositions pharmaceutiques les renfermant.
Les brevets US A 3936461 et US A 4956371 décrivent respectivement des dérivés 4-benzylquinolines et 4-benzylisoquinolines utiles dans le traitement de maladies cardiovasculaires.

La présente invention a pour objet les produits de formule (I_{B}) : 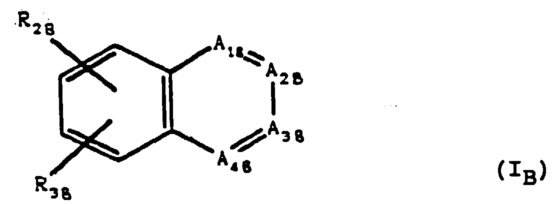 dans laquelle :
R_{2B} et R_{3B}, identiques ou différents, sont choisis dans le groupe formé par :
   - l'atome d'hydrogène,
   - les atomes d'halogène,
   - le radical hydroxyle,
   - le radical mercapto, cyano, nitro, formyle, benzoyle, acyle ayant au plus 6 atomes de carbone,
   - les radicaux carboxy libre, salifié ou estérifié par un radical alkyle linéaire ou ramifié renfermant au plus 4 atomes de carbone,
   - les radicaux alkyle, alkényl, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole, isoxazole, pyrrolidinyle, pyrrolidinylcarbonyle et phényle éventuellement substitué par un ou plusieurs radicaux choisis parmi les atomes d'halogène, le radical hydroxyle, les radicaux alkyle et alkoxy renfermant au plus 4 atomes de carbone,
   - les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pyrrolidinylcarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{1B}, A_{2B}, A_{3B} et A_{4B} sont tels que :
   - l'un représente un atome d'azote,
   - un autre représente un atome d'azote ou le radical méthine substitué par R_{4b},
   - un autre représente le radical méthine substitué par R_{4c},
   - et le dernier représente le radical méthine substitué par le radical benzyle lui-même substitué par un ou plusieurs radicaux choisis parmi les radicaux cyano, carboxy libre, salifié et estérifié,
R_{4b} et R_{4c} identiques ou différents, sont choisis parmi les valeurs de R_{4B},
   tel que R_{4B} représente :
   - l'atome d'hydrogène, le radical hydroxyle, cyano, carboxy libre, salifié ou estérifié par un radical alkyle renfermant au plus 4 atomes de carbone,
   - le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

Dans les produits de formule (I_{B}) et dans ce qui suit :
- le terme atome d'halogène désigne de préférence l'atome de chlore, mais peut aussi représenter un atome de fluor, de brome ou d'iode,
- le terme radical acyle désigne de préférence un radical ayant au plus 7 atomes de carbone tel que le radical acétyle, propionyle, butyryle ou benzoyle, mais peut également représenter un radical valéryle, hexanoyle, acryloyle, crotonoyle ou carbamoyle : on peut également citer le radical formyle ;
- le terme acyloxy désigne les radicaux dans lesquels les radicaux acyle ont la signification indiquée ci-dessus et par exemple les radicaux acétoxy ou propionyloxy,
- le terme carboxy estérifié désigne de préférence un groupe alkyloxy inférieur carbonyle tel que méthoxycarbonyle, éthoxycarbonyle, tert-butoxycarbonyle ou un groupe benzyloxycarbonyle,
- le terme cycloalkyle désigne de préférence un radical cyclopropyle, cyclopentyle ou cyclohexyle mais également cyclobutyle,
- le terme radical alkyle linéaire ou ramifié désigne de préférence les radicaux méthyle, éthyle, n-propyle, isopropyle, n-butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement butèn-1-yl, ou pentényle,
- le terme radical alkynyle linéaire ou ramifié désigne de préférence un radical éthynyle, propargyle, butynyle ou pentynyle,
- le terme radical alkyloxy linéaire ou ramifié désigne de préférence les radicaux méthoxy ou éthoxy, mais peut aussi représenter un radical propoxy, isopropoxy, butoxy linéaire, secondaire ou tertiaire,
- le terme radical alkylthio linéaire ou ramifié désigne les radicaux dans lesquels le radical alkyle peut représenter, par exemple, les valeurs indiquées ci-dessus pour le radical alkyle ; le radical alkylthio représente de préférence les radicaux méthylthio ou éthylthio, mais peut aussi représenter un radical propylthio, isopropylthio, n-butylthio, sec-butylthio, tert-butylthio, isopentylthio ou isohexylthio,

Les radicaux amino que peuvent représenter l'un ou plusieurs des éventuels substituants des radicaux définis dans les produits de formule (I) et dans ce qui suit et que peuvent représenter en particulier les radicaux : 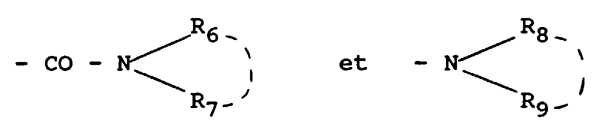 désignent des radicaux dans lesquels à l'atome d'azote sont liés deux radicaux, identiques ou différents, choisis parmi l'atome d'hydrogène ; les radicaux alkyle tels que définis ci-dessus pour donner de préférence les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, trifluorométhyle, pentafluoroéthyle, hydroxyméthyle, hydroxyéthyle, méthoxyméthyle, méthoxyéthyle, éthoxyéthyle ; les radicaux alkényle tels que définis ci-dessus et représentés de préférence par les radicaux vinyle et allyle ; les radicaux aryle ou arylalkyle tels que définis ci-dessus, carbocycliques ou hétérocycliques et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle.

Lorsque R₆ et R₇ d'une part ou R₈ et R₉ d'autre part forment ensemble avec l'atome d'azote auquel ils sont liés un hétérocycle, il s'agit, par exemple, d'un cycle pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués par les substituants déjà mentionnés précédemment et en particulier par un ou plusieurs radicaux choisis parmi les atomes de chlore et de fluor, les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle.

Les radicaux acyle que peuvent représenter R₈ et R₉ sont tels que définis précédemment et peuvent être choisis par exemple parmi les radicaux acétyle, propionyle, butyryle, valéryle ou carbamoyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcanemonosulfoniques tels que par exemple l'acide méthanesulfonique, l'acide éthanesulfonique, l'acide propanesulfonique, les acides alcanedisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Le ou les radicaux carboxy des produits de formule (I) peuvent être salifiés par des bases minérales telles que, par exemple, un équivalent de sodium, de potassium, de lithium, le calcium, le magnésium ou l'ammonium ou des bases organiques telles que, par exemple, la méthylamine, la propylamine, la triméthylamine, la diéthylamine, la triéthylamine, la N,N-diméthyléthanolamine, le tris(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Les radicaux alkyle et alkényle tels que définis ci-dessus ainsi que les radicaux alkyle ou alkényle des radicaux alkylthio, phenylalkyle et phenylalkényle tels que définis ci-dessus, peuvent ne pas être substitués ou porter un ou plusieurs substituants choisis, par exemple, dans le groupe formé par les atomes d'halogène, tel que chloro ou bromo, comme dans, par exemple, le groupe 2-bromoéthyle ; les radicaux hydroxyle ; phényle ; phénylalkyle ; cycloalkyle, par exemple cyclopropyle, cyclopentyle ou cyclohexyle ; cycloalkényle tel que par exemple le radical cyclohexényle peuvent être éventuellement substitués, parmi lesquels on peut citer le diméthyl-1,3 cyclohexène ; alkyloxy, tel que défini ci-dessus par exemple méthoxy, éthoxy, n-propoxy ou iso-propoxy comme dans par exemple les groupes méthoxyméthyle ou 1-éthoxyéthyle ; alkyloxy substitué tel que (trihaloalkyl) oxy comme, par exemple, trifluorométhoxy ; phénoxy ; benzyloxy ; mercapto ; alkylthio, par exemple méthylthio ou éthylthio ; alkylthio substitué tel que trihaloalkylthio comme, par exemple, trifluorométhylthio ; phenylthio , phenylalkylthio ; amino comme dans, par exemple, le groupe 2-aminoéthyle ; amino substitué par un ou deux radicaux choisis par exemple parmi les radicaux alkyle, alkényle, phenyle et phenylalkyle tels que définis ci-dessus comme par exemple monoalkylamino dans, par exemple, méthylamino ou éthylamino, comme par exemple dialkylamino dans, par exemple, diméthylamino ; nitro ; cyano ; azido ; carboxy ; carboxy estérifié, par exemple méthoxycarbonyle ou éthoxycarbonyle ; formyle ; acyle, par exemple acétyle, propionyle ou benzoyle ; acyle substitué par exemple par un radical amino tel que défini ci-dessus ou par un radical cyclique lié au radical acyle par un atome d'azote, ce radical cyclique pouvant renfermer éventuellement un ou plusieurs hétéroatomes choisis parmi les atomes d'azote, d'oxygène ou de soufre et tel que défini ci-dessus ; acyloxy, par exemple acétoxy ou propionyloxy ; carbamoyle ; carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle ; phtalimido ; acylamido, par exemple acétamido ou benzamido ; alcoxycarbonylaminc, par exemple méthoxycarbonylamino ou éthoxycarbonylamino ; ou (phenylalkyl) oxycarbonylamino, par exemple benzyloxycarbonylamino.

Les radicaux phényle et alkyloxy tels que définis ci-dessus et les radicaux phenyles des radicaux phenylalkyle et phenylalkényle tels que définis ci-dessus, peuvent ne pas être substitués ou porter un ou plusieurs substituants choisis, par exemple, dans la liste indiquée ci-dessus pour les éventuels substituants des radicaux alkyle et alkényle tels que définis ci-dessus,
comme par exemple pour donner le radical o-chloro-phényle mais peuvent également être substitués par un ou plusieurs radicaux choisis dans le groupe formé par les radicaux alkyle, tel que alkyle inférieur, par exemple méthyle, éthyle, ou également isopropyle ou tert-butyle ; alkényle ; alkyle substitué tel que par exemple trihaloalkyle comme dans trifluorométhyle ; alkényle tel que, par exemple, vinyle ou allyle ; alkynyle tel que, par exemple, propargyle.

Parmi les substituants que peuvent comporter les radicaux alkyle, alkényle, alkynyle, alkyloxy, alkylthio, phenyle, phenylalkyle et phenylalkényle tels que définis ci-dessus, peuvent être cités plus particulièrement les atomes d'halogène, tels que chloro et bromo ; les radicaux hydroxyle ; acyle tel que, par exemple, acétyle, propionyle, butyryle, valéryle, hexanoyle, acryloyle, crotonoyle ou carbamoyle ; benzoyle ; carboxy estérifié désignant de préférence un groupe alkyloxy inférieur carbonyle tel que méthoxycarbonyle, éthoxycarbonyle ou benzyloxycarbonyle ; alkyle tel que méthyle ou éthyle ; amino ; amino substitué, tel que monoalkyl- et dialkylamino, par exemple méthylamino, éthylamino ou diméthylamino ; alkyloxy, par exemple méthoxy, éthoxy ou isopropoxy ; aryle tel que phényle biphényle, naphtyle, indényle, indolyle ou indolinyle ; phenylalkyle tels que, par exemple benzyle ou phénéthyle ; radicaux alkyle, alkyloxy et phenyle tels que définis ci-dessus pouvant eux-mêmes être substitués par un ou plusieurs radicaux, identiques ou différents, choisis, par exemple, dans le groupe formé par les radicaux hydroxy, alkyle et alkyloxy linéaire ou ramifié, par exemple méthyle, éthyle, tert-butyle, méthoxy, éthoxy, isopropoxy ; amino substitué, tel que monoalkyl- et dialkylamino, par exemple méthylamino, éthylamino ou diméthylamino ; les radicaux monocycliques carbocycliques ou hétérocycliques renfermant 6 chaînons tels que les radicaux phényle, pyrannyle, pyridyle, pyrimidinyle, pyridazinyle, pyrazinyle, pipéridyle, pipérazinyle, pipéridino et morpholino ; les radicaux monocycliques carbocycliques ou hétérocycliques renfermant 5 chaînons, tel que par exemple le radical furyle, pyrrolyle, pyrrolinyle, imidazolyle ou pyrazolyle, isothiazolyle, isoxazolyle, pyrrolidinyle, imidazolidinyle, pyrazolidinyle ; les radicaux constitués de cycles condensés carhocycliques ou hétérocycliques parmi lesquels par exemple les radicaux naphtyle, indolyle, quinolyle ou purinyle ainsi que leurs isomères de position du ou des hétéroatomes par exemple d'azote tels que par exemple le radical indazolyle ou isoquinolyle.

Quand de tels radicaux hétérocycliques renferment un ou plusieurs atomes d'azote, ce ou ces atomes d'azote peuvent ne pas être substitués ou l'un ou plusieurs de ces atomes d'azote peuvent être substitués, par exemple, par un radical alkyle ou alkyloxy linéaire ou ramifié renfermant de 1 à 5 atomes de carbone, tels que définis ci-dessus, par exemple méthyle, éthyle, isopropyle, tert-butyle, méthoxy ou éthoxy, un radical phényle ou benzyle, ces radicaux pouvant eux-même être substitués par les substituants déjà mentionnés ci-dessus pour les radicaux phenyle et phenylalkyle : on peut citer, comme exemples, les radicaux méthylpipérazinyle, éthylpipérazinyle, propylpipérazinyle, phénylpipérazinyle ou benzylpipérazinyle.

Parmi les valeurs particulièrement préférées de tels radicaux, on peut citer particulièrement les radicaux phényle, naphtyle, pyridyle, pipérazinyle, pyrimidinyle, pyridazinyle et pyrazinyle.

De tels produits de formule (I_{B}) représentent ainsi en particulier des dérivés :
- de la quinoline dans lesquels l'un de A₁ et A₄ représente un atome d'azote,
- de l'isoquinoline dans lesquels l'un de A₂ et A₃ représente un atome d'azote,
   et des dérivés :
- de la cinnoline dans lesquels A₁ et A₂ ou A₃ et A₄ représentent tous deux un atome d'azote,
- de la quinoxaline dans lesquels A₁ et A₄ représentent tous deux un atome d'azote,
- de la quinazoline dans lesquels A₁ et A₃ ou A₂ et A₄ représentent tous deux un atome d'azote.

L'invention a plus particulièrement pour objet les produits de formule (I_{B}) telle que définie ci-dessus et répondant à la formule (Iₐ) : 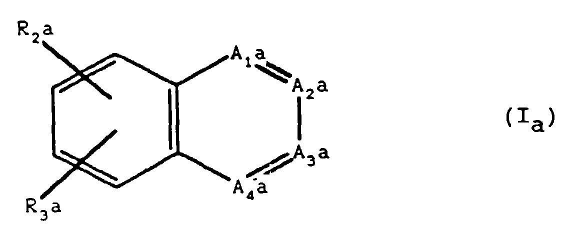 dans laquelle :
R₂ₐ et R₃ₐ, identiques ou différents, sont choisis dans le groupe formé par :
   - l'atome d'hydrogène,
   - les atomes d'halogène,
   - le radical hydroxyle,
   - le radical mercapto, cyano, nitro, formyle, benzoyle, acyle ayant au plus 6 atomes de carbone,
   - les radicaux carboxy libre, salifié ou estérifié par un radical alkyle linéaire ou ramifié renfermant au plus 4 atomes de carbone,
   - les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle les radicaux alkyloxy renfermant au plus 4 atomes de carbone, trifluorométhyle, cyano, acyle, carboxy libre, salifié ou estérifié, tétrazole et isoxazole,
   - les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A₁ₐ, A₂ₐ, A₃ₐ et A₄ₐ sont tels que :
   - l'un représente un atome d'azote,
   - un autre représente un atome d'azote ou le radical méthine substitué par R_{4ab},
   - un autre représente le radical méthine substitué par R_{4ac},
   - et le dernier représente le radical méthine substitué par le radical benzyle lui-même substitué par un ou plusieurs radicaux choisis parmi les radicaux cyano, carboxy libre, salifié et estérifié
R_{4ab} et R_{4ac} identiques ou différents, sont choisis parmi les valeurs de R₄ₐ,
   tel que R₄ₐ représente :
   - l'atome d'hydrogène, le radical hydroxyle, cyano, carboxy libre, salifié ou estérifié par un radical alkyle renfermant au plus 4 atomes de carbone,
   - le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ).

Parmi les produits objet de l'invention, peuvent être cités tout particulièrement les produits de formule (I_{B}) telle que définie ci-dessus, dans laquelle :
R_{2B} et R_{3B} représentent un atome d'hydrogène,
A_{1B}, A_{2B} et A_{3B} sont tels que l'un ou deux d'entre eux représentent un atome d'azote, et les autres, identiques ou différents, représentent le radical 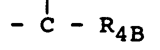 tel que R_{4B} est choisi parmi l'atome d'hydrogène, le radical n-butyle et alkylthio renfermant au plus 4 atomes de carbone,
et A_{4B} représente le radical méthine substitué par le radical benzyle éventuellement substitué par un ou plusieurs radicaux choisis parmi les radicaux cyano, carboxy libre, salifié et estérifié,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

L'invention a aussi pour objet un procédé de préparation de produits de formule (I_{B}) telle que définie ci-dessus, caractérisé en ce que :
a) soit l'on soumet le composé de formule (III) : 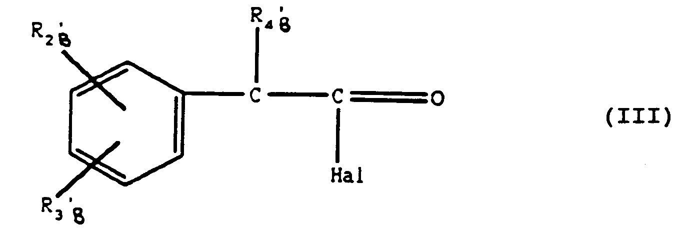 dans laquelle R_{2B}', R_{3B}' et R_{4B}' ont les significations indiquées ci-dessus, respectivement pour R_{2B}, R_{3B} et R_{4B} dans lesquelles les éventuelles fonctions réactives sont éventuellement protégées par des groupements protecteurs
   et Hal représente un atome d'halogène,
   à une réaction de substitution pour obtenir le produit de formule (IV) : 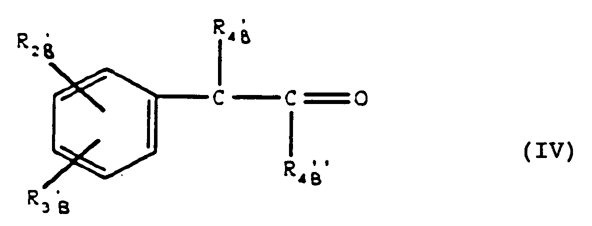 dans laquelle R_{2B}', R_{3B}' et R_{4B}' ont les significations indiquées ci-dessus et R_{4B}'', identique ou différent de R_{4B}', a la signification indiquée ci-dessus, pour R_{4B} dans laquelle les éventuelles fonctions réactives sont éventuellement protégées par des groupements protecteurs,
   que l'on fait réagir :
   avec un composé de formule (V) :

   R_{4B}'''-C≡N (V)

   dans laquelle R_{4B}''', identique ou différent de R_{4B}' ou R_{4B}'', a la signification indiquée ci-dessus, pour R_{4B} dans laquelle les éventuelles fonctions réactives sont éventuellement protégées par des groupements protecteurs, pour obtenir après cyclisation un produit de formule (Iᵢ) : 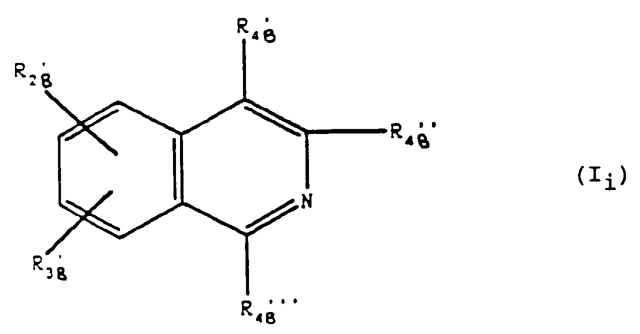 dans laquelle R_{2B}', R_{3B}', R_{4B}', R_{4B}'' et R_{4B}''' ont les significations indiquées ci-dessus,
b) soit l'on fait réagir un produit de formule (VI) : 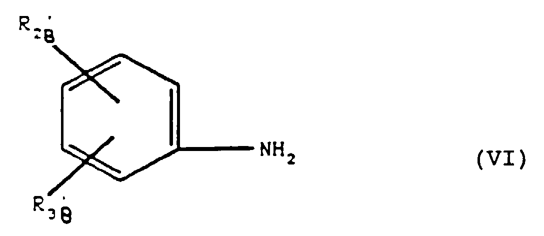 dans laquelle R_{2B}' et R_{3B}' ont les significations indiquées ci-dessus, avec un produit de formule (VII) :

   R_{4B}'-CH(CO₂alk)-CO-R_{4B}'' (VII)

   dans laquelle R_{4B}' et R_{4B}'', identiques ou différents, ont les significations indiquées ci-dessus, et alk représente un radical alkyle renfermant au plus 6 atomes de carbone, pour obtenir des produits de formule (VIII) : 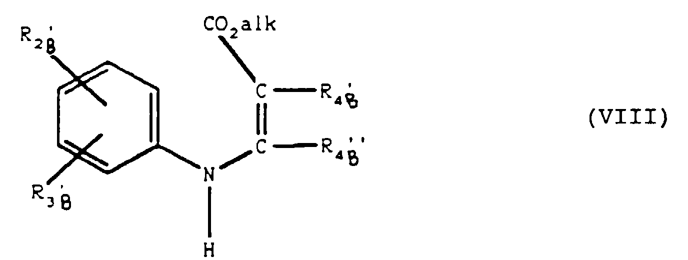 dans laquelle R_{2B}', R_{3B}', R_{4B}', R_{4B}'' et alk ont les significations indiquées ci-dessus, que l'on cyclise pour obtenir des produits de formule (II_{b}) : 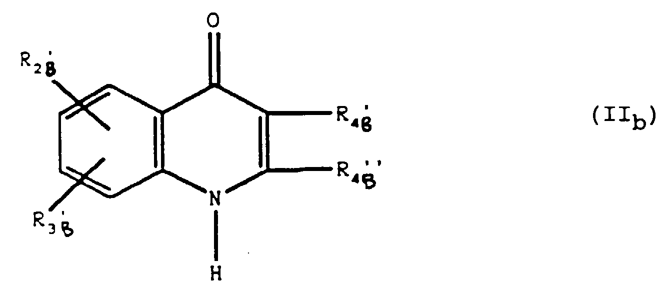 dans laquelle R_{2B}', R_{3B}', R_{4B}' et R_{4B}'' ont les significations indiquées ci-dessus,
c) soit l'on fait réagir un produit de formule (IX) : 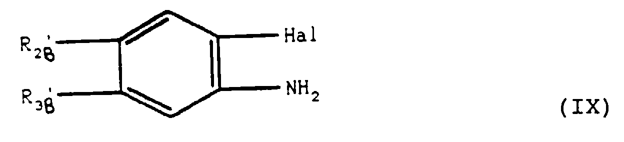 dans laquelle R_{2B}' et R_{3B}' ont les significations indiquées ci-dessus, avec un produit de formule (X) :

   R_{4B}'''-C≡CH (X)

   dans laquelle R_{4B}''' a la signification indiquée ci-dessus, pour obtenir des produits de formule (XI) : 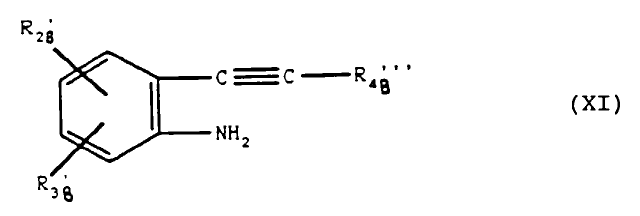 dans laquelle R_{2B}', R_{3B}' et R_{4B}''' ont les significations indiquées ci-dessus, que l'on soumet à une réaction de cyclisation en présence d'un donneur d'azote tel que le nitrite de sodium, pour obtenir un produit de formule (II_{c}) : 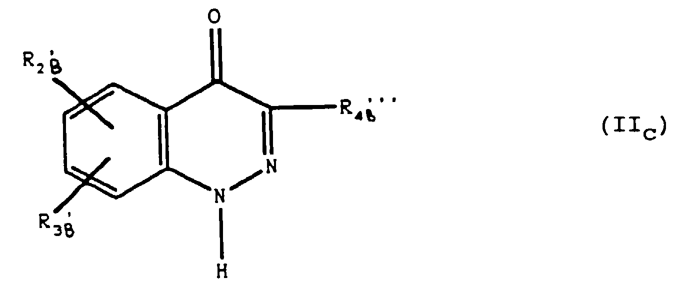 dans laquelle R_{2B}', R_{3B}' et R_{4B}''' ont les significations indiquées ci-dessus,
d) soit l'on fait réagir un produit de formule (XII) : 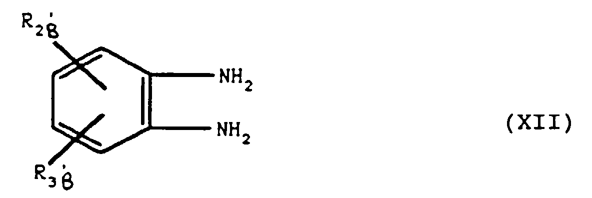 dans laquelle R_{2B}' et R_{3B}' ont les significations indiquées ci-dessus, avec un produit de formule (XIII) :

   R_{4B}'-CO-CO₂alk (XIII)

   dans laquelle R_{4B}' a la signification indiquée ci-dessus, et alk représente un radical alkyle renfermant au plus 6 atomes de carbone, pour obtenir après cyclisation des produits de formule (II_{d}) : 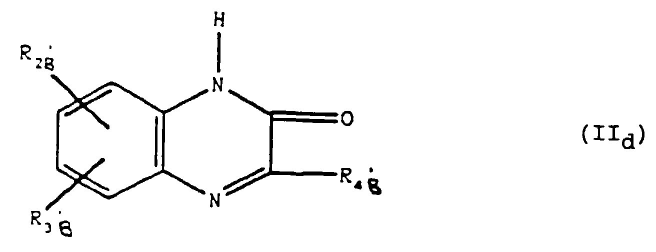 dans laquelle R_{2B}', R_{3B}' et R_{4B}' ont les significations indiquées ci-dessus,
e) soit l'on soumet un produit de formule (XIV) : 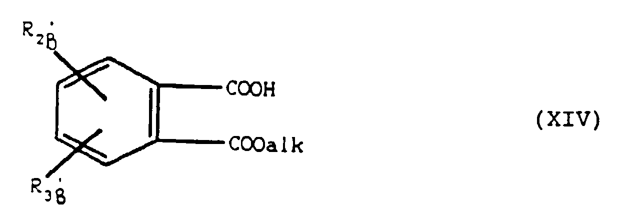 dans laquelle R_{2B}', R_{3B}' et alk ont les significations indiquées ci-dessus, après, si désiré, une réaction d'halogénation de la fonction carboxy libre, à une réaction d'addition sur cette fonction carboxy d'un composé de formule R_{4B}', R_{4B}' ayant la signification indiquée ci-dessus, pour obtenir après cyclisation en présence d'hydrazine ou d'un dérivé des produits de formule (IIₑ) : 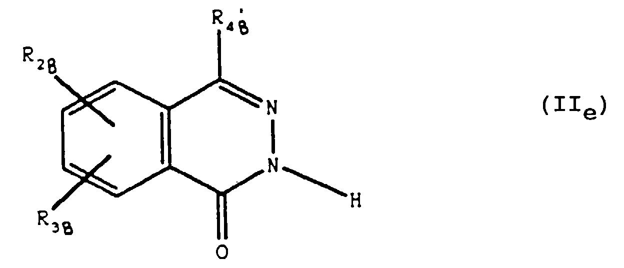 dans laquelle R_{2B}', R_{3B}' et R_{4B}' ont les significations indiquées ci-dessus,
f) soit l'on fait réagir un produit de formule (XV) : 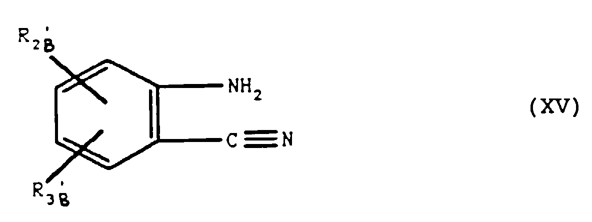 dans laquelle R_{2B}' et R_{3B}' ont les significations indiquées ci-dessus, avec un produit de formule (XVI) :

   R_{4B}'-CO-Cl (XVI)

   dans laquelle R_{4B}' a la signification indiquée ci-dessus, pour donner le produit de formule (XVII) : 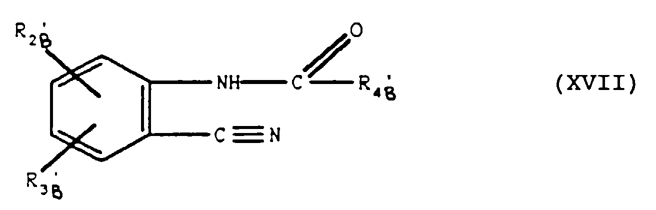 dans laquelle R_{2B}', R_{3B}' et R_{4B}' ont les significations indiquées précédemment, pour obtenir, après cyclisation, des produits de formule (II_{f}) : 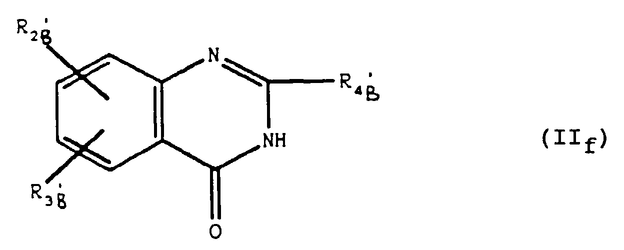 dans laquelle R_{2B}', R_{3B}' et R_{4B}' ont les significations indiquées ci-dessus,
produits de formule (Iᵢ) qui peuvent représenter des produits de formule (I_{B}) et produits de formules (II_{b}), (II_{c}), (II_{d}), (IIₑ) et (II_{f}) telles que définies ci-dessus qui peuvent représenter des produits de formule (I_{B}) dans laquelle l'un au moins de A_{1B}, A_{2B}, A_{3B} et A_{4B} représente un radical méthine portant un radical hydroxyle, que l'on soumet, si désiré et si nécessaire, à l'une ou plusieurs des réactions suivantes, dans un ordre quelconque :
- une réaction de réduction complète du radical hydroxyle ou oxo en radical méthine suivie d'une aromatisation,
- sur les produits de formule (Iᵢ) dans lesquels l'un de R_{4B}', R_{4B}'' ou R_{4B}''' représente un radical hydroxyle ou sur les produits de formules (II_{b}), (II_{c}), (II_{d}), (IIₑ) et (II_{f}),
   **soit** d'abord à une réaction de substitution du radical hydroxyle par un atome d'halogène suivie de l'action d'un produit de formule Rₚ₄ - M - Hal dans laquelle Rₚ₄ a la signification indiquée ci-dessus, pour R₄ dans lesquelles les éventuelles fonctions réactives sont éventuellement protégées par des groupements protecteurs, M représente un atome de métal choisi parmi le magnésium, le cuivre et le zinc et Hal représente un atome d'halogène,
   **soit** à l'action d'un produit de formule Rₚ₄ - Hal dans laquelle Hal représente un atome d'halogène,
   pour obtenir les produits de formule (I_{B}) correspondants,
- une réaction de transformation d'une fonction oxo (= O) en fonction thioxo (= S),
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification d'une fonction acide,
- une réaction de saponification d'une fonction ester en fonction acide,
- une réaction de transformation d'une fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques, lesdits produits de formule (I_{B}) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :
- la réaction de substitution sur le dérivé halogéné de formule (III) pour obtenir le produit de formule (IV) peut être réalisée selon les méthodes usuelles connues de l'homme de métier telles que par exemple par réaction d'un organométallique tel que par exemple un organozincique de formule Zn-Br-R_{4B}'' sur le chlorure d'acide de formule (III) ou encore, par exemple, en particulier lorsque R_{4B}'' représente un radical butyle, par réaction d'un dérivé de l'étain tel que le composé de formule (R_{4B}'')₄Sₙ de préférence en présence de palladium dans un solvant tel que par exemple l'éther ou le tétrahydrofuranne,
- la réaction d'addition du composé de formule (V) sur le composé de formule (IV) ainsi obtenu peut être réalisée selon les méthodes usuelles connues de l'homme de métier telles que par exemple dans du trichlorure de phosphoryle en présence d'un acide de Lewis et la réaction de cyclisation donnant le produit de formule (Iᵢ) se produit in situ,
- la réaction d'addition du composé de formule (VII) sur le composé de formule (VI) peut être réalisée selon les méthodes usuelles connues de l'homme de métier telles que, par exemple, en présence d'un agent dessicant tel qu'un tamis moléculaire ou encore d'un acide tel que, par exemple, l'acide paratoluène sulfonique,
- la réaction de cyclisation du composé de formule (VIII) en composé de formule (II_{b}) peut être réalisée par exemple dans un solvant tel que le DOWTHERM ® ou encore en absence de solvant en portant le composé à son point de fusion,
- la réaction d'addition du composé de formule (X) sur le composé de formule (IX) pour obtenir le composé de formule (XI) peut être réalisée, par exemple, en présence de sel cuivreux de préférence en présence d'un catalyseur tel que par exemple un catalyseur au palladium dans un solvant basique tel que par exemple la triéthylamine ou la diéthylisopropylamine,
- la réaction de cyclisation du composé de formule (XI) en composé de formule (II_{c}) peut être réalisée, par exemple, en présence d'un donneur d'azote tel que le nitrite de sodium dans un milieu acide tel que par exemple dans de l'acide chlorhydrique,
- la réaction d'addition du composé de formule (XIII) sur le composé de formule (XII) peut être réalisée, par exemple, dans un solvant tel que le toluène ou le tétrahydrofuranne de préférence en présence d'un agent dessicant tel que par exemple un tamis moléculaire, la réaction de cyclisation donnant le composé de formule (II_{d}) se produisant in situ,
- la réaction d'halogénation du composé de formule (XIV) peut être réalisée selon les méthodes usuelles connues de l'homme de métier telles que par exemple en présence de chlorure d'oxalyle ou encore de chlorure de thionyle et la réaction de cyclisation du composé ainsi obtenu pour donner le composé de formule (IIₑ) se produit en présence d'hydrazine ou d'un dérivé de l'hydrazine à chaud dans un alcool tel que par exemple du méthanol ou de l'éthanol,
- la réaction d'addition du chlorure d'acide de formule (XVI) sur le dérivé aminé de formule (XV) pour obtenir le composé de formule (XVII) peut être réalisée, par exemple, au reflux d'un solvant tel que, par exemple, la pyridine,
- la réaction de cyclisation du produit de formule (XVII) ainsi obtenu pour obtenir le produit de formule (II_{f}) se produit, par exemple, dans de l'eau oxygénée en présence de soude aqueuse, par exemple au reflux d'un solvant tel que le dioxanne.

Les produits de formules (Iᵢ) peuvent constituer des produits de formule (I_{B}), les produits de formules (II_{b}), (II_{c}), (II_{d}), (IIₑ) et (II_{f}) ainsi obtenus représentent des produits de formule (I_{B}) dans laquelle A_{1B}', A_{2B}', A_{3B}' et A_{4B}' peuvent représenter les radicaux 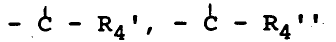 ou 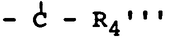 tels que définis ci-dessus soit les significations indiquées ci-dessus respectivement pour A_{1B}, A_{2B}, A_{3B} et A_{4B} dans lesquelles les éventuelles fonctions réactives sont éventuellement protégées par des groupements protecteurs et dans laquelle l'un au moins de A_{1B}, A_{2B}, A_{3B} et A_{4B} représente un radical méthine portant un radical hydroxyle.

Les produits de formule (Iᵢ) et les produits de formules (II_{b}), (II_{c}), (II_{d}), (IIₑ) et (II_{f}) ainsi obtenus, en particulier pour donner des produits de formule (I_{B}), peuvent être soumis, si désiré et si nécessaire, à l'une ou plusieurs des réactions indiquées ci-dessus, ces réactions pouvent être, dans des conditions préférentielles, réalisées de la façon indiquée ci-après.

Les radicaux hydroxyle existant ou issus de la forme tautomère oxo des composés de formules (II_{b}), (II_{c}), (II_{d}), (IIₑ) et (II_{f}) ou éventuellement des produits de formule (Iᵢ) obtenus ainsi qu'il est indiqué ci-dessus peuvent être, si nécessaire et si désiré, soumis à une réaction de réduction complète en radical méthine.

Cette réaction de réduction complète en radical méthine telle que définie ci-dessus peut être réalisée très préférentiellement après transformation de la fonction hydroxy par exemple en halogène ou mésylate, à l'aide d'un agent réducteur tel que par exemple l'hydrure de lithium et d'aluminium ou encore par réduction catalytique sur palladium en présence d'hydrogène.

La réaction de substitution des produits de formules (Iᵢ), (II_{b}), (II_{c}), (II_{d}), (IIₑ) et (II_{f}) par le radical R₄ₚ est soumise au préalable à une réaction de substitution du radical hydroxyle réalisée, par exemple, par la préparation du dérivé halogéné tel que par exemple le dérivé chloré préparé par traitement par un agent chlorant tel que par exemple le pentachlorure de phosphore ou l'oxychlorure de phosphore éventuellement dans un solvant tel que par exemple le dioxanne ou le tétrahydrofuranne ;
la réaction de substitution par le radical R₄ₚ telle que définie ci-dessus peut être réalisée par exemple par réaction avec un organométallique tel que par exemple un organozincique de formule R₄ₚ - Zn - Br si désiré en présence d'une quantité catalytique d'un complexe de métal de transition tel que par exemple le palladium ou le nickel au reflux d'un solvant tel que par exemple le tétrahydrofuranne.

Le radical hydroxyle peut être éventuellement activé par exemple en sulfonate tel que par exemple mésylate, tosylate ou encore triflate.

La réaction de transformation de la fonction oxo en fonction thioxo peut être réalisée selon les méthodes usuelles connues de l'homme de métier telles que par exemple à l'aide du réactif de Lawesson ou encore de pentasulfure de phosphore au reflux dans un solvant tel que par exemple le toluène ou un alcool tel que par exemple l'éthanol.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle, trialkylsilyle, dihydropyranne, méthoxyméthyle ou tétrahydropyrannyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acide des produits décrits ci-dessus peuvent être, si désiré, amidifiées par une amine primaire ou secondaire par exemple dans du chlorure de méthylène en présence de chlorhydrate de 1-éthyl-3-(diméthylaminopropyl) carbodiimide à la température ambiante,
- les fonctions acide peuvent être protégées par exemple sous forme d'esters formés avec les esters facilement clivables tels que les esters benzyliques ou tert-butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique ou par une base minérale ou organique, en particulier sur les éventuelles fonctions carboxy, ces réactions pouvant être réalisées selon les méthodes usuelles connues de l'homme de métier.

Les produits décrits ci-dessus peuvent, si désiré, faire l'objet, sur les éventuelles fonctions carboxy, de réactions d'estérification qui peuvent être réalisées selon les méthodes usuelles connues de l'homme de métier.

Les éventuelles fonctions ester des produits décrits ci-dessus peuvent être, si désiré, saponifiées en fonction acide, ces réactions de saponification pouvant être réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions alkyloxy telles que notamment méthoxy des produits décrits ci-dessus peuvent être, si désiré, transformées en fonction hydroxyle soit alcool dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles fonctions cyano des produits décrits ci-dessus peuvent être, si désiré, transformées en fonction acide dans les conditions usuelles connues de l'homme de métier par exemple par une hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions carboxy estérifiées des produits décrits ci-dessus peuvent, si désiré, être réduites en fonction alcool par les méthodes connues de l'homme de métier et notamment par de l'hydrure de lithium et d'aluminium dans un solvant tel que par exemple le tétrahydrofuranne ou encore le dioxanne ou l'éther éthylique.

Les éventuelles fonctions carboxy des produits décrits ci-dessus peuvent, si désiré, être réduites en fonction alcool par les méthodes connues de l'homme de métier et peuvent ainsi par exemple être d'abord estérifiées puis transformées en fonction alcool par exemple ainsi qu'il est indiqué ci-dessus.

Les éventuelles formes optiquement actives des produits de formule (I_{B}) peuvent être préparées par dédoublement des racémiques selon les méthodes usuelles.

Les composés de formule (I_{B}) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes. Certains produits de la présente invention possèdent également des propriétés antagonistes pour le récepteur à l'endothéline et sont ainsi notamment antagonistes de l'effet vasoconstricteur de l'endothéline.

Les composés de formule (I_{B}) possèdent également la propriété d'améliorer les fonctions cognitives.

Ces propriétés justifient leur application en thérapeutique et l'invention a également pour objet à titre de médicaments, les produits tels que définis par la formule (I_{B}) ci-dessus, lesdits produits de formule (I_{B}) étant sous toutes les formes isoméres possibles racémiques ou optiquement actives, ainsi que les sels d'addition avec les acides minéraux ou organiques pharmaceutiquement acceptables desdits produits de formule (I_{B}).

L'invention a tout particulièrement pour objet à titre de médicaments, les produits de formule (I_{B}) : 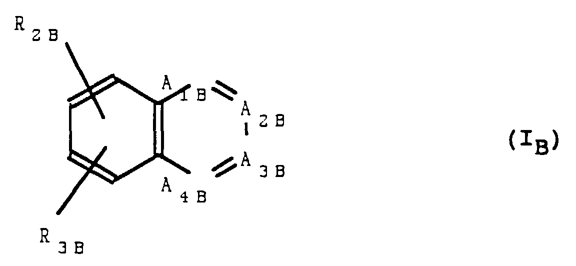 dans laquelle :
R_{2B} et R_{3B}, identiques ou différents, sont choisis dans le groupe formé par :
   - l'atome d'hydrogène,
   - les atomes d'halogène,
   - le radical hydroxyle,
   - le radical mercapto, cyano, nitro, formyle, benzoyle, acyle ayant au plus 6 atomes de carbone,
   - les radicaux carboxy libre, salifié ou estérifié par un radical alkyle linéaire ou ramifié renfermant au plus 4 atomes de carbone,
   - les radicaux alkyle, alkényl, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole, isoxazole, pyrrolidinyle, pyrrolidinylcarbonyle et phényle éventuellement substitué par un ou plusieurs radicaux choisis parmi les atomes d'halogène, le radical hydroxyle, les radicaux alkyle et alkoxy renfermant au plus 4 atomes de carbone,
   - les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pyrrolidinylcarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{1B}, A_{2B}, A_{3B} et A_{4B} sont tels que :
   - l'un représente un atome d'azote,
   - un autre représente un atome d'azote ou le radical méthine substitué par R_{4b},
   - un autre représente le radical méthine substitué par R_{4c},
   - et le dernier représente le radical méthine substitué par le radical benzyle lui-même substitué par un ou plusieurs radicaux choisis parmi les radicaux cyano, carboxy libre, salifié et estérifié,
R_{4b} et R_{4c} identiques ou différents, sont choisis parmi les valeurs de R_{4B},
   tel que R_{4B} représente :
   - l'atome d'hydrogène, le radical hydroxyle, cyano, carboxy libre, salifié ou estérifié par un radical alkyle renfermant au plus 4 atomes de carbone,
   - le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (I_{B}) étant sous toutes les formes isomères possibles racémiques, enantiomères et diastéréoisomères, ainsi que les sels d'addition avec les acides minéraux et organiques ou avec les bases minérales et organiques pharmaceutiquement acceptables desdits produits de formule (I_{B}).

L'invention a encore plus particulièrement pour objet, à titre de médicaments, les produits de formule (I_{B}) telle que définie ci-dessus, et répondant à la formule (Iₐ) : 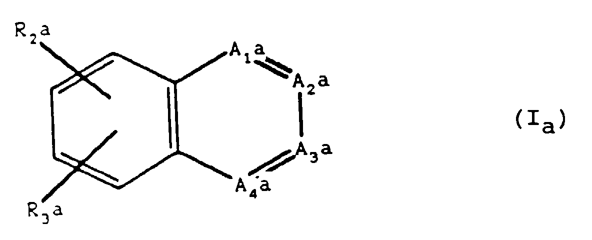 dans laquelle :
R₂ₐ et R₃ₐ, identiques ou différents, sont choisis dans le groupe formé par :
   - l'atome d'hydrogène,
   - les atomes d'halogène,
   - le radical hydroxyle,
   - le radical mercapto, cyano, nitro, formyle, benzoyle, acyle ayant au plus 6 atomes de carbone,
   - les radicaux carboxy libre, salifié ou estérifié par un radical alkyle linéaire ou ramifié renfermant au plus 4 atomes de carbone,
   - les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
   - les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A₁ₐ, A₂ₐ, A₃ₐ et A₄ₐ sont tels que :
   - l'un représente un atome d'azote,
   - un autre représente un atome d'azote ou le radical méthine substitué par R_{4ab},
   - un autre représente le radical méthine substitué par R_{4ac},
   - et le dernier représente le radical méthine substitué par le radical benzyle éventuellement substitué,
R_{4ab} et R_{4ac} identiques ou différents, sont choisis parmi les valeurs de R₄ₐ,
   tel que R₄ₐ représente :
   - l'atome d'hydrogène, le radical hydroxyle, cyano, carboxy libre, salifié ou estérifié par un radical alkyle renfermant au plus 4 atomes de carbone,
   - le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Iₐ).

L'invention a plus particulièrement pour objet, à titre de médicaments, les produits décrits ci-après dans les exemples et notamment les produits de formule (I_{B}) telle que définie ci-dessus, dans laquelle :
R_{2B} et R_{3B} représentent un atome d'hydrogène,
A_{1B}, A_{2B} et A_{3B} sont tels que l'un ou deux d'entre eux représentent un atome d'azote, et les autres, identiques ou différents, représentent le radical 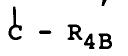 = tel que R_{4B} est choisi parmi l'atome d'hydrogène, le radical n-butyle et alkylthio renfermant au plus 4 atomes de carbone,
et A_{4B} représente le radical méthine substitué par le radical benzyle éventuellement substitué par un ou plusieurs radicaux choisis parmi les radicaux cyano, carboxy libre, salifié et estérifié,
lesdits produits de formule (I_{B}) étant sous toutes les formes isomères possibles racémiques, énantiomères et diastéréoisomères, ainsi que leurs sels d'addition avec les acides minéraux ou organiques pharmaceutiquement acceptables.

Les médicaments, objet de l'invention, peuvent être utilisés dans le traitement des affections cardiovasculaires présentant une altération de la vasomotricité : infarctus du myocarde, insuffisance cardiaque, insuffisance rénale, angine de poitrine, spasme vasculaire cérébral, maladie de Raynaud, hypertension artérielle et toutes les affections consécutives à une ischémie. Ces médicaments, objet de l'invention, pourraient également être utilisés pour le traitement du glaucome, de l'atherosclérose, de l'asthme et de différents types de spasmes viscéraux, ainsi qu'à titre de substances protectrices neuromales ou encore dans la prévention des resténoses postangioplastie.

Ils peuvent également être utilisés dans le traitement de certains désordres gastro-intestinaux, gynécologiques et en particulier pour un effet relaxant au niveau de l'utérus.

Les médicaments objets de l'invention peuvent également être utilisés dans le traitement de troubles de la mémoire, de la démence sénile et de la maladie d'Alzheimer.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifies, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (III), (V), (VI), (VII), (IX), (X), (XII), (XIII), (XIV), (XV) et (XVI) peuvent être disponibles dans le commerce ou peuvent être préparées selon les méthodes usuelles connues de l'homme de métier.

Les composés de formule (III) peuvent constituer des dérivés du chlorure de phénylacétyle.

Parmi les exemples de préparation de tels composés de formule (III) décrits dans la littérature, on peut citer notamment les références suivantes :
- Organic Synthesis 1972, 36
- Can. J. Chem. 1957, 35, 651.

Les composés de formule (V) peuvent constituer des dérivés de type nitrile.

Parmi les exemples de préparation de tels composés de formule (V) décrits dans la littérature, on peut citer notamment les références suivantes :
- Synthesis 1987, p. 514.

Les composés de formule (VI) peuvent constituer des dérivés de l'aniline.

Comme composés de formule (VI) qui peuvent être trouvés dans le commerce, on peut citer par exemple certains composés de formule (VI) telle que l'un de R₂ ou R₃ représente un radical carbométhoxy, comme par exemple l'anthranilate de méthyle, ou le 3-amino benzoate d'éthyle, que l'on peut trouver, par exemple, sous forme de produit commercialisé par Aldrich.

Les composés de formule (VII) peuvent constituer des esters dérivés de l'acide formylacétique.

Parmi les exemples de préparation de tels composés de formule (VII) décrits dans la littérature, on peut citer notamment les références suivantes :
- J. Het. Chem. 20, p. 623-628, 1983,
- Liebigs Ann. Chem. 697, p. 62-68, 1966.

Les composés de formule (IX) peuvent constituer notamment des dérivés de l'ortho halo aniline.

Parmi les exemples de préparation de tels composés de formule (IX) décrits dans la littérature, on peut citer notamment les références suivantes :
- Ann. Chim. 1962, 52, p. 727.

Les composés de formule (X) peuvent constituer notamment des dérivés de l'acétylène.

Parmi les exemples de préparation de tels composés de formule (X) décrits dans la littérature, on peut citer notamment les références suivantes :
- J. Am. Chem. Soc. 1937, 59, 1490.

Parmi les composés de formule (XII) qui peuvent être trouvés dans le commerce, on peut citer par exemple le 3,4 diaminobenzoate de méthyle que l'on peut trouver, par exemple, sous forme de produit commercialisé par exemple par LANCASTER.

Parmi les exemples de préparation de tels composés de formule (XII) décrits dans la littérature, on peut citer notamment les références suivantes :
- Org. Synth. 1943, 501.

Les composés de formule (XIII) peuvent constituer des dérivés de l'acide glyoxylique.

Parmi les exemples de préparation de tels composés de formule (XIII) décrits dans la littérature, on peut citer notamment les références suivantes :
- J. Org. Chem. 1979, 44, 1613.

Les composés de formule (XIV) peuvent constituer des dérivés de l'acide phtalique.

Parmi les exemples de préparation de tels composés de formule (XIV) décrits dans la littérature, on peut citer notamment les références suivantes :
- J. Org. Chem. 1963, 28, 582,
- J. Chem. Soc. 1952, 553.

Les composés de formule (XV) peuvent constituer des dérivés de la cyanoaniline.

Ces composés pouvant être trouvés dans le commerce, on peut citer par exemple le 2-aminobenzonitrile commercialisé par Aldrich, ou encore la 5-chloro 2-cyanoaniline commercialisée par Bayer.

Les composés de formule (XVI) peuvent constituer par exemple des dérivés de chlorure d'acyle.

Parmi les exemples de préparation de tels composés de formule (XVI) décrits dans la littérature, on peut citer notamment les références suivantes :
- Org. Synth. Vol. III, p. 190.

D'autre part, certains produits intermédiaires peuvent être trouvés dans le commerce tel que par exemple des dérivés chlorés tel que le composé 4-chloro 2-phénylquinazoline qui peut être fourni par exemple par ALDRICH : un exemple de préparation de ce composé peut être trouvé dans la référence suivante :
- J. Org. Chem., 37, 1681 (1972).

Le composé 4-chloro 2-phénylquinazoline permet de préparer des produits de formule (I) dérivés de la 2-phénylquinazoline dans lesquels le radical R₄ tel que défini ci-dessus peut être introduit, par exemple, par l'intermédiaire d'un composé organométallique tel que R₄ - Zn - Br par substitution sur l'atome de chlore dans les conditions usuelles connues de l'homme de métier.

La présente invention a également pour objet à titre de produits industriels nouveaux, et notamment à titre de produits intermédiaires nécessaires à la préparation des produits de formule (I), les composés de formule (VIII), (II_{b}), (II_{c}), (II_{d}), (IIₑ) et (II_{f}) telles que définies ci-dessus à l'exception des composés suivants :
a) les composés de formule (VIII) dans lesquels R_{2'B} et R₃'_{B} = H, R₄'_{B} = CO₂Et, Me, Et, CF₃, H et R4''_{B} = CO₂Et ;
   ainsi que le composé :
   2-[1-[2-(trifluorométhyl) phényl amino]pent-1-ylidène]propanedioate d'éthyle.
b) les composés de formule (II_{b}) dans lesquels R₄'_{B} = H, Hal ou alkyle de 1 à 4 C et R4''_{B} = CF₃ ;
   ainsi que le composé :
   2-butyl 4-hydroxy 8-trifluorométhyl 3-quinoléine carboxylate d'éthyle
c) les composés de formule (II_{c}) dans lesquels R'_{2B} et R'_{3B} représentent l'un H et l'autre phényle et R₄'''_{B} représente carboxy libre ou estérifié ;
d) le composé de formule (IIₑ) :
   4-butyle-1-phthalazinone
e) les composés de formule (II_{f}) dans lesquels R_{2'B} et R₃'_{B} représentent H, Hal, CF₃ ou alkyle et R'_{4B} représente alkyle ou CF₃.

La présente invention a particulièrement pour objet l'utilisation des produits de formule (I_{B}) telle que définie ci-dessus lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}), pour la préparation de médicaments destinés :
**soit** au traitement de l'hypertension artérielle, des insuffisances cardiaques, des insuffisances rénales et dans la prévention des resténoses post-angioplastie,
**soit** au traitement de certains désordres gastro-intestinaux ou gynécologiques.

La présente invention a tout particulièrement pour objet l'utilisation selon ci-dessus caractérisée en ce que les produits de formule (I_{B}) sont utilisés dans la préparation de médicaments destinés au traitement de l'hypertension artérielle, des insuffisances cardiaques, des insuffisances rénales et dans la prévention des resténoses post-angioplastie.

La présente invention a plus particulièrement pour objet l'utilisation selon ci-dessus, caractérisée en ce que les produits de formule (I_{B}) répondent à la formule (Iₐ) : 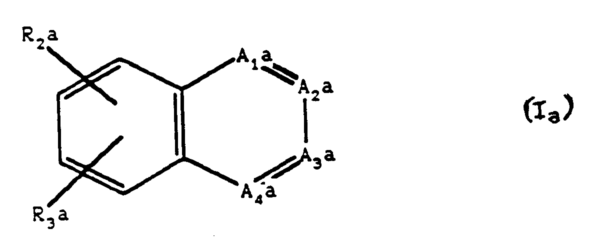 dans laquelle :
R₂ₐ et R₃ₐ, identiques ou différents, sont choisis dans le groupe formé par :
   - l'atome d'hydrogène,
   - les atomes d'halogène,
   - le radical hydroxyle,
   - le radical mercapto, cyano, nitro, formyle, benzoyle, acyle ayant au plus 6 atomes de carbone,
   - les radicaux carboxy libre, salifié ou estérifié par un radical alkyle linéaire ou ramifié renfermant au plus 4 atomes de carbone,
   - les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
   - les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A₁ₐ, A₂ₐ, A₃ₐ et A₄ₐ sont tels que :
   - l'un représente un atome d'azote,
   - un autre représente un atome d'azote ou le radical méthine substitué par R₄ₐ,
   - un autre représente le radical méthine substitué par R₄ₐ,
   - et le dernier représente le radical méthine substitué par le radical benzyle lui-même substitué, par un ou plusieurs radicaux choisis parmi les radicaux cyano, carboxy libre, salifié et estérifié tel que R₄ₐ représente :
   - l'atome d'hydrogène, le radical hydroxyle, cyano, carboxy libre, salifié ou estérifié par un radical alkyle renfermant au plus 4 atomes de carbone,
   - le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ).

Parmi les produits de formule (I_{B}) tout particulièrement préférés se trouvent également les produits répondant aux formules indiquées ci-dessous dans lesquelles R_{2B} et R_{3B} ont les significations indiquées ci-dessus, n-bu représente le radical n-butyle et R', R'' et R''' représentent les substituants du radical benzyle que porte le radical méthine de l'un de A_{1B}, A_{2B}, A_{3B} ou A_{4B} tel que définis ci-dessus :

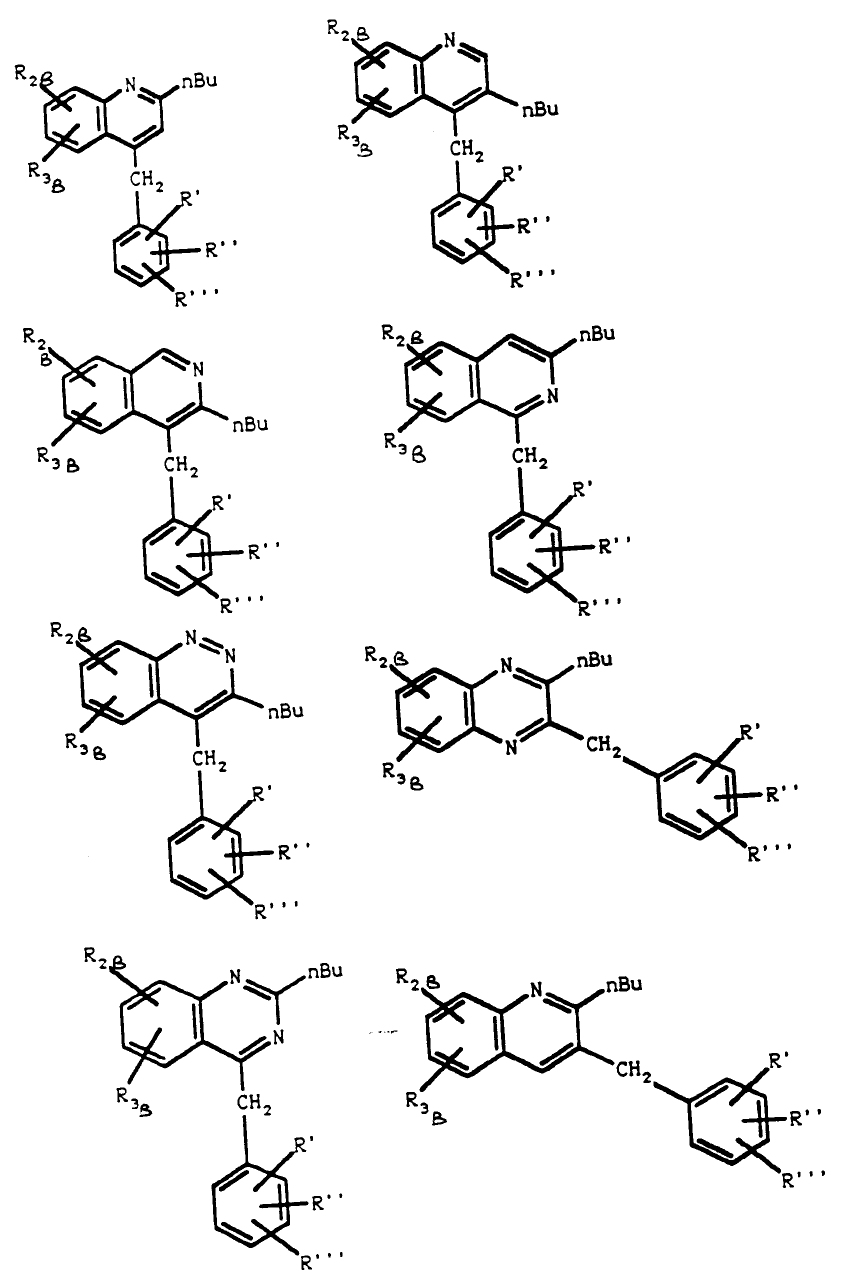

Les exemples suivants illustrent l'invention sans toutefois la limiter.

### EXEMPLE 1 : 4-[(3-butyl 4-quinoléinyl) méthyl] benzonitrile

A un mélange agité de 0,720 g de 4-chloro 3-butyl quinoléine obtenu au stade F de la préparation ci-après, avec 0,8 cm³ de tétrahydrofuranne et 0,663 g de tétrakis triphénylphosphine palladium, on ajoute 14 cm³ d'une solution 0,38 M de zincique de 4-bromométhyl benzonitrile dans le tétrahydrofuranne (préparé selon Knochel J. Org. Chem. 1988, Vol. 53, pages 5789-5791). On agite 4 heures à 60°C puis 16 heures à température ambiante. Le milieu est hydrolysé avec 100 cm³ d'acide chlorhydrique 0,1 N puis extrait avec de l'acétate d'éthyle, lave avec une solution saturée de chlorure de sodium, sèche et évapore à sec. On chromatographie le résidu sur silice (éluant : hexane-acétate d'éthyle (7-3)). On obtient 0,445 g de produit recherché.

### Spectre IR (CHCl₃)

- C≡N: 2232 cm⁻¹
- Aromatiques: 1609 - 1573 - 1506 cm⁻¹

### Préparation de l'exemple 1 : 3-butyl 4-chloro quinoléine

Le produit utilisé au départ de l'exemple 1 a été préparé de la façon suivante.

### Stade A : 2-butyl 3-oxo butanedioate de diéthyle

A une suspension d'éthylate de sodium préparé par agitation pendant 1 heure à 40°C de 2,3 g de sodium et 150 cm³ d'éthanol, puis évaporation du solvant, dans 100 cm³ d'éther, on ajoute 13,55 cm³ de diéthyl oxalate. On chauffe 15 minutes au reflux, refroidit légèrement et ajoute 50 cm³ de caproate d'éthyle, on agite 3 heures au reflux et 16 heures à 30-35°C. On ajoute 50 cm³ d'eau sépare la phase aqueuse, par décantation, la lave avec de l'éther par deux fois et acidifie avec de l'acide chlorhydrique 2N. On extrait 3 fois à l'éther, lave les phases organiques à l'eau puis avec une solution saturée de chlorure de sodium, sèche et évapore à sec. Le produit obtenu, 9,5 g est utilisé tel quel pour le stade suivant.

### Stade B : 2-butyl 3-(phénylamino) 2-butènedioate de diéthyle

On agite 3 jours à 75°C, un mélange de 1 g d'aniline avec 2,65 g du produit obtenu au stade A ci-dessus et 150 mg de siliporite ® NK10, on refroidit et chromatographie le milieu réactionnel sur silice (éluant : hexane-acétate d'éthyle (9-1)) on obtient 1,55 g de produit attendu.

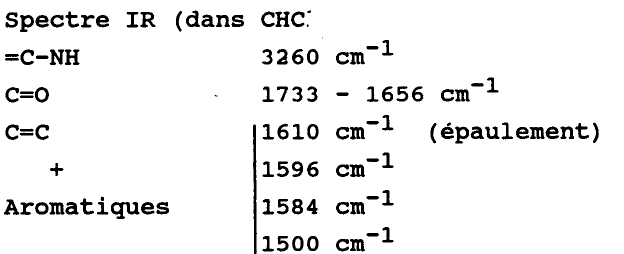

### Stade C : 3-butyl 4-hydroxy 2-quinoléinecarboxylate d'éthyle et 3-butyl 1,4-dihydro 4-oxo 2-quinoléinecarboxylate d'éthyle

On chauffe pendant 30 minutes à l'aide d'un bain à 250°C un mélange de 2,5 g du produit obtenu au stade B ci-dessus, avec 30 cm³ de diphényléther. On laisse revenir à température ambiante, essore, lave avec du pentane et recueille 1,82 g de produit recherché.

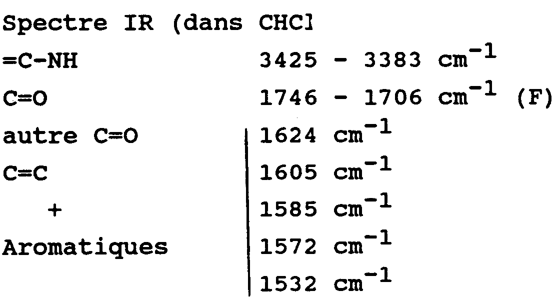

### Stade D : Acide 3-butyl 1,4-dihydro 4-oxo 2-quinoléinecarboxylique

On chauffe 1 heure à 60°C, 1,8 g de l'ester obtenu au stade C ci-dessus, avec 25 cm³ de soude en solution N. On refroidit et acidifie avec de l'acide chlorhydrique N, on filtre, lave à l'eau et sèche à 60°C sous pression réduite, on obtient 1,58 g du produit recherché.

### RMN 60 MHz (D.M.S.O.) ppm

- CH₃-CH₂-CH₂-CH₂-: 0,89 (t)
- C-C-C-C: 1,39 (m)
- C-C-C-C-: 2,78
- H₆ et H₇: 7,30 (t) - 7,63 (t)
- H₅ et H₈: 7,80 (d) - 8,08 (d)
- Proton mobile: 11,64

### Stade E : 3-butyl 4(1H)-quinolone

On chauffe 30 minutes à 250°C, un mélange de 1,55 g du produit obtenu au stade D ci-dessus et 10 cm³ de diphényl éther. On refroidit, filtre, lave avec du pentane et sèche sous pression réduite. On obtient 1,17 g de produit que l'on dissout dans 80 cm³ d'éthanol et traite 15 minutes au reflux en présence de charbon actif, on filtre sur hyflosupercel, évapore l'éthanol jusqu'à sec et reprend avec du pentane, filtre, lave avec du pentane et sèche à 50°C sous pression réduite. On obtient 971 mg de produit recherché.

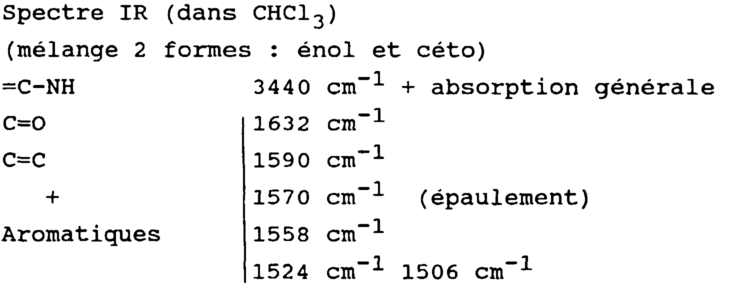

### Stade F : 3-butyl 4-chloro quinoléine

On chauffe 2 heures à 120°C un mélange de 515 mg du produit obtenu au stade E ci-dessus avec 0,6 cm³ d'oxychlorure de phosphore. On refroidit, ajoute 10 cm³ d'eau et alcalinise à pH 9 avec de l'ammoniaque concentré. On extrait 2 fois avec du chlorure de méthylène, lave avec une solution saturée de chlorure de sodium, sèche et évapore à sec. Le résidu est dissous dans 30 cm³ d'éthanol et traite 15 minutes au reflux en présence de charbon actif, on filtre sur hyflosupercel, on obtient 511 mg du produit recherché.

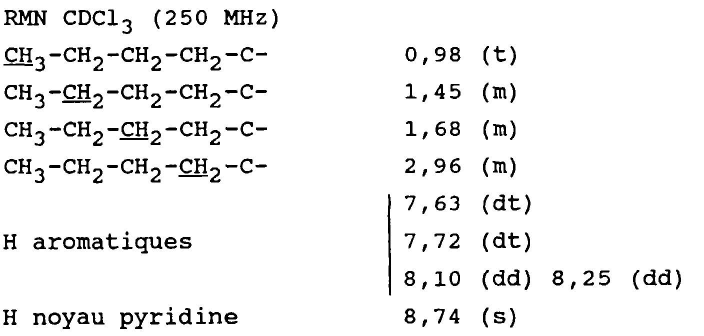

### EXEMPLE 2 : Acide 4-[(3-butyl 4-quinoléinyl) méthyl] benzoïque

On agite pendant 15 heures à 80°C, 0,340 g du composé obtenu à l'exemple 1, avec 2 cm³ d'éthanol et 5 cm³ de soude en solution 2N. On évapore l'éthanol, reprend à l'eau et acidifie à pH 4 avec de l'acide acétique, on filtre, lave à l'eau et sèche à 50°C sous pression réduite. On obtient 345 mg du produit recherché. On dissout le produit obtenu dans 50 cm³ d'un mélange éthanol-chlorure de méthylène (1-1) et traite au reflux avec du charbon actif. On filtre sur hyflosupercel, évapore presqu'à sec et reprend par 50 cm³ d'isopropanol, on obtient 205 mg du produit attendu. F # 233°C.

| Analyse pour C₂₁H₂₁NO₂ = 319,41 | | | |
|---|---|---|---|
| | C | H | N |
| % calculés | 78,97 | 6,62 | 4,38 |
| % trouvés | 79,0 | 6,8 | 4,2 |

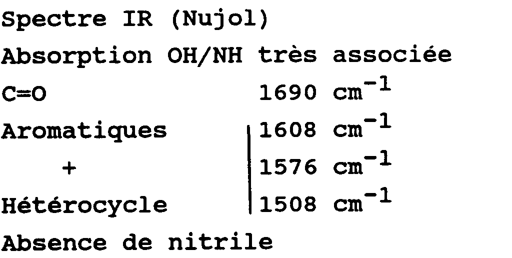

### EXEMPLE 3 : 4-[(2-butyl 4-quinoléinyl) méthyl] benzonitrile

On opère comme à l'exemple 1, à partir de 0,614 g de 4-chloro 2-butyl quinoléine, obtenue comme décrit ci-après, 0,8 cm³ de tétrahydrofuranne, 0,323 g de tétrakis triphényl phosphine palladium et 10 cm³ d'une solution de zincique de 4-bromo méthyl benzonitrile à 0,5 M dans le tétrahydrofuranne. On obtient après chromatographie sur silice (éluant : hexane-acétate d'éthyle (8-2)) 0,781 g du produit recherché.

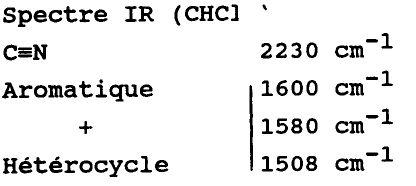

### Préparation de l'exemple 3 : 2-butyl 4-chloro quinoléine

Le produit utilisé au départ de l'exemple 3 a été préparé comme suit :

### Stade A : 3-oxo heptanoate d'éthyle

A une suspension de 27,3 g d'hydrure de sodium à 50 % dans l'huile (préalablement lavé à l'heptane) et 250 cm³ d'éther, on ajoute 70,8 g de carbonate d'éthyle en solution dans 50 cm³ d'éther. On agite 10 minutes, ajoute en 30 minutes 30 g d'hexanone et chauffe 2 heures au reflux. On ajoute 35 cm³ d'éther contenant 12 cm³ d'éthanol et agite 16 heures à température ambiante. On refroidit à 0°C et ajoute une solution de 36 cm³ d'acide acétique dans 300 cm³ d'eau puis 12 cm³ d'une solution saturée de bicarbonate de sodium le pH est alors de 7. On extrait avec de l'éther, lave à l'eau, sèche, évapore à sec. On obtient, après distillation à 70°C sous une pression réduite de 7 mbar, 32,5 g du produit recherché.

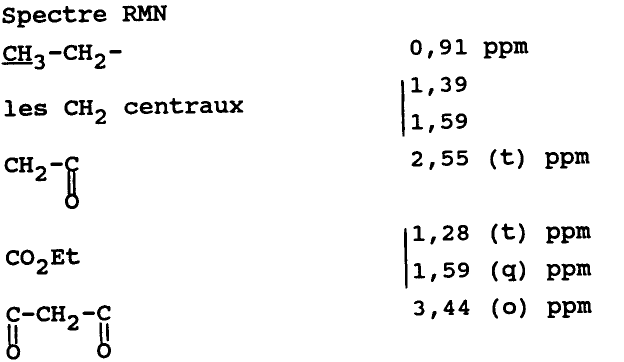

### Stade B : 3-(phénylamino) heptanoate d'éthyle

On opère comme au stade B de la préparation de l'exemple 1 à partir de 45 g de produit obtenu comme au stade A, ci-dessus. On obtient, après chromatographie sur silice (éluant hexane-acétate d'éthyle (95-5)) 28,7 g du produit attendu.

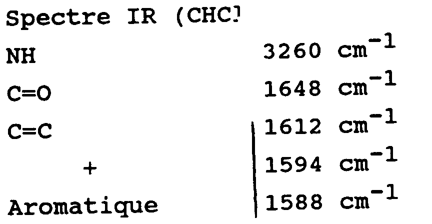

### Stade C : 2-butyl 4-quinolone

On opère comme au stade C de la préparation de l'exemple 1 à partir de 28,7 g du composé obtenu au stade B ci-dessus. On obtient 16,95 g du produit recherché. F = 140°C.

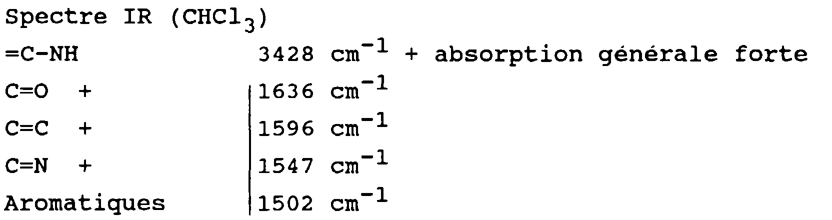

### Stade D : 2-butyl 4-chloro quinoléine

On opère comme à l'exemple F de la préparation de l'exemple 1, à partir de 4 g du composé obtenu au stade C ci-dessus, on obtient 3,89 g de produit recherché.

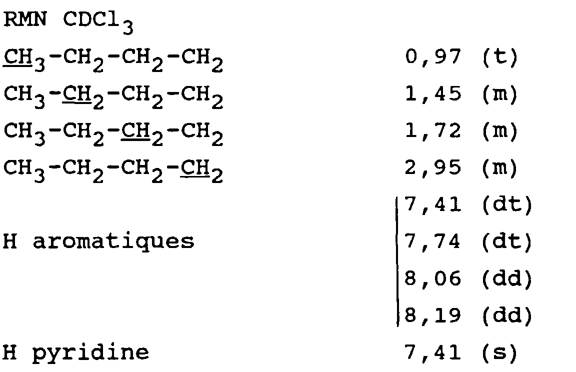

### EXEMPLE 4 : Acide 4-[(2-butyl 4-quinoléinyl) méthyl] benzoïque

On opère comme à l'exemple 2 à partir de 0,750 g du produit obtenu à l'exemple 3 avec 2,5 cm³ d'eau et 2,5 cm³ de lessive de soude. On obtient 205 mg du produit recherché, après recristallisation. F = 158°C.

| Analyse pour : C₂₁H₂₁NO₂ = 319,41 | | | |
|---|---|---|---|
| | C | H | N |
| % calculés | 78,97 | 6,62 | 4,38 |
| % trouvés | 79,2 | 6,7 | 4,3 |

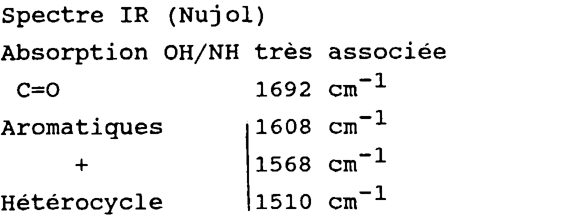

### EXEMPLE 5 : 4-[(3-butyl 4-cinnolinyl) méthyl] benzonitrile

A une solution de 0,87 g de 3-butyl 4-chloro cinnoline obtenu au stade C de la préparation décrite ci-après, avec 30 cm³ de tétrahydrofuranne, on ajoute 100 mg de chlorure de nickel diphényl phosphino propane, chauffe au reflux et ajoute 10 cm³ d'une solution 0,5 M dans le tétrahydrofuranne de zincique de 4-bromométhyl benzonitrile (préparé selon Knochel J. Org. Chem. 1988, Vol. 53, p. 5789-5791) on agite 1 heure à 50°C puis verse dans l'eau glacée. On alcalinise par addition d'ammoniaque, concentre et extrait avec de l'acétate d'éthyle, lave à l'eau, sèche et évapore à sec. On chromatographie le résidu sur silice, (éluant : acétate d'éthyle-hexane (1-1)). On obtient 680 mg du produit recherché.

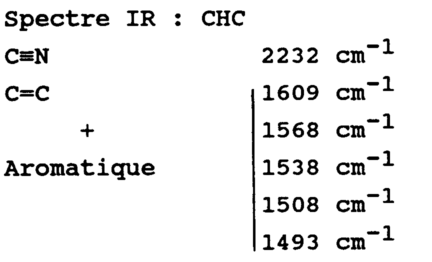

### Préparation du produit utilisé au départ de l'exemple 5 : 3-butyl 4-chloro cinnoline

### Stade A : 2-(1-hexynyl) benzeneamine

A une solution de 4,4 g de 2-iodo aniline dans 100 cm³ de triéthylamine, on ajoute 32 mg d'iodure de cuivre, 140 mg de chlorure de bistriphényl phosphine palladium puis 2,3 cm³ de 1-hexyne. On agite 15 heures à température ambiante. On évapore à sec et reprend à l'éther, filtre l'insoluble, le lave à l'éther et les fractions éthérées sont évaporées à sec. On chromatographie le résidu sur silice (éluant : hexane-acétate d'éthyle (9-1)). On obtient 3,27 g de produit recherché.

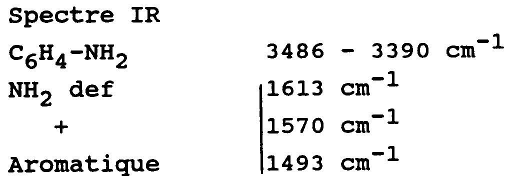

### Stade B : 3-butyl 4-hydroxy cinnoline

A une suspension à 0°C de 3,2 g du produit obtenu au stade A, ci-dessus, dans 100 cm³ d'acide chlorhydrique concentré, on ajoute à 0°C, une solution de 2 g de nitrite de sodium dans 60 cm³ d'eau, on agite 1 heure 30 à 0°C, puis 1 heure à 100°C. On verse le milieu réactionnel dans 100 cm³ d'eau glacée, essore, lave à l'eau glacée. Le produit humide est repris par 100 cm³ d'eau et alcalinise avec de l'ammoniaque concentrée. On essore, lave à l'eau et sèche à 70°C sous pression réduite. On obtient 1,47 g du produit attendu. F = 180°C.

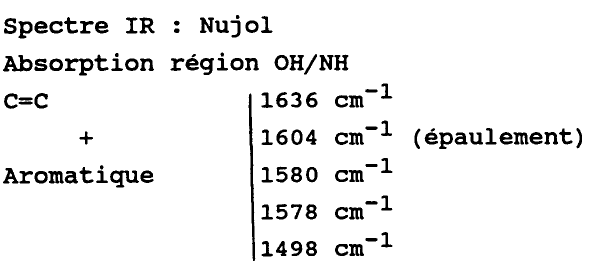

### Stade C : 3-butyl 4-chloro cinnoline

On opère comme au stade F de la préparation 1, à partir de 1,2 g du produit obtenu au stade B ci-dessus, en utilisant 10 cm³ d'oxychlorure de phosphore. Après chromatographie sur silice (éluant : hexane-acétate d'éthyle (6-4)) on obtient 1,16 g du produit recherché. F < 50°C.

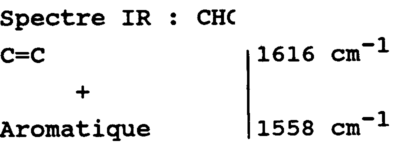

### EXEMPLE 6 : Chlorhydrate de l'acide 4-[(3-butyl 4-cinnolinyl) méthyl] benzoïque

On agite 6 heures au reflux, une solution de 0,38 g du produit obtenu à l'exemple 5, avec 1,3 cm³ de soude en solution 5N et 10 cm³ d'éthanol. On verse dans de l'eau et acidifie par addition d'hydrogénophosphate de sodium, essore, lave à l'eau et sèche à 60°C, sous pression réduite. On obtient 380 mg du produit attendu. F = 220°C.

### Préparation du chlorhydrate :

A une solution de 320 mg de la base obtenue ci-dessus dans 20 cm³ d'acétate d'éthyle et 10 cm³ d'acétonitrile, on ajoute 0,09 cm³ d'acide chlorhydrique concentré. On agite 1 heure à température ambiante, essore, lave à l'acétate d'éthyle et sèche à 40°C, sous pression réduite. On obtient 190 mg du produit recherché. F = 212°C.

| Analyse pour C₂₀H₂₁N₂O₂Cl = 356,85 | | | | |
|---|---|---|---|---|
| | C | H | N | Cl |
| % calculés | 67,32 | 5,93 | 7,85 | 9,93 |
| % trouvés | 67,5 | 6,0 | 8,0 | 10,0 |

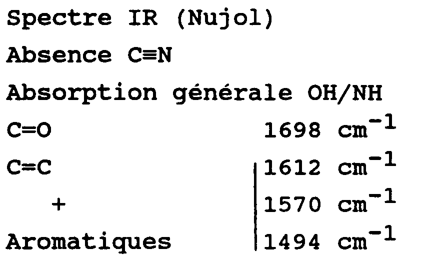

### EXEMPLE 7 : 4-[(3-butyl 1-isoquinoléinyl) méthyl] benzoate de méthyle

A une solution de 400 mg de 1-phényl 2-hexanone, obtenu à la préparation B décrite ci-après, avec 400 mg de 4-cyanométhyl benzoate de méthyle, dont la préparation A est décrite ci-après, et 10 cm³ d'oxychlorure de phophone, on ajoute 0,3 cm³ de chlorure stannique. On agite 2 heures au reflux. On verse sur de l'eau et alcalinise par addition d'ammoniaque concentré, extrait avec de l'acétate d'éthyle, lave à l'eau, sèche et évapore à sec. On chromatographie le résidu sur silice (éluant : hexane-acétate d'éthyle (7-3)). On obtient 470 mg du produit recherché.

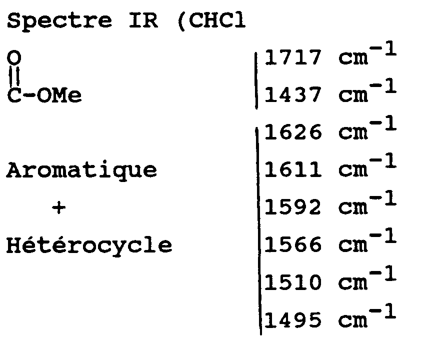

### Préparation A de l'exemple 7 : 4-(cyanométhyl) benzoate de méthyle

Préparation du composé utilisé au départ de l'exemple 7.

### Stade A : 4-(bromométhyl) benzoate de méthyle

A une suspension de 10 g d'acide 4-(bromométhyl) benzoïque dans 50 cm³ de chlorure de méthylène, on ajoute lentement du diazométhane jusqu'à coloration jaune persistante du milieu. L'excès de diazométhane est détruit par addition d'acide acétique. On amène à sec, lave à l'eau et sèche sous pression réduite à température ambiante.

On obtient 9,25 g du produit recherché F = 54°C.

### Stade B : 4-cyanométhyl benzoate de méthyle

A une solution de 4 g du produit obtenu au stade A ci-dessus, dans 50 cm³ de méthanol, on ajoute 3,4 g de cyanure de potassium et agite pendant 4 heures au reflux.

On verse sur de l'eau, extrait avec de l'acétate d'éthyle, lave à l'eau, sèche et évapore à sec. Le résidu est chromatographié sur silice (éluant : hexane-acétate d'éthyle (7-3)). On obtient 2,4 g du produit attendu F = 154°C

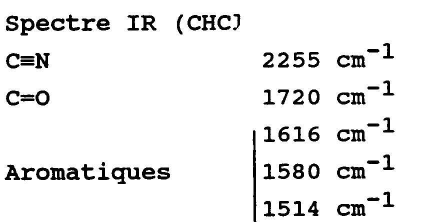

### Préparation B de l'exemple 7 : 1-phényl 2-hexanone

A une solution de 40 cm³ de chlorure de phényl acétyle dans 500 cm³ de tétrahydrofuranne, on ajoute 100 cm³ de n-tétrabutyl étain puis 120 mg de [benzyl (chloro) bis(triphényl phosphine)] palladium. On agite 15 heures au reflux, filtre sur clarcel et évapore à sec. Le résidu est distillé à 65-68°C sous 0,5 mn de mercure. La fraction principale est chromatographiée sur silice (éluant : hexane-acétate d'éthyle (9-1)). On obtient 27,5 g du produit recherché.

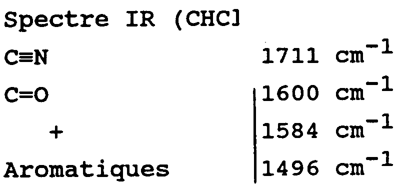

### EXEMPLE 8 : Acide 4-[(3-butyl 1-isoquinoléinyl) méthyl] benzoïque

On opère comme à l'exemple 2 à partir de 550 mg du composé obtenu à l'exemple 7. On obtient 500 mg de produit F = 110°C que l'on recristallise dans 10 cm³ d'un mélange acétonitrile-éther isopropylique (1-1)). On obtient 240 mg du produit attendu. F = 112°C.

| Analyse pour C₂₁H₂₁NO₂ = 319,41 | | | |
|---|---|---|---|
| | C | H | N |
| % calculés | 78,97 | 6,63 | 4,38 |
| % trouvés | 78,8 | 6,5 | 4,3 |

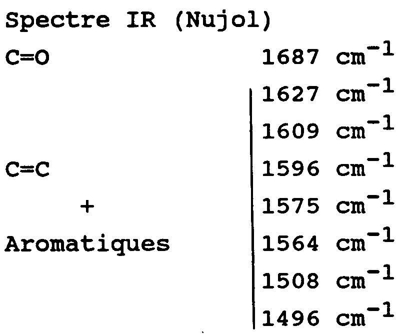

### EXEMPLE 9 : 4-[(3-butyl 1-éthylthio 4-isoquinoléinyl) méthyl] benzoate de méthyle

A une solution de 1 g de 4-(3-oxo 2-phényl heptyl) benzoate de méthyle, dont la préparation est donnée ci-après, dans 20 cm³ d'oxychlorure de phosphore, on ajoute 0,4 cm³ de thiocyanate d'éthyle et 0,45 cm³ de chlorure stannique. On agite 10 heures au reflux puis verse dans de l'eau, glacée, agite 15 minutes et alcalinise avec de l'ammoniaque concentrée. On extrait avec de l'acétate d'éthyle, lave à l'eau, sèche et évapore à sec. On chromatographie le résidu sur silice (éluant : hexane-acétate d'éthyle (9-1)). On obtient 0,57 g du produit recherché. F = 70°C.

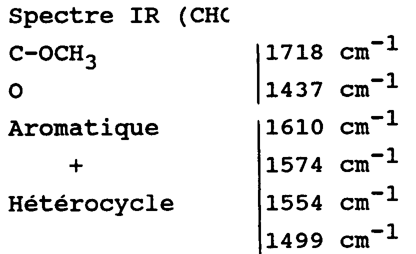

### Préparation de l'exemple 9 : le 4-(3-oxo 2-phényl heptyl) benzoate de méthyle a été préparé de la façon suivante :

### Stade A : 4-(3-oxo 2-phényl heptyl) benzonitrile

A une solution de 0,81 g de 1-phényl 2-hexanone obtenu à la préparation A de l'exemple 7 dans 20 cm³ de tétrahydrofuranne, on ajoute 240 mg d'hydrure de sodium en dispersion à 50 % dans l'huile. On agite 1 h 30 à 40°C, on ajoute alors une solution de 900 mg de 4-(bromométhyl) benzonitrile dans 10 cm³ de tétrahydrofuranne. On agite 2 heures à température ambiante puis verse sur de l'eau. On extrait avec de l'acétate d'éthyle et lave à l'eau, sèche, évapore à sec. On chromatographie le résidu sur silice (éluant : chlorure de méthylène-hexane (1-1)). On obtient 1,1 g du produit recherché. F = 70°C.

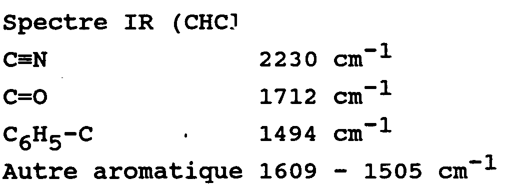

### Stade B : Acide 4-(3-oxo 2-phényl heptyl) benzoïque

On agite 15 heures au reflux 3,5 g de produit obtenu comme au stade A ci-dessus, avec 20 cm³ de soude en solution N et 50 cm³ d'éthanol. On verse sur de l'eau glacée, acidifie avec de l'acide chlorhydrique concentré. On essore, lave à l'eau, sèche. On empâte le produit brut avec 100 cm³ d'éther isopropylique. On obtient 3,27 g du composé recherché.
F = 140°C.

### Spectre IR (Nujol)

- Absence de C≡N:
- C=O: 1708 - 1677 cm⁻¹
- Aromatique: 1610 - 1575 - 1494 cm⁻¹

### Stade C : 4-(3-oxo 2-phényl heptyl) benzoate de méthyle

On opère comme au stade A de la préparation B de l'exemple 7, à partir de 3,27 g du produit obtenu au stade B, ci-dessus, on obtient 3,36 g du produit attendu. F = 58°C.

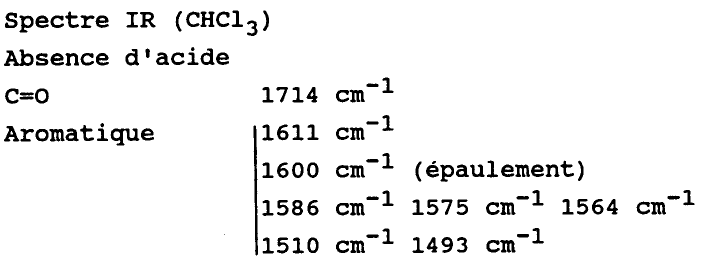

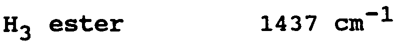

### EXEMPLE 10 : Acide 4-[(3-butyl 1-éthylthio 4-isoquinoléinyl) méthyl] benzoïque

On opère comme à l'exemple 2, à partir de 500 mg du produit obtenu à l'exemple 9, on obtient 490 mg du produit recherché F = 150°C. Après recristallisation dans 30 cm³ d'acétonitrile, on recueille 270 mg de produit. F = 171°C.

| Analyse pour C₂₃H₂₅NO₂S = 379,53 | | | | |
|---|---|---|---|---|
| | C | H | N | S |
| % calculés | 72,78 | 6,64 | 3,69 | 8,44 |
| % trouvés | 72,6 | 6,7 | 3,9 | 8,5 |

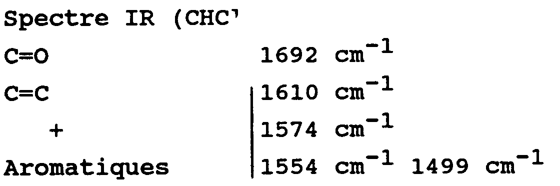

### EXEMPLE 11 : 4-[(3-butyl 4-isoquinoléinyl) méthyl] benzoate de méthyle

A une solution de 1,1 g de 4-[[(3-butyl 1-éthylthio) 4-isoquinoléinyl] méthyl] benzoate de méthyle obtenu à l'exemple 9, dans 50 cm³ de méthanol, on ajoute 5 g de Nickel de Raney (préalablement lavé au méthanol) on chauffe 2 heures au reflux, filtre le catalyseur, lave au méthanol M, évapore le filtrat jusqu'à sec. On chromatographie le résidu sur silice (éluant : hexane-acétate d'éthyle (8-2)). On obtient 680 mg du produit recherché.

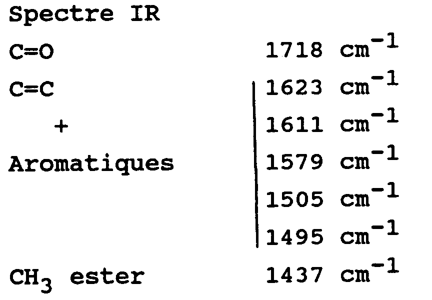

### EXEMPLE 12 : Acide 4-[(3-butyl 4-isoquinoléinyl) méthyl] benzoïque

On opère comme à l'exemple 2 à partir de 680 mg de produit obtenu à l'exemple 11. Après recristallisation dans l'éthanol, on obtient 500 mg de produit recherché. F = 212°C.

| Analyse pour C₂₁H₂₁NO₂ = 319,407 | | | |
|---|---|---|---|
| | C | H | N |
| % calculés | 78,97 | 6,63 | 4,38 |
| % trouvés | 79,3 | 6,5 | 4,3 |

### Spectre IR (Nujol) Absorption région OH/NH très associée

- C=C: 1624 - 1612 - 1588 - 1580 cm⁻¹

### EXEMPLE 13 : composition pharmaceutique

On a préparé des comprimés répondant à la formule suivante :

| | |
|---|---|
| Produit de l'exemple 12 | 10 mg |
| Excipient pour un comprimé terminé à | 100 mg |
| (détail de l'excipient : lactose, talc, amidon, stéarate de magnésium). | |

## Revendications (Revendications pour l'(les) Etat(s) contractant(s) suivant(s): AT, BE, CH, LI, DE, DK, FR, GB, IT, LU, NL, SE)

1. Produits de formule (I_{B}) : 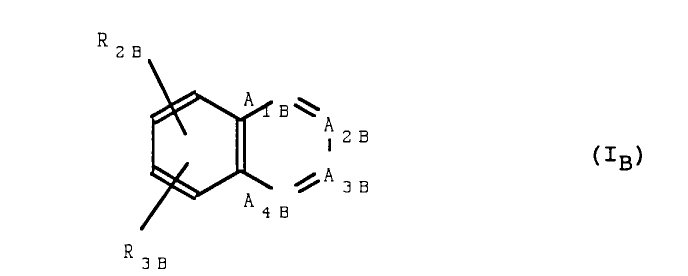 dans laquelle :
R_{2B} et R_{3B}, identiques ou différents, sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkényl, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole, isoxazole, pyrrolidinyle, pyrrolidinylcarbonyle et phényle éventuellement substitué par un ou plusieurs radicaux choisis parmi les atomes d'halogène, le radical hydroxyle, les radicaux alkyle et alkoxy renfermant au plus 4 atomes de carbone,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pyrrolidinylcarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{1B}, A_{2B}, A_{3B} et A_{4B} sont tels que :
- l'un représente un atome d'azote,
- un autre représente un atome d'azote ou le radical méthine substitué par R_{4b},
- un autre représente le radical méthine substitué par R_{4c},
- et le dernier représente le radical méthine substitué par le radical benzyle lui-même substitué par un ou plusieurs radicaux choisis parmi les radicaux cyano, carboxy libre, salifié et estérifié,
R_{4b} et R_{4c} identiques ou différents, sont choisis parmi les valeurs de R_{4B},
tel que R_{4B} représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

2. Produits de formule (I_{B}) telle que définie à la revendication 1 et répondant à la formule (Iₐ) : 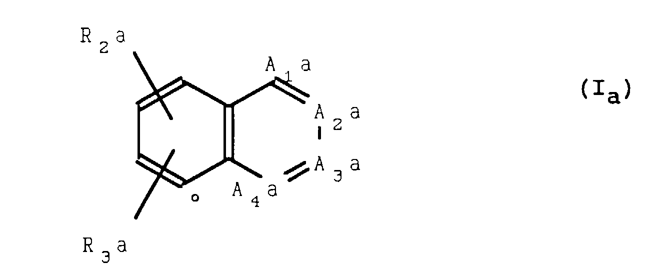 dans laquelle :
R₂ₐ et R₃ₐ, identiques ou différents, sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A₁ₐ, A₂ₐ, A₃ₐ et A₄ₐ sont tels que :
- l'un représente un atome d'azote,
- un autre représente un atome d'azote ou le radical méthine substitué par R_{4ab},
- un autre représente le radical méthine substitué par R_{4ac},
- et le dernier représente le radical méthine substitué par le radical benzyle lui-même substitué par un ou plusieurs radicaux choisis parmi les radicaux cyano, carboxy libre, salifié et estérifié,
R_{4ab} et R_{4ac} identiques ou différents, sont choisis parmi les valeurs de R₄ₐ,
tel que R₄ₐ représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ).

3. Produits de formule (I_{B}) telle que définie aux revendications 1 et 2 dans laquelle :
R_{2B} et R_{3B} représentent un atome d'hydrogène,
A_{1B}, A_{2B} et A_{3B} sont tels que l'un ou deux d'entre eux représentent un atome d'azote, et les autres, identiques ou différents, représentent le radical = C - R_{4B} tel que R_{4B} est choisi parmi l'atome d'hydrogène, le radical n-butyle et alkylthio renfermant au plus 4 atomes de carbone,
et A_{4B} représente le radical méthine substitué par le radical benzyle lui-même substitué par un ou plusieurs radicaux choisis parmi les radicaux cyano, carboxy libre, salifié et estérifié,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

4. Procédé de préparation de produits de formule (I_{B}) telle que définie à la revendication 1 caractérisé en ce que :
a) soit l'on soumet le composé de formule (III) : 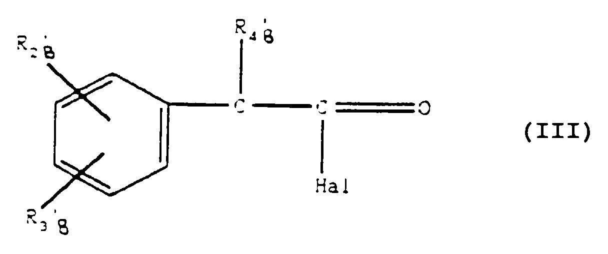 dans laquelle R_{2B}', R_{3B}' et R_{4B}' ont les significations indiquées à la revendication 1 respectivement pour R_{2B}, R_{3B} et R_{4B} dans lesquelles les éventuelles fonctions réactives sont éventuellement protégées par des groupements protecteurs et Hal représente un atome d'halogène,
à une réaction de substitution pour obtenir le produit de formule (IV) : 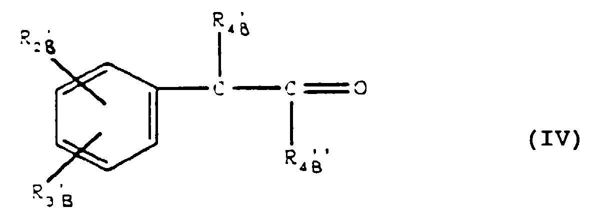 dans laquelle R_{2B}', R_{3B}' et R_{4B}' ont les significations indiquées ci-dessus et R_{4B}'', identique ou différent de R_{4B}', a la signification indiquée a la revendication 1 pour R_{4B} dans laquelle les eventuelles fonctions réactives sont éventuellement protégées par des groupements protecteurs,
que l'on fait réagir :
avec un composé de formule (V) :
R_{4B}'''-C≡N (V)
dans laquelle R_{4B}''', identique ou différent de R_{4B}' ou R_{4B}'', a la signification indiquée à la revendication 1 pour R_{4B} dans laquelle les éventuelles fonctions réactives sont éventuellement protégées par des groupements protecteurs, pour obtenir après cyclisation un produit de formule (Iᵢ) : 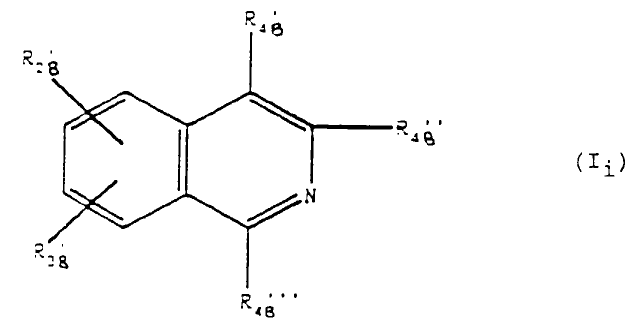 dans laquelle R_{2B}', R_{3B}', R_{4B}', R_{4B}'' et R_{4B}''' ont les significations indiquées ci-dessus,
b) soit l'on fait réagir un produit de formule (VI) : 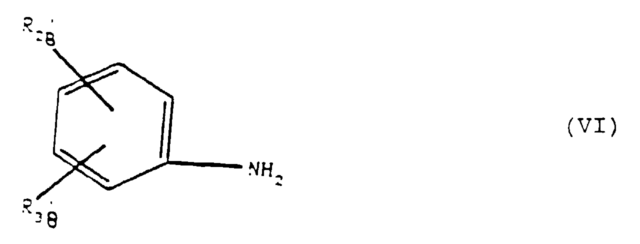 dans laquelle R_{2B}' et R_{3B}' ont les significations indiquées ci-dessus, avec un produit de formule (VII) :
R_{4B}'-CH(CO₂alk)-CO-R_{4B}'' (VII)
dans laquelle R_{4B}' et R_{4B}'', identiques ou différents, ont les significations indiquees ci-dessus, et alk représente un radical alkyle renfermant au plus 6 atomes de carbone, pour obtenir des produits de formule (VIII) : 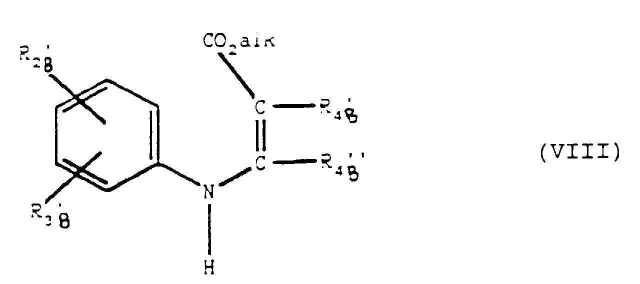 dans laquelle R_{2B}', R_{3B}', R_{4B}', R_{4B}'' et alk ont les significations indiquées ci-dessus, que l'on cyclise pour obtenir des produits de formule (II_{b}) : 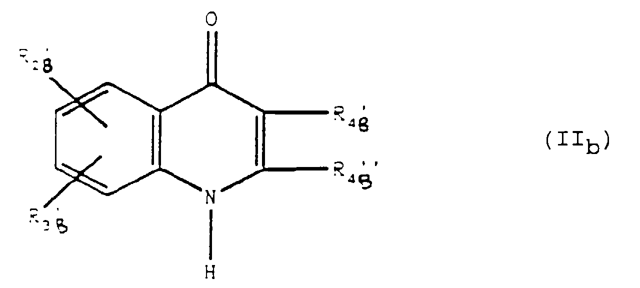 dans laquelle R_{2B}', R_{3B}', R_{4B}' et R_{4B}'' ont les significations indiquées ci-dessus,
c) soit l'on fait reagir un produit de formule (IX) : 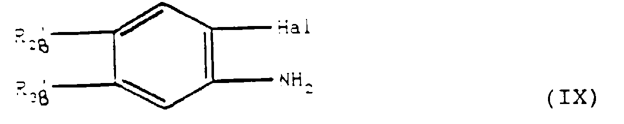 dans laquelle R_{2B}' et R_{3B}' ont les significations indiquées ci-dessus, avec un produit de formule (X) :
R_{4B}'''-C≡CH (X)
dans laquelle R_{4B}''' a la signification indiquée ci-dessus, pour obtenir des produits de formule (XI) : 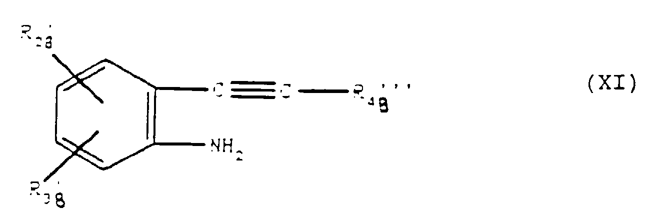 dans laquelle R_{2B}', R_{3B}' et R_{4B}''' ont les significations indiquées ci-dessus, que l'on soumet à une réaction de cyclisation en présence d'un donneur d'azote tel que le nitrite de sodium, pour obtenir un produit de formule (II_{c}) : 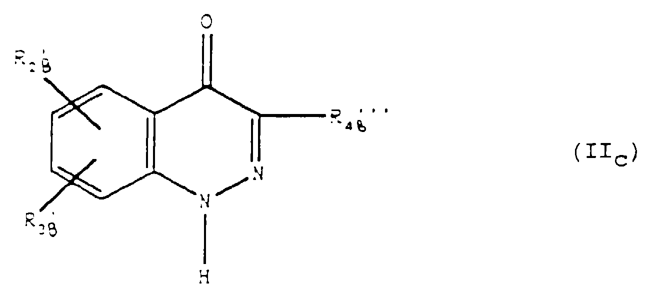 dans laquelle R_{2B}', R_{3B}' et R_{4B}''' ont les significations indiquées ci-dessus,
d) soit l'on fait réagir un produit de formule (XII) : 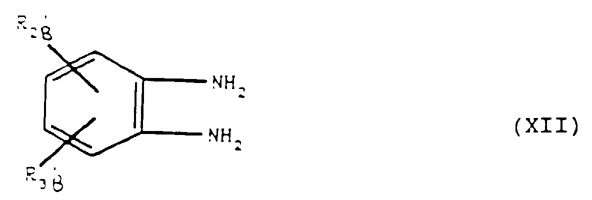 dans laquelle R_{**2B**}' et R_{**3B**}' ont les significations indiquées ci-dessus, avec un produit de formule (XIII) :
R_{**4B**}'-CO-CO_{**2**}alk (XIII)
dans laquelle R_{**4B**}' a la signification indiquée ci-dessus, et alk représente un radical alkyle renfermant au plus 6 atomes de carbone, pour obtenir après cyclisation des produits de formule (II_{**d**}) : 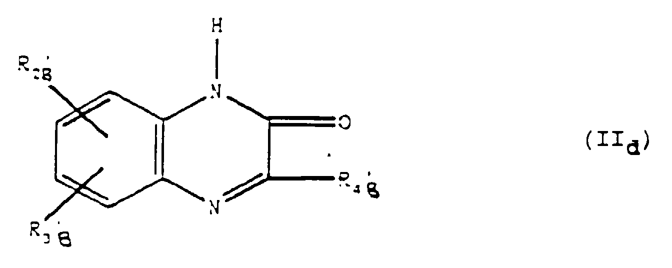 dans laquelle R_{**2B**}', R_{**3B**}' et R_{**4B**}' ont les significations indiquées ci-dessus,
e) soit l'on soumet un produit de formule (XIV) : 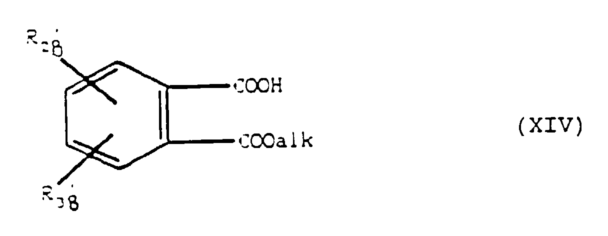 dans laquelle R_{**2B**}', R_{**3B**}' et alk ont les significations indiquées ci-dessus, après, si désiré, une réaction d'halogénation de la fonction carboxy libre, à une réaction d'addition sur cette fonction carboxy d'un composé de formule R_{**4B**}'-H, R_{**4B**}' ayant la signification indiquée ci-dessus, pour obtenir après cyclisation en présence d'hydrazine ou d'un dérivé des produits de formule (II_{**e**}) : 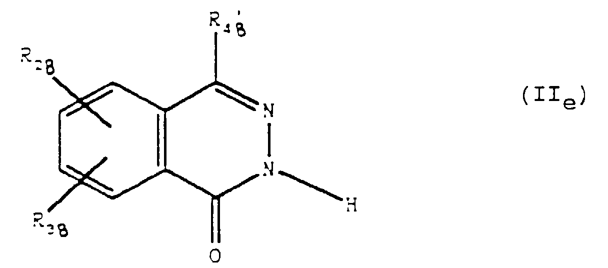 dans laquelle R_{2B}', R_{3B}' et R_{4B}' ont les significations indiquées ci-dessus,
f) soit l'on fait réagir un produit de formule (XV) : 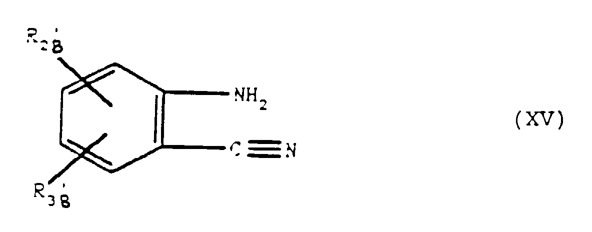 dans laquelle R_{2B}' et R_{3B}' ont les significations indiquées ci-dessus, avec un produit de formule (XVI) :
R_{4B}'-CO-Cl (XVI)
dans laquelle R_{4B}' a la signification indiquée ci-dessus, pour donner le produit de formule (XVII) : 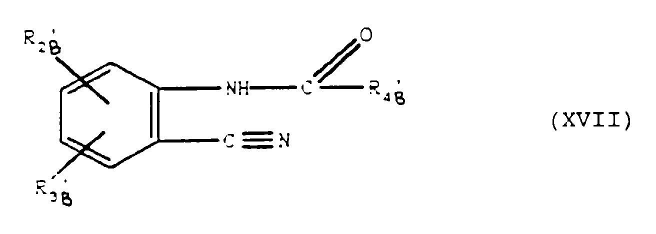 dans laquelle R_{2B}', R_{3B}' et R_{4B}' ont les significations indiquées précédemment, pour obtenir, après cyclisation, des produits de formule (II_{f}) : 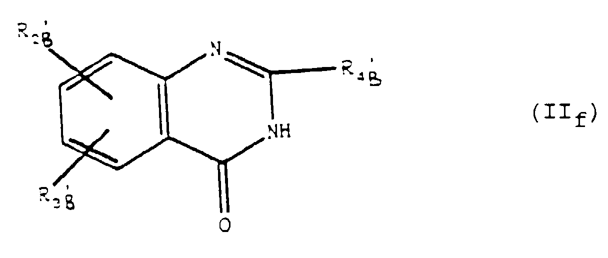 dans laquelle R_{2B}', R_{3B}' et R_{4B}' ont les significations indiquées ci-dessus,
produits de formule (Iᵢ) qui peuvent représenter des produits de formule (I_{B}) et produits de formules (II_{b}), (II_{c}), (II_{d}), (IIₑ) et (II_{f}) telles que définies ci-dessus qui peuvent représenter des produits de formule (I_{B}) dans laquelle l'un au moins de A_{1B}, A_{2B}, A_{3B} et A_{4B} représente un radical méthine portant un radical hydroxyle, que l'on soumet, si désiré et si nécessaire, à l'une ou plusieurs des réactions suivantes, dans un ordre quelconque :
- une réaction de réduction complète du radical hydroxyle ou oxo en radical méthine suivie d'une aromatisation,
- sur les produits de formule (Iᵢ) dans lesquels l'un de R_{4B}', R_{4B}'' ou R_{4B}''' représente un radical hydroxyle ou sur les produits de formules (II_{b}), (II_{c}), (II_{d}), (IIₑ) et (II_{f}),
**soit** d'abord à une réaction de substitution du radical hydroxyle par un atome d'halogéne suivie de l'action d'un produit de formule Rₚ₄ - M - Hal dans laquelle Rₚ₄ a la signification indiquée à la revendication 1 pour R_{4B} dans lesquelles les éventuelles fonctions reactives sont éventuellement protégées par des groupements protecteurs, M représente un atome de metal choisi parmi le magnésium, le cuivre et le zinc et Hal représente un atome d'halogène,
**soit** à l'action d'un produit de formule Rₚ₄ - Hal dans laquelle Hal représente un atome d'halogène, pour obtenir les produits de formule (I_{B}) correspondants,
- une réaction de transformation de fonction oxo (= O) en fonction thioxo (= S),
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
lesdits produits de formule (I_{B}) ainsi obtenus étant sous toutes les formes isomères possibles racémiques, énantiomères et diastéréoisomères.

5. A titre de médicaments, les produits tels que définis par la formule (I_{B}) de la revendication 1, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I_{B}).

6. A titre de médicaments, les produits de formule (Iₐ) tels que définis à la revendication 2, ainsi que les sels d'addition avec les acides minéraux et organiques ou avec les bases minérales et organiques pharmaceutiquement acceptables desdits produits de formule (Iₐ).

7. Les compositions pharmaceutiques contenant à titre de principe actif, l'un au moins des médicaments tels que définis à l'une quelconque des revendications 5 et 6.

8. A titre de produits industriels nouveaux, les composés de formules (VIII), (II_{b}), (II_{c}), (II_{d}), (IIₑ) et (II_{f}) telles que définies à la revendication 4 à l'exception des composés suivants :
a) les composés de formule (VIII) dans lesquels R_{2'B} et R₃'_{B} = H, R₄'_{B} = CO₂Et, Me, Et, CF₃, H et R4''_{B} = CO₂Et ;
ainsi que le composé :
2-[1-[2-(trifluorométhyl) phényl amino]pent-1-ylidène]propanedioate d'éthyle.
b) les composés de formule (II_{b}) dans lesquels R₄'_{B} = H, Hal, alkyle de 1 à 4 C et R4''_{B} = CF₃ ;
ainsi que le composé :
2-butyl 4-hydroxy 8-trifluorométhyl 3-quinoléine carboxylate d'éthyle
c) les composés de formule (II_{c}) dans lesquels R'_{2B} et R'_{3B} représentent l'un H et l'autre phényle et R₄'''_{B} représente carboxy libre ou estérifié ;
d) le composé de formule (IIₑ) :
4-butyle-1-phthalazinone
e) les composés de formule (II_{f}) dans lesquels R_{2'B} et R₃'_{B} représentent H, Hal, CF₃ ou alkyle et R'_{4B} représente alkyle ou CF₃.

9. Utilisation des produits de formule (I_{B}) telle que définie à la revendication 1,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}), pour la préparation de médicaments destinés :
**soit** au traitement de l'hypertension artérielle, des insuffisances cardiaques, des insuffisances rénales et dans la prévention des resténoses post-angioplastie,
**soit** au traitement de certains désordres gastro-intestinaux ou gynécologiques.

## Revendications (Revendications pour l'(les) Etat(s) contractant(s) suivant(s): ES)

1. Procédé pour préparer des produits de formule (I_{**B**}) : 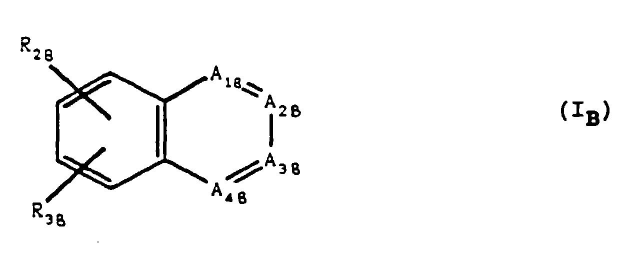 dans laquelle :
R_{**2B**} et R_{**3B**}, identiques ou différents, sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkényl, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole, isoxazole, pyrrolidinyle, pyrrolidinylcarbonyle et phényle éventuellement substitué par un ou plusieurs radicaux choisis parmi les atomes d'halogène, le radical hydroxyle, les radicaux alkyle et alkoxy renfermant au plus 4 atomes de carbone,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pyrrolidinylcarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{1B}, A_{2B}, A_{3B} et A_{4B} sont tels que :
- l'un représente un atome d'azote,
- un autre représente un atome d'azote ou le radical méthine substitué par R_{4b},
- un autre représente le radical méthine substitué par R_{4c},
- et le dernier représente le radical méthine substitué par le radical benzyle lui-même substitué par un ou plusieurs radicaux choisis parmi les radicaux cyano, carboxy libre, salifié et estérifié,
R_{4b} et R_{4c} identiques ou différents, sont choisis parmi les valeurs de R_{4B},
tel que R_{4B} représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone,
lesdits produits de formule (I_{**B**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**B**}), caractérisé en ce que :
a) soit l'on soumet le composé de formule (III) : 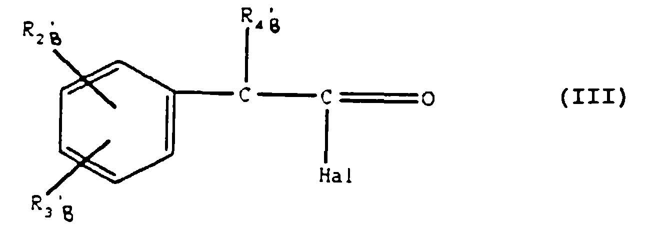 dans laquelle R_{**2B**}', R_{**3B**}' et R_{**4B**}' ont les significations indiquées ci-dessus respectivement pour R_{**2B**}, R_{**3B**} et R_{**4B**} dans lesquelles les éventuelles fonctions réactives sont éventuellement protégées par des groupements protecteurs et Hal représente un atome d'halogène,
à une réaction de substitution pour obtenir le produit de formule (IV) : 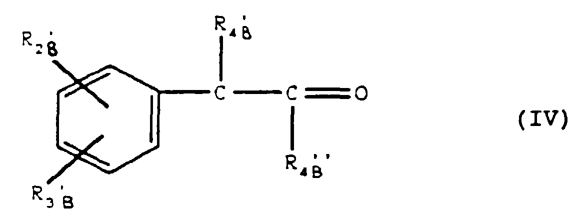 dans laquelle R_{**2B**}', R_{**3B**}' et R_{**4B**}' ont les significations indiquées ci-dessus et R_{**4B**}'', identique ou différent de R_{**4B**}', a la signification indiquée ci-dessus pour R_{**4B**} dans laquelle les éventuelles fonctions réactives sont éventuellement protégées par des groupements protecteurs, que l'on fait réagir : avec un composé de formule (V) :
R_{**4B**}'''-C≡N (V)
dans laquelle R_{**4B**}''', identique ou différent de R_{**4B**}' ou R_{**4B**}'', a la signification indiquée ci-dessus pour R_{**4B**} dans laquelle les éventuelles fonctions réactives sont éventuellement protégées par des groupements protecteurs, pour obtenir après cyclisation un produit de formule (I_{**i**}) : 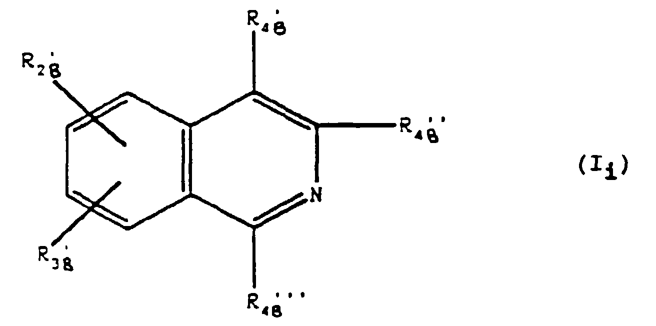 dans laquelle R_{**2B**}', R_{**3B**}', R_{**4B**}', R_{**4B**}'' et R_{**4B**}''' ont les significations indiquées ci-dessus,
b) soit l'on fait réagir un produit de formule (VI) : 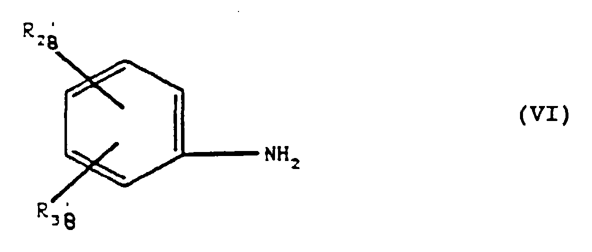 dans laquelle R_{**2B**}' et R_{**3B**}' ont les significations indiquées ci-dessus, avec un produit de formule (VII) :
R_{**4B**}'-CH(CO_{**2**}alk)-CO-R_{**4B**}'' (VII)
dans laquelle R_{**4B**}' et R_{**4B**}'', identiques ou différents, ont les significations indiquées ci-dessus, et alk représente un radical alkyle renfermant au plus 6 atomes de carbone, pour obtenir des produits de formule (VIII) : 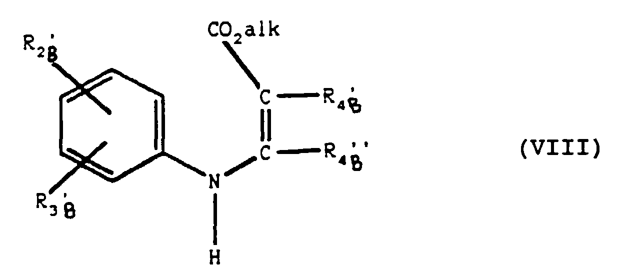 dans laquelle R_{**2B**}', R_{**3B**}', R_{**4B**}', R_{**4B**}'' et alk ont les significations indiquées ci-dessus, que l'on cyclise pour obtenir des produits de formule (II_{**b**}) : 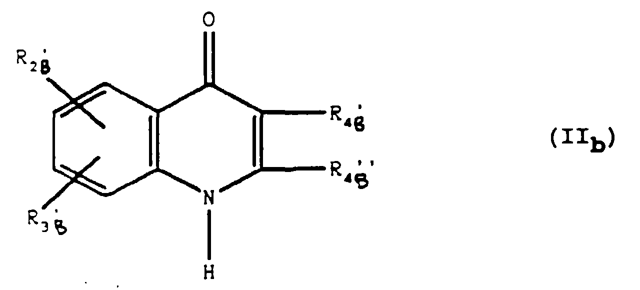 dans laquelle R_{**2B**}', R_{**3B**}', R_{**4B**}' et R_{**4B**}'' ont les significations indiquées ci-dessus,
c) soit l'on fait réagir un produit de formule (IX) : 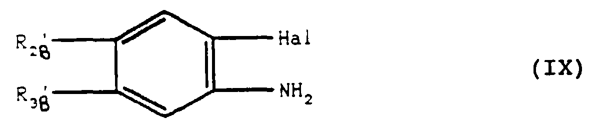 dans laquelle R_{**2B**}' et R_{**3B**}' ont les significations indiquées ci-dessus, avec un produit de formule (X) :
R_{**4B**}'''-C≡CH (X)
dans laquelle R_{**4B**}''' a la signification indiquée ci-dessus, pour obtenir des produits de formule (XI) : 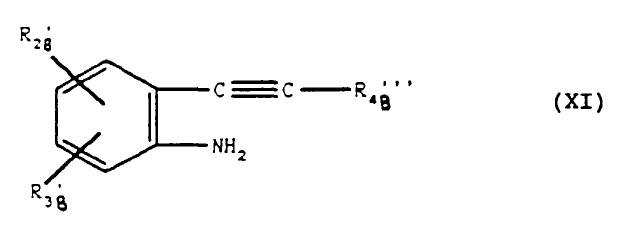 dans laquelle R_{**2B**}', R_{**3B**}' et R_{**4B**}''' ont les significations indiquées ci-dessus, que l'on soumet à une réaction de cyclisation en présence d'un donneur d'azote tel que le nitrite de sodium, pour obtenir un produit de foule (II_{**c**}) : 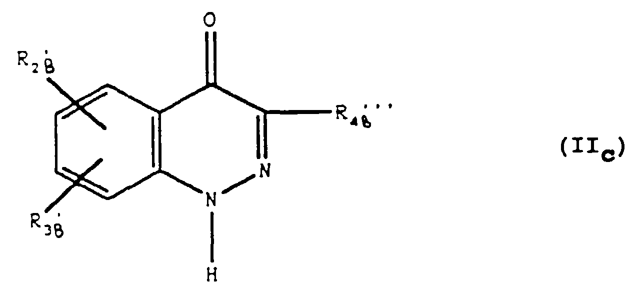 dans laquelle R_{**2B**}', R_{**3B**}' et R_{**4B**}''' ont les significations indiquées ci-dessus,
d) soit l'on fait réagir un produit de formule (XII) : 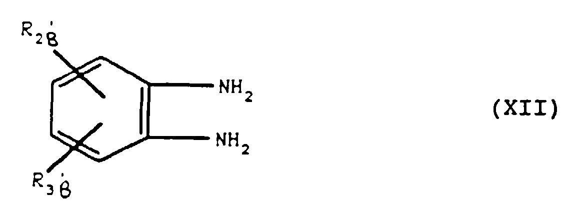 dans laquelle R_{**2B**}' et R_{**3B**}' ont les significations indiquées ci-dessus, avec un produit de formule (XIII) :
R_{**4B**}'-CO-CO₂alk (XIII)
dans laquelle R_{**4B**}' a la signification indiquée ci-dessus, et alk représente un radical alkyle renfermant au plus 6 atomes de carbone, pour obtenir après cyclisation des produits de formule (II_{**d**}) : 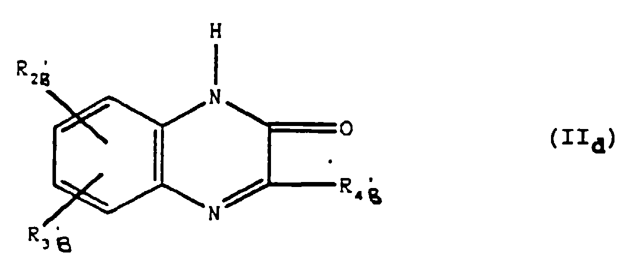 dans laquelle R_{**2B**}', R_{**3B**}' et R_{**4B**}' ont les significations indiquées ci-dessus,
e) soit l'on soumet un produit de formule (XIV) : 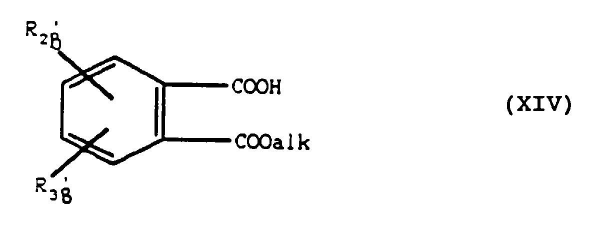 dans laquelle R_{**2B**}', R_{**3B**}' et alk ont les significations indiquées ci-dessus, après, si désiré, une réaction d'halogénation de la fonction carboxy libre, à une réaction d'addition sur cette fonction carboxy d'un composé de formule R_{**4B**}'-H, R_{**4B**}' ayant la signification indiquée ci-dessus, pour obtenir après cyclisation en présence d'hydrazine ou d'un dérivé des produits de formule (II_{**e**}) : 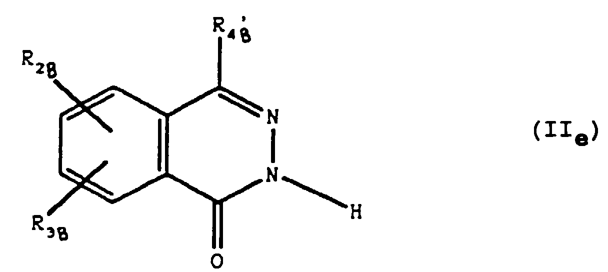 dans laquelle R_{**2B**}', R_{**3B**}' et R_{**4B**}' ont les significations indiquées ci-dessus,
f) soit l'on fait réagir un produit de formule (XV) : 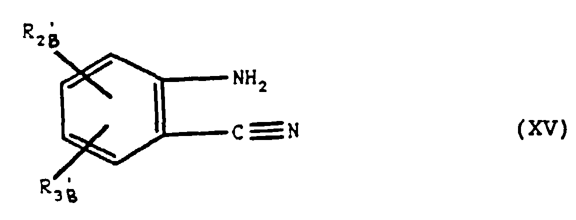 dans laquelle R_{**2B**}' et R_{**3B**}' ont les significations indiquées ci-dessus, avec un produit de formule (XVI) :
R_{**4B**}'-CO-Cl (XVI)
dans laquelle R_{**4B**}' a la signification indiquée ci-dessus, pour donner le produit de formule (XVII) : 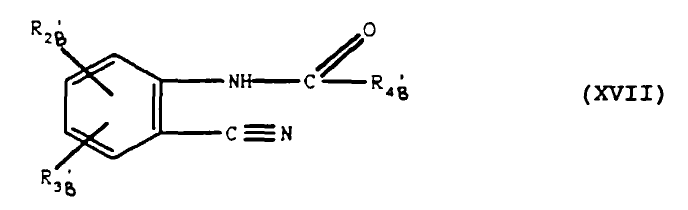 dans laquelle R_{**2B**}', R_{**3B**}' et R_{**4B**}' ont les significations indiquées précédemment, pour obtenir, après cyclisation, des produits de formule (II_{**f**}) : 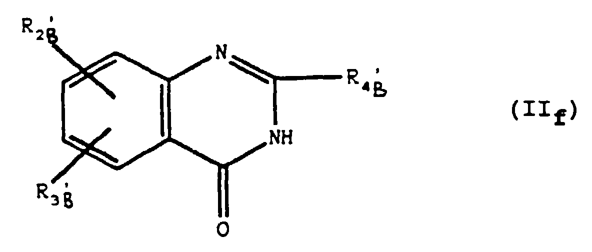 dans laquelle R_{**2B**}', R_{**3B**}' et R_{**4B**}' ont les significations indiquées ci-dessus,
produits de formule (I_{**i**}) qui peuvent représenter des produits de formule (I_{**B**}) et produits de formules (II_{**b**}), (II_{**c**}), (II_{**d**}), (II_{**e**}) et (II_{**f**}) telles que définies ci-dessus qui peuvent représenter des produits de formule (I_{**B**}) dans laquelle l'un au moins de A_{**1B**}, A_{**2B**}, A_{**3B**} et A_{**4B**} représente un radical méthine portant un radical hydroxyle, que l'on soumet, si désiré et si nécessaire, à l'une ou plusieurs des réactions suivantes, dans un ordre quelconque :
- une réaction de réduction complète du radical hydroxyle ou oxo en radical méthine suivie d'une aromatisation,
- sur les produits de formule (I_{**i**}) dans lesquels l'un de R_{**4B**}', R_{**4B**}'' ou R_{**4B**}''' représente un radical hydroxyle ou sur les produits de formules (II_{**b**}), (II_{**c**}), (II_{**d**}), (II_{**e**}) et (II_{**f**}), soit d'abord à une réaction de substitution du radical hydroxyle par un atome d'halogène suivie de l'action d'un produit de formule R_{**p4**} - M - Hal dans laquelle R_{**p4**} a la signification indiquée à la revendication 1 pour R_{**4B**} dans lesquelles les éventuelles fonctions réactives sont éventuellement protégées par des groupements protecteurs, M représente un atome de métal choisi parmi le magnésium, le cuivre et le zinc et Hal représente un atome d'halogène,
**soit** à l'action d'un produit de formule R_{**p4**} - Hal dans laquelle Hal représente un atome d'halogène,
pour obtenir les produits de formule (I_{**B**}) correspondants,
- une réaction de transformation de fonction oxo (= O) en fonction thioxo (= S),
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
lesdits produits de formule (I_{**B**}) ainsi obtenus étant sous toutes les formes isomères possibles racémiques, énantiomères et diastéréoisomères.

2. Procédé selon la revendication 1 pour préparer des produits de formule (I_{**B**}) telle que définie à la revendication 1 et répondant à la formule (I_{**a**}) : 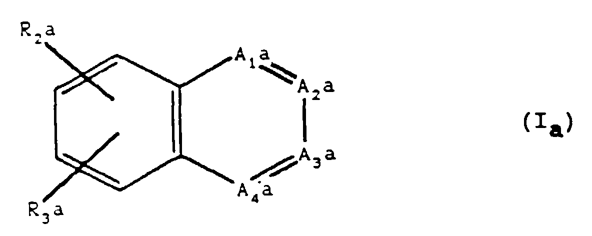 dans laquelle :
R_{**2a**} et R_{**3a**}, identiques ou différents, sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{**1a**}, A_{**2a**}, A_{**3a**} et A_{**4a**} sont tels que :
- l'un représente un atome d'azote,
- un autre représente un atome d'azote ou le radical méthine substitué par R_{**4ab**},
- un autre représente le radical méthine substitué par R_{**4ac**},
- et le dernier représente le radical méthine substitué par le radical benzyle lui-même substitué, par un ou plusieurs radicaux choisis parmi les radicaux cyano, carboxy libre, salifié ou esterifié
R_{**4ab**} et R_{**4ac**} identiques ou différents, sont choisis parmi les valeurs de R_{**4a**},
tel que R_{**4a**} représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, étant entendu que les produits de formule (I_{**a**}) ne peuvent pas représenter les produits suivants :
ou bien R_{**2a**} et R_{**3a**} sont choisis parmi les atomes d'halogène, les radicaux hydroxyle, alkyle et alkyloxy et A_{**1a**}, A_{**2a**}, A_{**3a**} et A_{**4a**} sont tels que :
- l'un représente l'atome d'azote,
- un autre représente le radical méthine ou l'atome d'azote,
- un autre représente le radical méthine, ces deux radicaux méthine étant éventuellement substitués par un radical choisi parmi les radicaux carboxy libre ou estérifié, benzoyle, phényle éventuellement substitué par un radical alkyle renfermant au plus deux atomes de carbone lui-même éventuellement substitué par un radical hydroxyle ou acyloxy,
- et le dernier représente le radical benzyle éventuellement substitué par un ou plusieurs radicaux choisis parmi les atomes d'halogène, les radicaux alkyle, alkyloxy, amino, nitro et acétyle,
ou bien R_{**2a**} et R_{**3a**} identiques représentent le radical méthoxy, A_{**1a**} représente le radical benzyle substitué par un atome de chlore, A_{**2a**} représente le radical méthine substitué par le radical isopropyle ou isobutyle, A_{**3a**} représente l'atome d'azote et A_{**4a**} représente le radical méthine, lesdits produits de formule (I_{**a**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**a**}), caractérisé en ce que l'on choisit les produits de départ et les réactifs de manière telle que l'on prépare les produits de formule (I_{**a**}) telle que définie ci-dessus.

3. Procédé selon la revendication 1 pour préparer des produits de formule (I_{**B**}) dans laquelle :
R_{**2B**} et R_{**3B**} représentent un atome d'hydrogène,
A_{**1B**}, A_{**2B**} et A_{**3B**} sont tels que l'un ou deux d'entre eux représentent un atome d'azote, et les autres, identiques ou différents, représentent le radical = 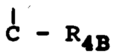 tel que R_{**4B**} est choisi parmi l'atome d'hydrogène, le radical n-butyle et alkylthio renfermant au plus 4 atomes de carbone,
et A_{**4B**} représente le radical méthine substitué par le radical benzyle éventuellement substitué par un ou plusieurs radicaux choisis parmi les radicaux cyano, carboxy libre, salifié et estérifié,
lesdits produits de formule (I_{**B**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**B**}), caractérisé en ce que l'on choisit les produits de départ et les réactifs de manière telle que l'on prépare les produits de formule (I_{**B**}) telle que définie ci-dessus.

## Revendications (Revendications pour l'(les) Etat(s) contractant(s) suivant(s): GR)

1. Procédé pour préparer des produits de formule (I_{**B**}) : 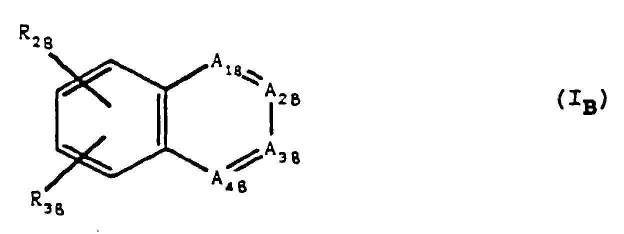 dans laquelle :
R_{**2B**} et R_{**3B**}, identiques ou différents, :
dans lesquels : sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkényl, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole, isoxazole, pyrrolidinyle, pyrrolidinylcarbonyle et phényle éventuellement substitué par un ou plusieurs radicaux choisis parmi les atomes d'halogène, le radical hydroxyle, les radicaux alkyle et alkoxy renfermant au plus 4 atomes de carbone,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pyrrolidinylcarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{1B}, A_{2B}, A_{3B} et A_{4B} sont tels que :
- l'un représente un atome d'azote,
- un autre représente un atome d'azote ou le radical méthine substitué par R_{4b},
- un autre représente le radical méthine substitué par R_{4c},
- et le dernier représente le radical méthine substitué par le radical benzyle lui-même substitué par un ou plusieurs radicaux choisis parmi les radicaux cyano, carboxy libre, salifié et estérifié,
R_{4b} et R_{4c} identiques ou différents, sont choisis parmi les valeurs de R_{4B},
tel que R_{4B} représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone,
lesdits produits de formule (I_{**B**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**B**}), caractérisé en ce que :
a) soit l'on soumet le composé de formule (III) : 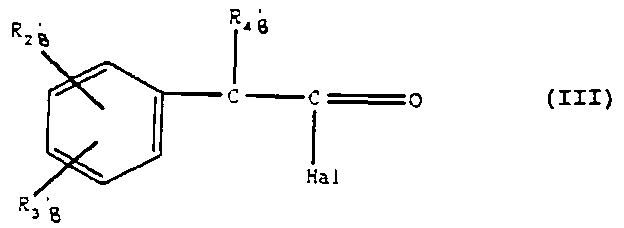 dans laquelle R_{**2B**}', R_{**3B**}' et R_{**4B**}' ont les significations indiquées ci-dessus respectivement pour R_{**2B**}, R_{**3B**} et R_{**4B**} dans lesquelles les éventuelles fonctions réactives sont éventuellement protégées par des groupements protecteurs et Hal représente un atome d'halogène,
à une réaction de substitution pour obtenir le produit de formule (IV) : 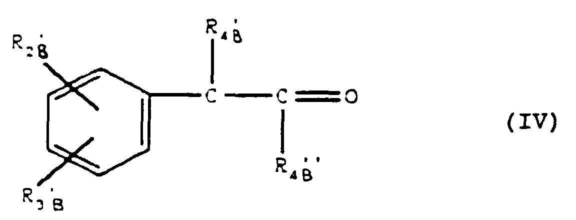 dans laquelle R_{**2B**}', R_{**3B**}' et R_{**4B**}' ont les significations indiquées ci-dessus et R_{**4B**}'', identique ou différent de R_{**4B**}', a la signification indiquée ci-dessus pour R_{**4B**} dans laquelle les éventuelles fonctions réactives sont éventuellement protégées par des groupements protecteurs, que l'on fait réagir : avec un composé de formule (V) :
R_{**4B**}'''-C≡N (V)
dans laquelle R_{**4B**}''', identique ou différent de R_{**4B**}' ou R_{**4B**}'', a la signification indiquée ci-dessus pour R_{**4B**} dans laquelle les éventuelles fonctions réactives sont éventuellement protégées par des groupements protecteurs, pour obtenir après cyclisation un produit de formule (I_{**i**}) : 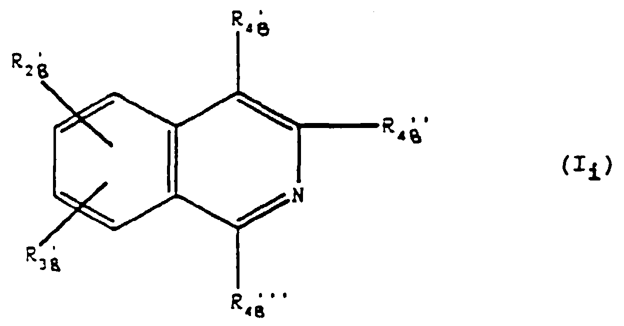 dans laquelle R_{**2B**}**'**, R_{**3B**}', R_{**4B**}', R_{**4B**}'' et R_{**4B**}''' ont les significations indiquées ci-dessus,
b) soit l'on fait réagir un produit de formule (VI) : 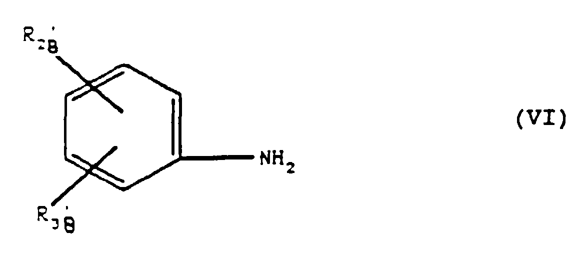 dans laquelle R_{**2B**}' et R_{**3B**}' ont les significations indiquées ci-dessus, avec un produit de formule (VII) :
R_{**4B**}'-CH(CO₂alk)-CO-R_{**4B**}'' (VII)
dans laquelle R_{**4B**}' et R_{**4B**}'', identiques ou différents, ont les significations indiquées ci-dessus, et alk représente un radical alkyle renfermant au plus 6 atomes de carbone, pour obtenir des produits de formule (VIII) : 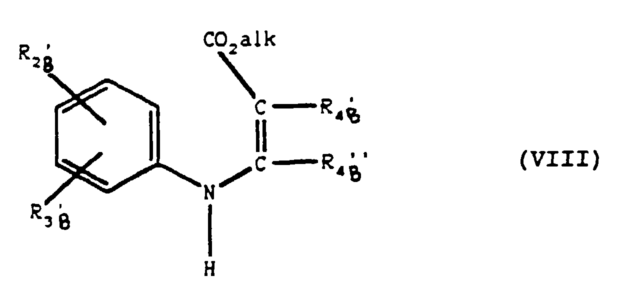 dans laquelle R_{**2B**}', R_{**3B**}', R_{**4B**}', R_{**4B**}'' et alk ont les significations indiquées ci-dessus, que l'on cyclise pour obtenir des produits de formule (II_{**b**}) : 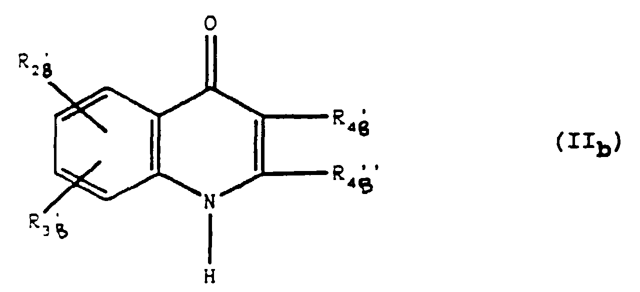 dans laquelle R_{**2B**}', R_{**3B**}', R_{**4B**}' et R_{**4B**}'' ont les significations indiquées ci-dessus,
c) soit l'on fait réagir un produit de formule (IX) : 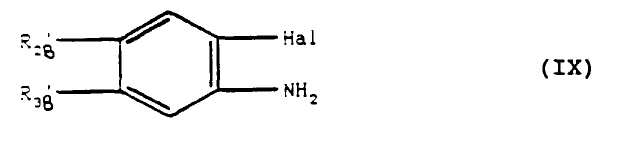 dans laquelle R_{**2B**}' et R_{**3B**}' ont les significations indiquées ci-dessus, avec un produit de formule (X) :
R_{**4B**}**'**''-C≡CH (X)
dans laquelle R_{**4B**}''' a la signification indiquée ci-dessus, pour obtenir des produits de formule (XI) : 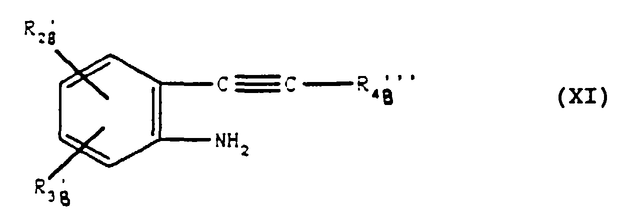 dans laquelle R_{**2B**}', R_{**3B**}' et R_{**4B**}''' ont les significations indiquées ci-dessus, que l'on soumet à une réaction de cyclisation en présence d'un donneur d'azote tel que le nitrite de sodium, pour obtenir un produit de formule (II_{**c**}) : 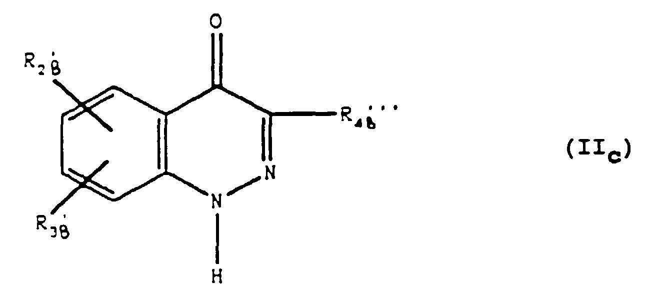 dans laquelle R_{**2B**}', R_{**3B**}' et R_{**4B**}''' ont les significations indiquées ci-dessus,
d) soit l'on fait réagir un produit de formule (XII) : 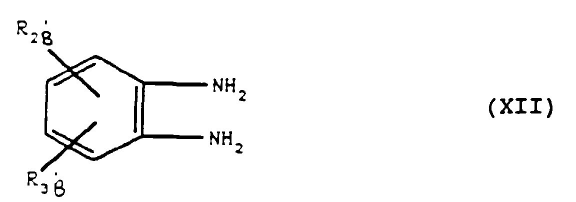 dans laquelle R_{**2B**}' et R_{**3B**}' ont les significations indiquées ci-dessus, avec un produit de formule (XIII) :
R_{**4B**}'-CO-CO_{**2**}alk (XIII)
dans laquelle R_{**4B**}' a la signification indiquée ci-dessus, et alk représente un radical alkyle renfermant au plus 6 atomes de carbone, pour obtenir après cyclisation des produits de formule (II_{**d**}) : 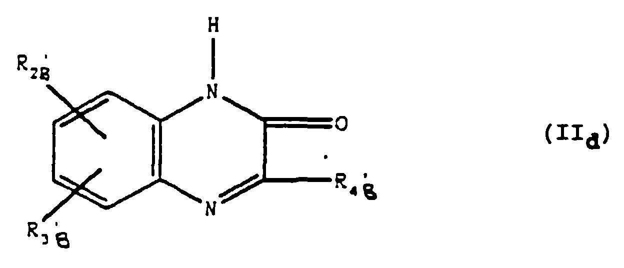 dans laquelle R_{**2B**}', R_{**3B**}' et R_{**4B**}' ont les significations indiquées ci-dessus,
e) soit l'on soumet un produit de formule (XIV) : 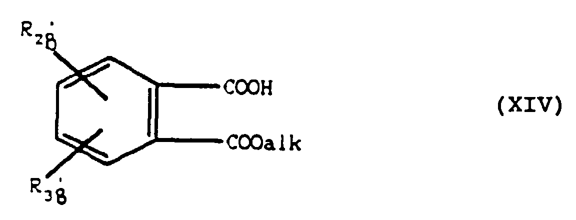 dans laquelle R_{**2B**}', R_{**3B**}' et alk ont les significations indiquées ci-dessus, après, si désiré, une réaction d'halogénation de la fonction carboxy libre, à une réaction d'addition sur cette fonction carboxy d'un composé de formule R_{**4B**}'-H, R_{**4B**}' ayant la signification indiquée ci-dessus, pour obtenir après cyclisation en présence d'hydrazine ou d'un dérivé des produits de formule (II_{**e**}) : 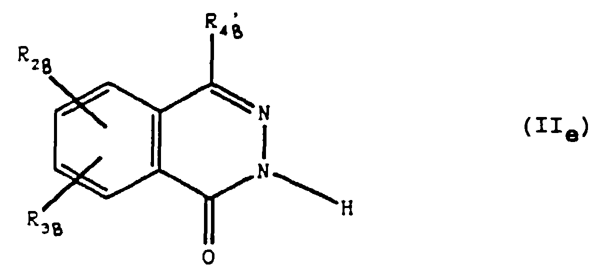 dans laquelle R_{**2B**}', R_{**3B**}' et R_{**4B**}' ont les significations indiquées ci-dessus,
f) soit l'on fait réagir un produit de formule (XV) : 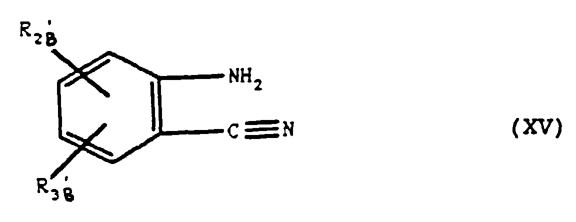 dans laquelle R_{**2B**}' et R_{**3B**}' ont les significations indiquées ci-dessus, avec un produit de formule (XVI) :
R_{**4B**}'-CO-Cl (XVI)
dans laquelle R_{**4B**}' a la signification indiquée ci-dessus, pour donner le produit de formule (XVII) : 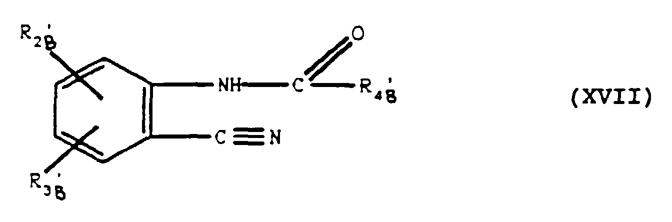 dans laquelle R_{**2B**}', R_{**3B**}' et R_{**4B**}' ont les significations indiquées précédemment, pour obtenir, après cyclisation, des produits de formule (II_{**f**}) : 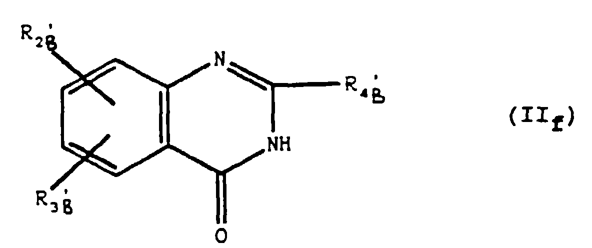 dans laquelle R_{**2B**}', R_{**3B**}' et R_{**4B**}' ont les significations indiquées ci-dessus,
produits de formule (I_{**i**}) qui peuvent représenter des produits de formule (I_{**B**}) et produits de formules (II_{**b**}), (II_{**c**}), (II_{**d**}), (II_{**e**}) et (II_{**f**}) telles que définies ci-dessus qui peuvent représenter des produits de formule (I_{**B**}) dans laquelle l'un au moins de A_{**1B**}, A_{**2B**}, A_{**3B**} et A_{**4B**} représente un radical méthine portant un radical hydroxyle, que l'on soumet, si désiré et si nécessaire, à l'une ou plusieurs des réactions suivantes, dans un ordre quelconque :
- une réaction de réduction complète du radical hydroxyle ou oxo en radical méthine suivie d'une aromatisation,
- sur les produits de formule (I_{**i**}) dans lesquels l'un de R_{**4B**}', R_{**4B**}'' ou R_{**4B**}''' représente un radical hydroxyle ou sur les produits de formules (II_{**b**}), (II_{**c**}), (II_{**d**}), (II_{**e**}) et (II_{**f**}),
**soit** d'abord à une réaction de substitution du radical hydroxyle par un atome d'halogène suivie de l'action d'un produit de formule R_{**p4**} - M - Hal dans laquelle R_{**p4**} a la signification indiquée à la revendication 1 pour R_{**4B**} dans lesquelles les éventuelles fonctions réactives sont éventuellement protégées par des groupements protecteurs, M représente un atome de métal choisi parmi le magnésium, le cuivre et le zinc et Hal représente un atome d'halogène, **soit** à l'action d'un produit de formule R_{**p4**} - Hal dans
laquelle Hal représente un atome d'halogène,
pour obtenir les produits de formule (I_{**B**}) correspondants,
- une réaction de transformation de fonction oxo (= O) en fonction thioxo (= S),
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
lesdits produits de formule (I_{**B**}) ainsi obtenus étant sous toutes les formes isomères possibles racémiques, énantiomères et diastéréoisomères.

2. Procédé selon la revendication 1 pour préparer des produits de formule (I_{**B**}) telle que définie à la revendication 1 et répondant à la formule (I_{**a**}) : 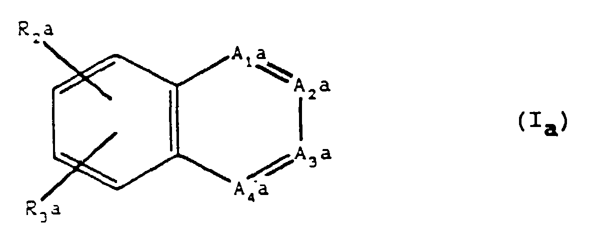 dans laquelle :
R_{**2a**} et R_{**3a**}, identiques ou différents, sont choisis dans le groupe formé par :
- l'atome d'hydrogène,
- les atomes d'halogène,
- le radical hydroxyle,
- le radical mercapto, cyano, nitro, formyle, benzoyle, acyle ayant au plus 6 atomes de carbone,
- les radicaux carboxy libre, salifié ou estérifié par un radical alkyle linéaire ou ramifié renfermant au plus 4 atomes de carbone,
- les radicaux alkyle, alkyloxy et alkylthio linéaires ou ramifiés renfermant au plus 6 atomes de carbone, phényle, naphtyle, benzyle et phénylthio, tous ces radicaux étant éventuellement substitués par un ou plusieurs radicaux identiques ou différents choisis parmi les atomes d'halogène, le radical hydroxyle, les radicaux alkyloxy renfermant au plus 4 atomes de carbone, trifluorométhyle, cyano, acyle, carboxy libre, salifié ou estérifié, tétrazole et isoxazole,
- les radicaux amino, mono- ou dialkylamino, carbamoyle, pyrrolyle, morpholino, pipérazinyle, pyrrolylméthyle, morpholinométhyle, pipérazinylméthyle, pyrrolylcarbonyle, morpholinocarbonyle, pipérazinylcarbonyle, tous les radicaux pipérazinyle de ces radicaux étant éventuellement substitués sur le second atome d'azote par un radical alkyle ou phényle, ces radicaux alkyle et phényle étant eux-mêmes éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié, tétrazole et isoxazole,
A_{**1a**}, A_{**2a**}, A_{**3a**} et A_{**4a**} sont tels que :
- l'un représente un atome d'azote,
- un autre représente un atome d'azote ou le radical méthine substitué par R_{**4ab**},
- un autre représente le radical méthine substitué par R_{**4ac**},
- et le dernier représente le radical méthine substitué par le radical benzyle lui-même substitue, par un ou plusieurs radicaux choisis parmi les radicaux cyano, carboxy libre, salifié ou esterifié
R_{**4ab**} et R_{**4ac**} identiques ou différents, sont choisis parmi les valeurs de R_{**4a**}**,**
tel que R_{**4a**} représente :
- l'atome d'hydrogène, le radical hydroxyle, cyano, carboxy libre, salifié ou estérifié par un radical alkyle renfermant au plus 4 atomes de carbone,
- le radical alkyle, alkényle, alkyloxy, acyle ou alkylthio, ces radicaux étant linéaires ou ramifiés et renfermant au plus 7 atomes de carbone, tous ces radicaux étant éventuellement substitués par un ou plusieurs radicaux choisis parmi les atomes d'halogène, les radicaux hydroxyle, nitro, alkyle, alkyloxy ou acyle renfermant au plus 4 atomes de carbone, trifluorométhyle, cyano, carboxy libre, salifié ou estérifié par un radical alkyle renfermant au plus 4 atomes de carbone, étant entendu que les produits de formule (I_{**a**}) ne peuvent pas représenter les produits suivants :
ou bien R_{**2a**} et R_{**3a**} sont choisis parmi les atomes d'halogène, les radicaux hydroxyle, alkyle et alkyloxy et A_{**1a**}, A_{**2a**}, A_{**3a**} et A_{**4a**} sont tels que :
- l'un représente l'atome d'azote,
- un autre représente le radical méthine ou l'atome d'azote,
- un autre représente le radical méthine,
ces deux radicaux méthine étant éventuellement substitués par un radical choisi parmi les radicaux carboxy libre ou estérifié, benzoyle, phényle éventuellement substitué par un radical alkyle renfermant au plus deux atomes de carbone lui-même éventuellement substitué par un radical hydroxyle ou acyloxy,
- et le dernier représente le radical benzyle éventuellement substitué par un ou plusieurs radicaux choisis parmi les atomes d'halogène, les radicaux alkyle, alkyloxy, amino, nitro et acétyle,
ou bien R_{**2a**} et R_{**3a**} identiques représentent le radical méthoxy, A_{**1a**} représente le radical benzyle substitué par un atome de chlore, A_{**2a**} représente le radical méthine substitué par le radical isopropyle ou isobutyle, A_{**3a**} représente l'atome d'azote et A_{**4a**} représente le radical méthine, lesdits produits de formule (I_{**a**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**a**}), caractérisé en ce que l'on choisit les produits de départ et les réactifs de manière telle que l'on prépare les produits de formule (I_{**a**}) telle que définie ci-dessus.

3. Procédé selon la revendication 1 pour préparer des produits de formule (I_{**B**}) dans laquelle :
R_{**2B**} et R_{**3B**} représentent un atome d'hydrogène,
A_{**1B**}, A_{**2B**} et A_{**3B**} sont tels que l'un ou deux d'entre eux représentent un atome d'azote, et les autres, identiques ou différents, représentent le radical = C - R_{**4B**} tel que R_{**4B**} est choisi parmi l'atome d'hydrogène, le radical n-butyle et alkylthio renfermant au plus 4 atomes de carbone,
et A_{**4B**} représente le radical méthine substitué par le radical benzyle éventuellement substitué par un ou plusieurs radicaux choisis parmi les radicaux cyano, carboxy libre, salifié et estérifié,
lesdits produits de formule (I_{**B**}) étant sous toutes les formes isomères possibles racémiques, énantiomères et diastéréoisomères, ainsi que les sels d'addition avec les acides minéraux et organiques ou avec les bases minérales et organiques desdits produits de formule (I_{**B**}), caractérisé en ce que l'on choisit les produits de départ et les réactifs de manière telle que l'on prépare les produits de formule (I_{**B**}) telle que définie ci-dessus.

4. Procédé de préparation de compositions pharmaceutiques, caractérisé en ce que l'on met à titre de principe actif l'un au moins des produits de formule (I_{**B**}) telle que définie à la revendication 1 sous toutes les formes isomères possibles racémiques, énantiomères et diastéréoisomères ou l'un au moins des sels d'addition avec les acides minéraux et organiques ou avec les bases minérales et organiques pharmaceutiquement acceptables desdits produits de formule (I_{**B**}) sous une forme destinée à cet usage.

5. Procédé de préparation de compositions pharmaceutiques, caractérisé en ce que l'on met à titre de principe actif l'un au moins des produits de formule (I_{**a**}) telle que définie à l'une des revendications 2 à 3 sous toutes les formes isomères possibles racémiques, énantiomères et diastéréoisomères ou l'un au moins des sels d'addition avec les acides minéraux et organiques ou avec les bases minérales et organiques pharmaceutiquement acceptables desdits produits de formule (I_{**a**}) sous une forme destinée à cet usage.

6. A titre de produits industriels nouveaux, les composés de formules (VIII), (II_{**b**}), (II_{**c**}), (II_{**d**}), (II_{**e**}) et (II_{**f**}). telles que définies à la revendication 1 à l'exception des composés suivants :
a) les composés de formule (VIII) dans lesquels R_{2'B} et R₃'_{B} = H, R₄'_{B} = CO₂Et, Me, Et, CF₃, H et R4''_{B} = CO₂Et ;
ainsi que le composé :
2-[1-[2-(trifluorométhyl) phényl amino]pent-1-ylidène]propanedioate d'éthyle.
b) les composés de formule (II_{b}) dans lesquels R₄'_{B} = H, Hal ou alkyle de 1 à 4 C et R4''_{B} = CF₃ ;
ainsi que le composé :
2-butyl 4-hydroxy 8-trifluorométhyl 3-quinoléine carboxylate d'éthyle
c) les composés de formule (II_{c}) dans lesquels R'_{2B} et R'_{3B} représentent l'un H et l'autre phényle et R₄'''_{B} représente carboxy libre ou estérifié ;
d) le composé de formule (IIₑ) :
4-butyle-1-phthalazinone
e) les composés de formule (II_{f}) dans lesquels R₂,_{B} et R₃'_{B} représentent H, Hal, CF₃ ou alkyle et R'_{4B} représente alkyle ou CF₃.

## Claims (Claims for the following Contracting State(s): AT, BE, CH, LI, DE, DK, FR, GB, IT, LU, NL, SE)

1. Products of formula (I_{B}): 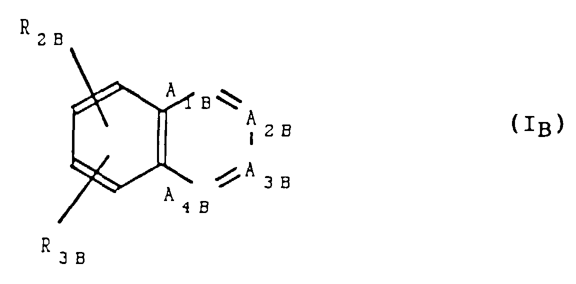 in which:
R_{2B} and R_{3B}, identical or different, are chosen from the group formed by:
- the hydrogen atom,
- halogen atoms,
- the hydroxyl radical,
- the mercapto, cyano, nitro, formyl, benzoyl, acyl radical having at most 6 carbon atoms,
- carboxy radicals free, salified or esterified by a linear or branched alkyl radical containing at most 4 carbon atoms,
- linear or branched alkyl, alkenyl, alkyloxy and alkylthio radicals containing at most 6 carbon atoms, phenyl, naphthyl, benzyl and phenylthio radicals, all these radicals being optionally substituted by one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole, isoxazole, pyrrolidinyl, pyrrolidinylcarbonyl radicals and the phenyl radical optionally substituted by one or more radicals chosen from halogen atoms, the hydroxyl radical, alkyl and alkoxy radicals containing at most 4 carbon atoms,
- the following radicals: amino, mono- or dialkylamino, carbamoyl, pyrrolyl, morpholino, piperazinyl, pyrrolylmethyl, morpholinomethyl, piperazinylmethyl, pyrrolylcarbonyl, morpholinocarbonyl, pyrrolidinylcarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, the following radicals: hydroxyl, nitro, alkyl, alkyloxy or acyl containing at most 4 carbon atoms, trifluoromethyl, cyano, free, salified or esterified carboxy, tetrazole and isoxazole,
A_{1B}, A_{2B}, A_{3B} and A_{4B} are such that:
- one represents a nitrogen atom,
- another represents a nitrogen atom or the methine radical substituted by R_{4b},
- another represents the methine radical substituted by R_{4c},
- and the last one represents the methine radical substituted by the benzyl radical itself substituted by one or more radicals chosen from cyano radicals, free, salified and esterified carboxy radicals,
- R_{4b} and R_{4c}, identical or different, are chosen from the values of R_{4B},
such that R_{4B} represents:
- the hydrogen atom, one of the following radicals: hydroxyl, cyano, carboxy free, salified or esterified by an alkyl radical containing at most 4 carbon atoms,
- the alkyl, alkenyl, alkyloxy, acyl or alkylthio radical, these radicals being linear or branched and containing at most 7 carbon atoms, all these radicals being optionally substituted by one or more radicals chosen from the halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, the said products of formula (I_{B}) being in all possible racemic, enantiomeric and diastereoisomeric isomer forms, as well as the addition salts with mineral and organic acids or mineral and organic bases of said products of formula (I_{B}).

2. Products of formula (I_{B}) as defined in claim 1 and corresponding to formula (Iₐ): 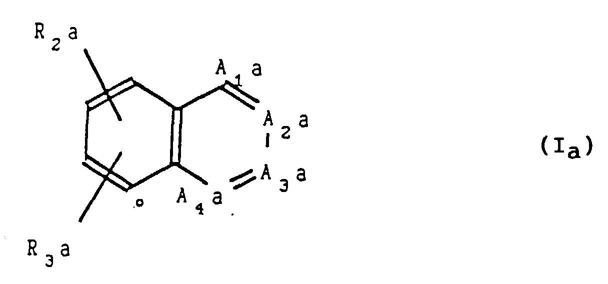 in which:
R₂ₐ and R₃ₐ, identical or different, are chosen from the group formed by:
- the hydrogen atom,
- halogen atoms,
- the hydroxyl radical,
- one of the following radicals: mercapto, cyano, nitro, formyl, benzoyl, acyl having at most 6 carbon atoms,
- carboxy radicals free, salified or esterified by a linear or branched alkyl radical containing at most 4 carbon atoms,
- linear or branched alkyl, alkyloxy and alkylthio radicals containing at most 6 carbon atoms, phenyl, naphthyl, benzyl and phenylthio radicals, all these radicals being optionally substituted by one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole and isoxazole radicals,
- the following radicals: amino, mono- or dialkylamino, carbamoyl, pyrrolyl, morpholino, piperazinyl, pyrrolylmethyl, morpholinomethyl, piperazinylmethyl, pyrrolylcarbonyl, morpholinocarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, hydroxyl, nitro radicals, alkyl, alkyloxy or acyl radicals containing at most 4 carbon atoms, trifluoromethyl, cyano radicals, free, salified or esterified carboxy radicals, tetrazole and isoxazole radicals,
A₁ₐ, A₂ₐ, A₃ₐ and A₄ₐ are such that:
- one represents a nitrogen atom,
- another represents a nitrogen atom or the methine radical substituted by R_{4ab},
- another represents the methine radical substituted by R_{4ac},
- and the last one represents the methine radical substituted by the benzyl radical itself substituted by one or more radicals chosen from cyano radicals, free, salified and esterified carboxy radicals,
- R_{4ab} and R_{4ac}, identical or different, are chosen from the values of R₄ₐ,
such that R₄ₐ represents:
- the hydrogen atom, one of the following radicals: hydroxyl, cyano, carboxy free, salified or esterified by an alkyl radical containing at most 4 carbon atoms,
- an alkyl, alkenyl, alkyloxy, acyl or alkylthio radical, these radicals being linear or branched and containing at most 7 carbon atoms, all these radicals being optionally substituted by one or more radicals chosen from halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, the said products of formula (Iₐ) being in all possible racemic, enantiomeric and diastereoisomeric isomer forms, as well as the addition salts with mineral and organic acids or mineral and organic bases of said products of formula (Iₐ).

3. Products of formula (I_{B}) as defined in claims 1 and 2 in which:
R_{2B} and R_{3B} represent a hydrogen atom,
A_{1B}, A_{2B} and A_{3B} are such that one or two of them represent a nitrogen atom, and the others, identical or different, represent the = C - R_{4B} radical such that R_{4B} is chosen from the hydrogen atom, the n-butyl radical and the alkylthio radical containing at most 4 carbon atoms,
and A_{4B} represents the methine radical substituted by the benzyl radical itself substituted by one or more radicals chosen from cyano radicals, free, salified and esterified carboxy radicals,
the said products of formulae (I_{B}) being in all possible racemic, enantiomeric and diastereoisomeric isomer forms, as well as the addition salts with mineral and organic acids or with mineral and organic bases of said products of formula (I_{B}).

4. Preparation process for products of formula (I_{B}) as defined in claim 1, characterized in that:
a) either the compound of formula (III): 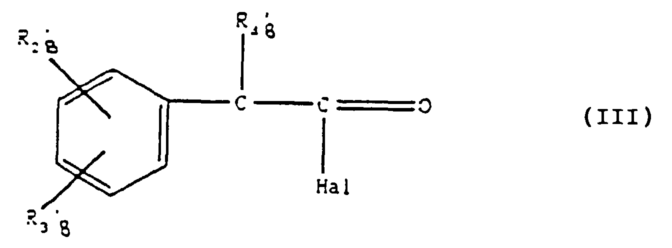 in which R_{2B'}, R_{3B'} and R_{4B'} have the meanings indicated in claim 1 for R_{2B}, R_{3B} and R_{4B} respectively in which the optional reactive functions are optionally protected by protective groups and Hal represents a halogen atom, is subjected to a substitution reaction in order to obtain the product of formula (IV): 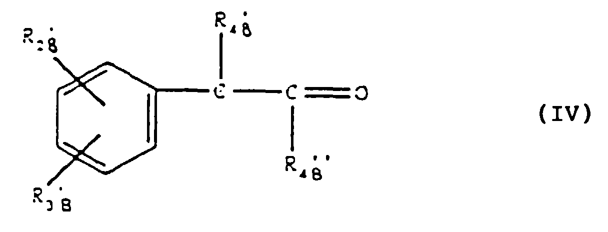 in which R_{2B'}, R_{3B'} and R_{4B'} have the meanings indicated above and R_{4B''}, identical to or different from R_{4B'}, has the meaning indicated in claim 1 for R_{4B} in which the optional reactive functions are optionally protected by protective groups,
which is reacted:
with a compound of formula (V):
R_{4B'''}-C≡N (V)
in which R_{4B'''}, identical to or different from R_{4B'} or R_{4B''}, has the meaning indicated in claim 1 for R_{4B} in which the optional reactive functions are optionally protected by protective groups, in order to obtain after cyclization a product of formula (Iᵢ): 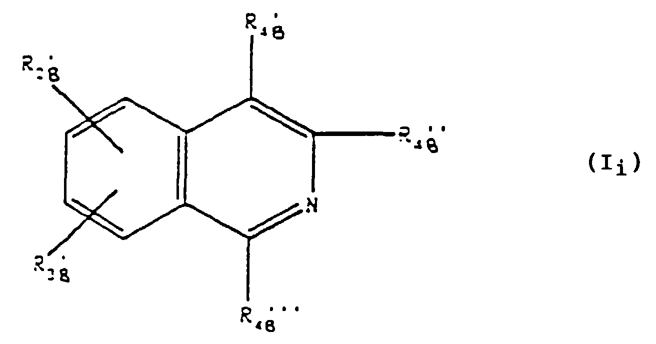 in which R_{2B'}, R_{3B'}, R_{4B'}, R_{4B''} and R_{4B'''} have the meanings indicated above,
b) or a product of formula (VI): 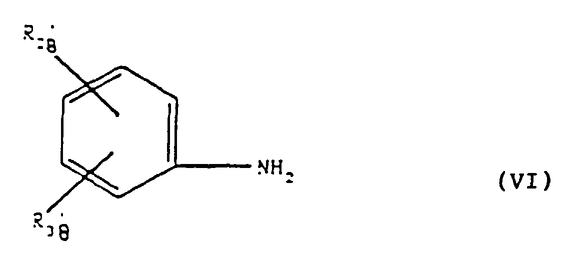 in which R_{2B'} and R_{3B'} have the meanings indicated above, is reacted with a product of formula (VII):
R_{4B'}-CH(CO₂alk)-CO-R_{4B''} (VII)
in which R_{4B'} and R_{4B''}, identical or different, have the meanings indicated above, and alk represents an alkyl radical containing at most 6 carbon atoms, in order to obtain the products of formula (VIII): 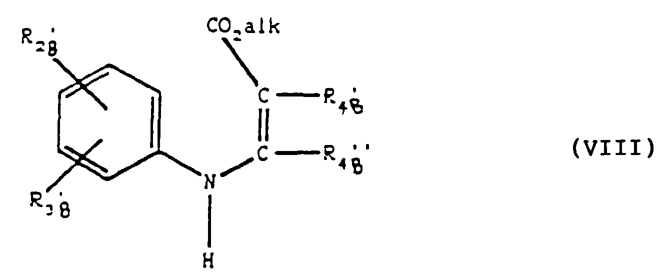 in which R_{2B'}, R_{3B'}, R_{4B'}, R_{4B''} and alk have the meanings indicated above, which are cyclized in order to obtain the products of formula (II_{b}): 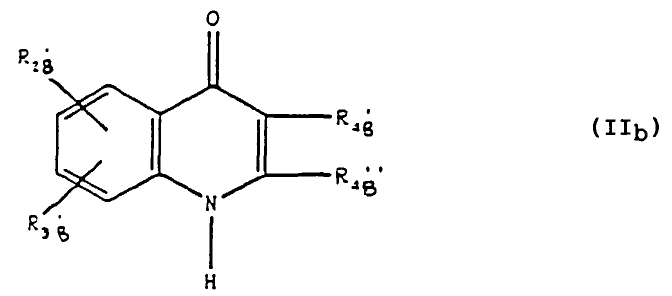 in which R_{2B'}, R_{3B'}, R_{4B'} and R_{4B''} have the meanings indicated above,
c) or a product of formula (IX): 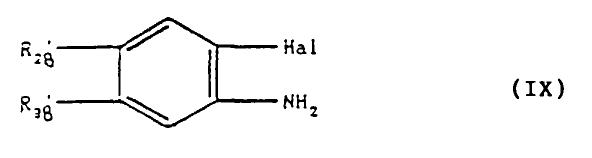 in which R_{2B'} and R_{3B'} have the meanings indicated above, is reacted with a product of formula (X):
R_{4B'''}-C≡CH (X)
in which R_{4B'''} has the meaning indicated above, in order to obtain the products of formula (XI): 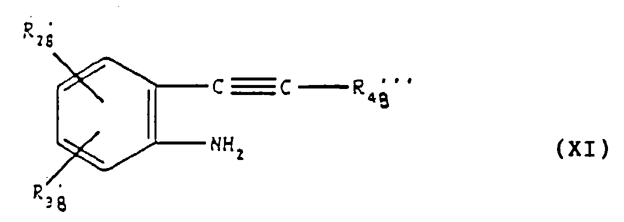 in which R_{2B'}, R_{3B'} and R_{4B'''} have the meanings indicated above, which is subjected to a cyclization reaction in the presence of a nitrogen donor such as sodium nitrite, in order to obtain a product of formula (II_{c}): 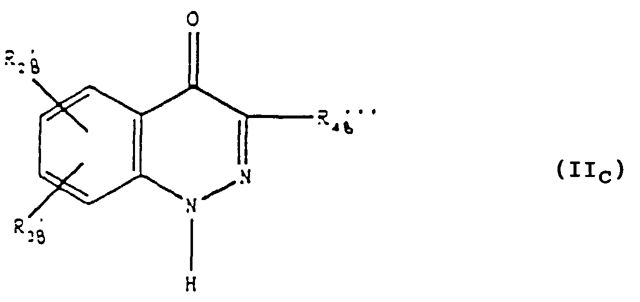 in which R_{2B'}, R_{3B'} and R_{4B'''} have the meanings indicated above,
d) or a product of formula (XII): 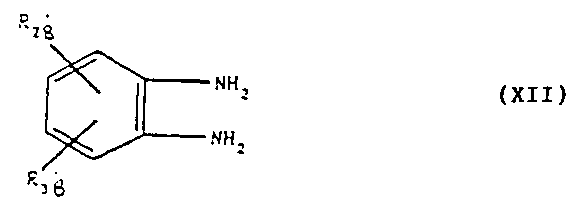 in which R_{2B}' and R_{3B}' have the meanings indicated above, is reacted with a product of formula (XIII):
R_{4B}'-CO-CO₂alk (XIII)
in which R_{4B}' has the meaning indicated above, and alk represents an alkyl radical containing at most 6 carbon atoms, in order to obtain, after cyclization, the products of formula (II_{d}): 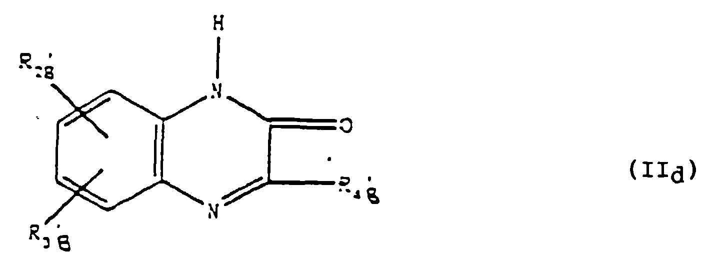 in which R_{2B}', R_{3B}' and R_{4B}' have the meanings indicated above,
e) or a product of formula (XIV): 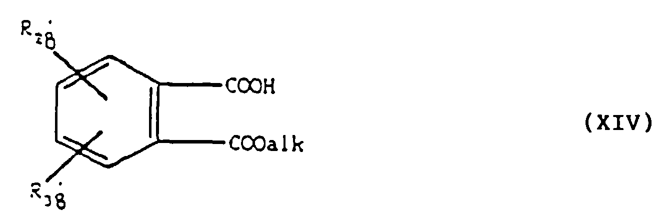 in which R_{2B}', R_{3B}' and alk have the meanings indicated above, after, if desired, a halogenation reaction of the free carboxy function, is subjected to an addition reaction on this carboxy function of a compound of formula R_{4B}'-H, R_{4B}' having the meaning indicated above, in order to obtain, after cyclization in the presence of hydrazine or a derivative, the products of formula (IIₑ): 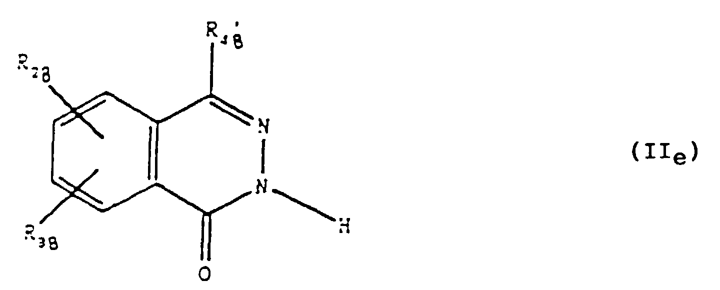 in which R_{2B}', R_{3B}' and R_{4B}' have the meanings indicated above,
f) or a product of formula (XV): 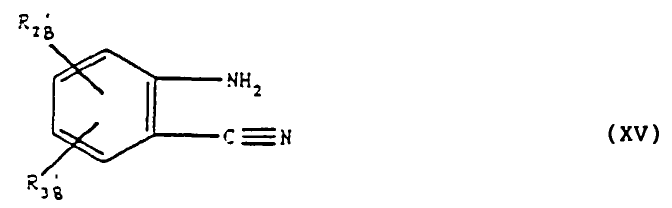 in which R_{2B}' and R_{3B}' have the meanings indicated above, is reacted with a product of formula (XVI):
R_{4B}'-CO-Cl (XVI)
in which R_{4B}' has the meaning indicated above, in order to obtain the product of formula (XVII): 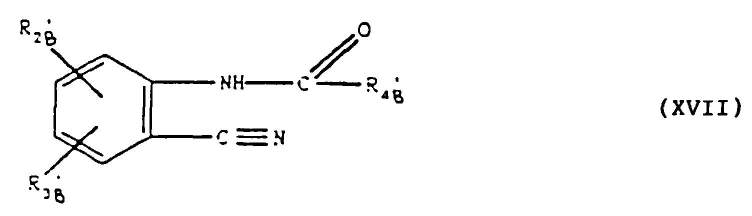 in which R_{2B}', R_{3B}' and R_{4B}' have the meanings indicated previously, in order to obtain, after cyclization, the products of formula (II_{f}): 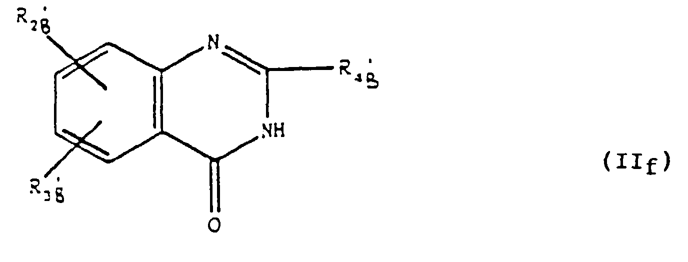
in which R_{2B}', R_{3B}' and R_{4B}' have the meanings indicated above,
which products of formula (Iᵢ) can represent products of formula (I_{B}) and products of formulae (II_{b}), (II_{c}), (II_{d}), (IIₑ) and (II_{f}) as defined above which can represent products of formula (I_{B}) in which at least one of A_{1B}, A_{2B}, A_{3B} and A_{4B} represents a methine radical carrying a hydroxyl radical, which are subjected, if desired and if necessary, to one or more of following reactions, in any order:
- a complete reduction reaction of the hydroxyl or oxo radical to a methine radical followed by an aromatization,
- on the products of formula (Iᵢ) in which one of R_{4B}', R_{4B}'' or R_{4B}''' represents a hydroxyl radical or on the products of formulae (II_{b}), (II_{c}), (II_{d}), (IIₑ) and (II_{f}),
**either** firstly, to a substitution reaction of the hydroxyl radical by a halogen atom followed by the action of a product of formula Rₚ₄ - M - Hal in which Rₚ₄ has the meaning indicated in claim 1 for R_{4B} in which the optional reactive functions are optionally protected by protective groups, M represents a metal atom chosen from magnesium, copper and zinc and Hal represents a halogen atom,
**or** to the action of a product of formula Rₚ₄ - Hal in which Hal represents a halogen atom,
in order to obtain the corresponding products of formula (I_{B}),
- a conversion reaction of an (= O) oxo function into a (= S) thioxo function,
- an elimination reaction of the protective groups which can be carried by the protected reactive functions,
- a salification reaction by a mineral or organic acid or a mineral or organic base to obtain the corresponding salt,
- an esterification reaction of an acid function,
- a saponification reaction of the ester function into an acid function,
- a conversion reaction of the alkyloxy function into a hydroxyl function,
- a conversion reaction of the cyano function into an acid function,
- a reduction reaction of the carboxy function to an alcohol function,
- a resolution reaction of the racemic forms, the said products of formula (I_{B}) thus obtained being in all possible racemic, enantiomeric and diastereoisomeric isomer forms.

5. As medicaments, the products as defined by formula (I_{B}) of claim 1, the said products of formula (I_{B}) being in all possible racemic, enantiomeric and diastereoisomeric isomer forms, as well as the addition salts with pharmaceutically acceptable mineral and organic acids or mineral and organic bases of said products of formula (I_{B}).

6. As medicaments, the products of formula (Iₐ) as defined in claim 2, as well as the addition salts with pharmaceutically acceptable mineral and organic acids or mineral and organic bases of said products of formula (Iₐ).

7. The pharmaceutical compositions containing as active ingredient at least one of the medicaments as defined in any one of claims 5 and 6.

8. As new industrial products, the compounds of formulae (VIII), (II_{b}), (II_{c}), (II_{d}), (IIₑ) and (II_{f}) as defined in claim 4, with the exception of the following compounds:
a) the compounds of formula (VIII) in which R_{2'B} and R_{3'B} = H, R_{4'B} = CO₂Et, Me, Et, CF₃, H and R_{4''B} = CO₂Et;
as well as the compound:
ethyl 2-[1-[2-(trifluoromethyl) phenyl amino]pent-1-ylidene]propanedioate.
b) the compounds of formula (II_{b}) in which R_{4'B} = H, Hal, alkyl having 1 to 4 C and R_{4''B} = CF₃;
as well as the compound:
ethyl 2-butyl 4-hydroxy 8-trifluoromethyl 3-quinoline carboxylate.
c) the compounds of formula (II_{c}) in which one of R'_{2B} and R'_{3B} represents H and the other represents phenyl and R_{4'''B} represents free or esterified carboxy;
d) the compound of formula (IIₑ):
4-butyl-1-phthalazinone
e) the compounds of formula (II_{f}) in which R_{2'B} and R_{3'B} represent H, Hal, CF₃ or alkyl and R'_{4B} represents alkyl or CF₃.

9. Use of the products of formula (I_{B}) as defined in claim 1,
the said products of formula (I_{B}) being in all possible racemic, enantiomeric and diastereoisomeric isomer forms, as well as the addition salts with mineral and organic acids or mineral and organic bases of said products of formula (I_{B}), for the preparation of medicaments intended:
**either** for the treatment of arterial hypertension, cardiac insufficiencies, renal insufficiencies and in the prevention of the post-angioplastic recurrence of stenosis,
or for the treatment of certain gastro-intestinal or gynaecological disorders.

## Claims (Claims for the following Contracting State(s): ES)

1. Process for preparing products of formula (I_{B}): 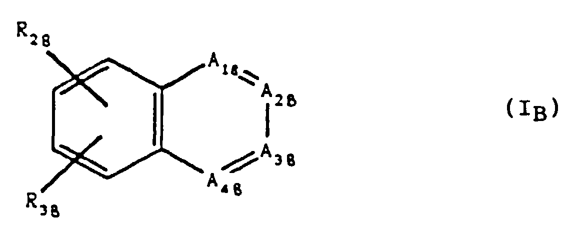 in which:
R_{2B} and R_{3B}, identical or different, are chosen from the group formed by:
- the hydrogen atom,
- halogen atoms,
- the hydroxyl radical,
- the mercapto, cyano, nitro, formyl, benzoyl, acyl radical having at most 6 carbon atoms,
- carboxy radicals free, salified or esterified by a linear or branched alkyl radical containing at most 4 carbon atoms,
- linear or branched alkyl, alkenyl, alkyloxy and alkylthio radicals containing at most 6 carbon atoms, phenyl, naphthyl, benzyl and phenylthio radicals, all these radicals being optionally substituted by one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole, isoxazole, pyrrolidinyl, pyrrolidinylcarbonyl radicals and the phenyl radical optionally substituted by one or more radicals chosen from halogen atoms, the hydroxyl radical, alkyl and alkoxy radicals containing at most 4 carbon atoms,
- the following radicals: amino, mono- or dialkylamino, carbamoyl, pyrrolyl, morpholine, piperazinyl, pyrrolylmethyl, morpholinomethyl, piperazinylmethyl, pyrrolylcarbonyl, morpholinocarbonyl, pyrrolidinylcarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, the following radicals: hydroxyl, nitro, alkyl, alkyloxy or acyl containing at most 4 carbon atoms, trifluoromethyl, cyano, free, salified or esterified carboxy, tetrazole and isoxazole,
A_{1B}, A_{2B}, A_{3B} and A_{4B} are such that:
- one represents a nitrogen atom,
- another represents a nitrogen atom or the methine radical substituted by R_{4b},
- another represents the methine radical substituted by R_{4c},
- and the last one represents the methine radical substituted by the benzyl radical itself substituted by one or more radicals chosen from cyano radicals, free, salified and esterified carboxy radicals,
- R_{4b} and R_{4c}, identical or different, are chosen from the values of R_{4B},
such that R_{4B} represents:
- the hydrogen atom, one of the following radicals: hydroxyl, cyano, carboxy free, salified or esterified by an alkyl radical containing at most 4 carbon atoms,
- the alkyl, alkenyl, alkyloxy, acyl or alkylthio radical, these radicals being linear or branched and containing at most 7 carbon atoms, all these radicals being optionally substituted by one or more radicals chosen from the halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, the said products of formula (I_{B}) being in all possible racemic, enantiomeric and diastereoisomeric isomer forms, as well as the addition salts with mineral and organic acids or mineral and organic bases of said products of formula (I_{B}),
characterized in that:
a) either the compound of formula (III): 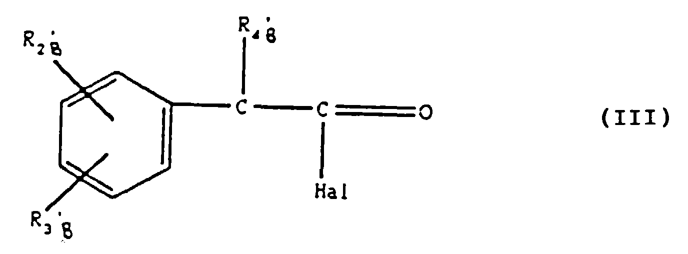 in which R_{2B'}, R_{3B'} and R_{4B'} have the meanings indicated above for R_{2B}, R_{3B} and R_{4B} respectively in which the optional reactive functions are optionally protected by protective groups and Hal represents a halogen atom,
is subjected to a substitution reaction in order to obtain the product of formula (IV): 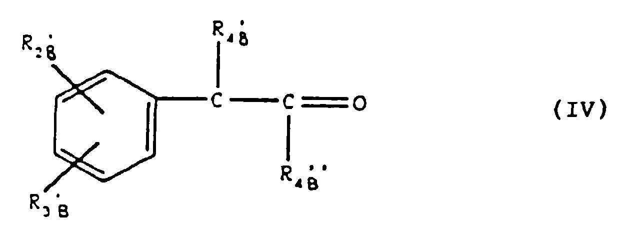 in which R_{2B'}, R_{3B'} and R_{4B'} have the meanings indicated above and R_{4B''}, identical to or different from R_{4B'}, has the meaning indicated above for R_{4B} in which the optional reactive functions are optionally protected by protective groups,
which is reacted:
with a compound of formula (V):
R_{4B'''}-C≡N (V)
in which R_{4B'''}, identical to or different from R_{4B'} or R_{4B''}, has the meaning indicated above for R_{4B} in which the optional reactive functions are optionally protected by protective groups, in order to obtain after cyclization a product of formula (Iᵢ): 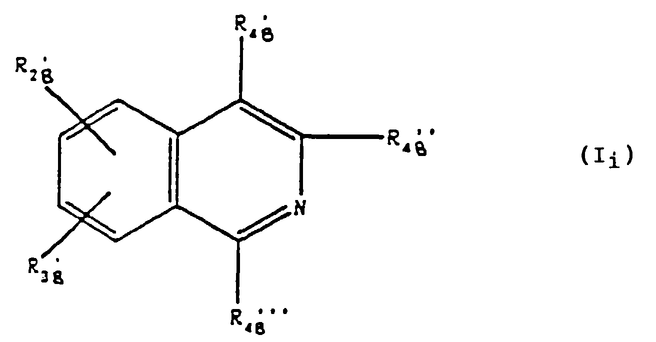 in which R_{2B'}, R_{3B'}, R_{4B'}, R_{4B''} and R_{4B'''} have the meanings indicated above,
b) or a product of formula (VI): 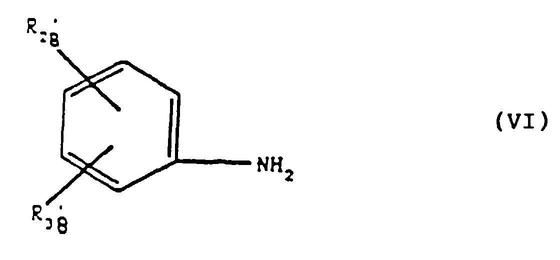 in which R_{2B'} and R_{3B'} have the meanings indicated above, is reacted with a product of formula (VII):
R_{4B'}-CH(CO₂alk)-CO-R_{4B''} (VII)
in which R_{4B'} and R_{4B''}, identical or different, have the meanings indicated above, and alk represents an alkyl radical containing at most 6 carbon atoms, in order to obtain the products of formula (VIII): 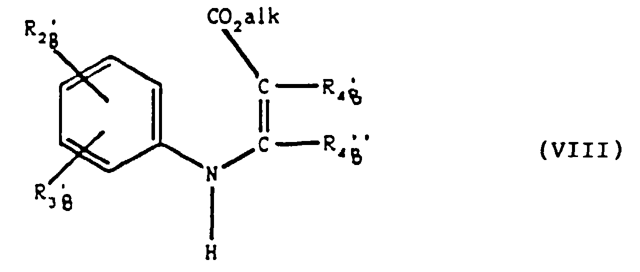 in which R_{2B'}, R_{3B'}, R_{4B'}, R_{4B''} and alk have the meanings indicated above, which are cyclized in order to obtain the products of formula (II_{b}): 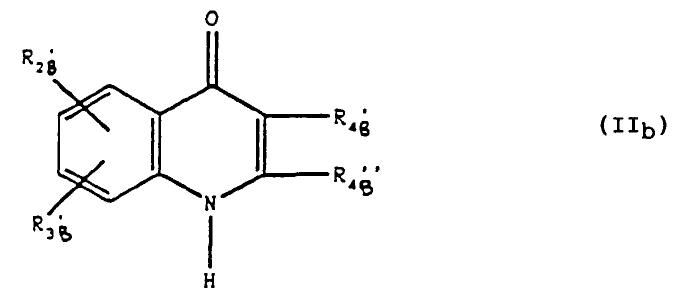 in which R_{2B'}, R_{3B'}, R_{4B'} and R_{4B''} have the meanings indicated above,
c) or a product of formula (IX): 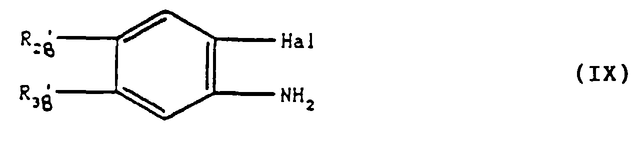 in which R_{2B'} and R_{3B'} have the meanings indicated above, is reacted with a product of formula (X):
R_{4B'''}-C≡CH (X)
in which R_{4B'''} has the meaning indicated above, in order to obtain the products of formula (XI): 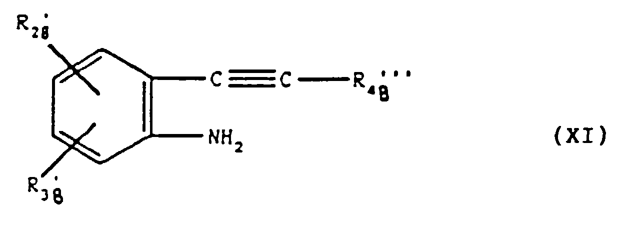 in which R_{2B'}, R_{3B'} and R_{4B'''} have the meanings indicated above, which is subjected to a cyclization reaction in the presence of a nitrogen donor such as sodium nitrate, in order to obtain a product of formula (II_{c}): 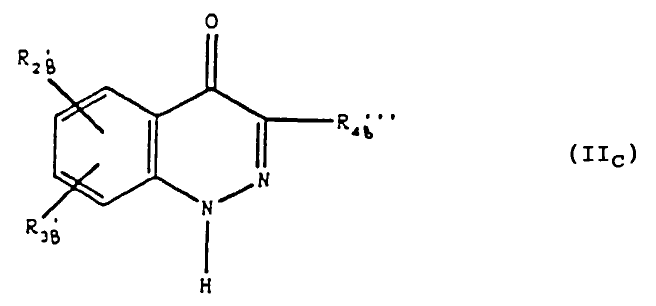 in which R_{2B'}, R_{3B'} and R_{4B'''} have the meanings indicated above,
d) or a product of formula (XII): 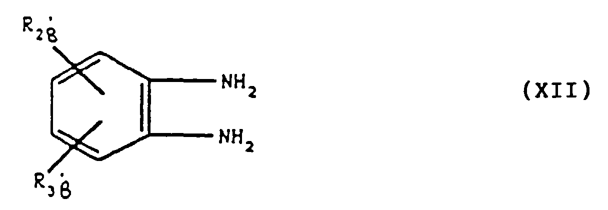 in which R_{2B}' and R_{3B}' have the meanings indicated above, is reacted with a product of formula (XIII):
R_{4B}'-CO-CO₂alk (XIII)
in which R_{4B}' has the meaning indicated above, and alk represents an alkyl radical containing at most 6 carbon atoms, in order to obtain, after cyclization, the products of formula (II_{d}): 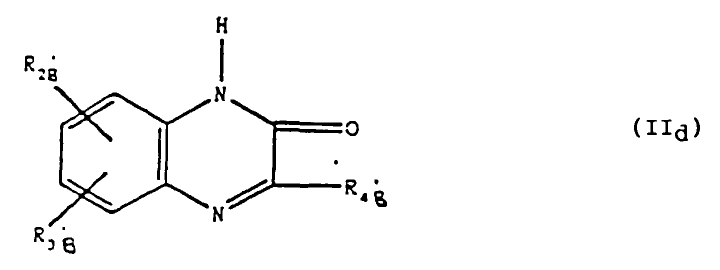 in which R_{2B}', R_{3B}' and R_{4B}' have the meanings indicated above,
e) or a product of formula (XIV): 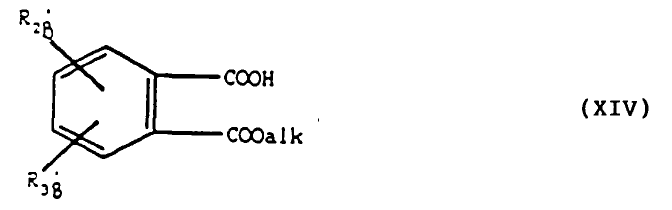 in which R_{2B}', R_{3B}' and alk have the meanings indicated above, after, if desired, a halogenation reaction of the free carboxy function, is subjected to an addition reaction on this carboxy function of a compound of formula R_{4B}'-H, R_{4B}' having the meaning indicated above, in order to obtain, after cyclization in the presence of hydrazine or a derivative, the products of formula (IIₑ): 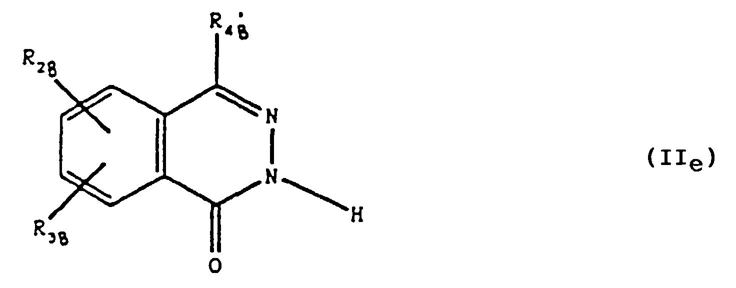 in which R_{2B}', R_{3B}' and R_{4B}' have the meanings indicated above,
f) or a product of formula (XV): 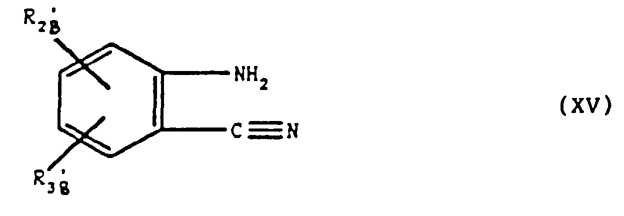 in which R_{2B}' and R_{3B}' have the meanings indicated above, is reacted with a product of formula (XVI):
R_{4B}'-CO-Cl (XVI)
in which R_{4B}' has the meaning indicated above, in order to obtain the product of formula (XVII): 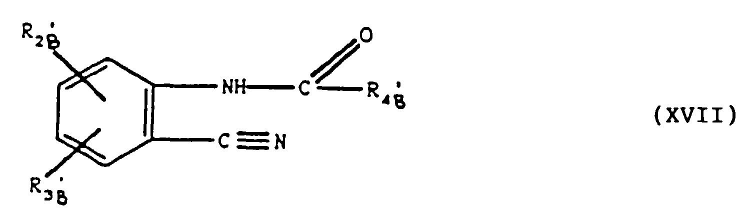 in which R_{2B}', R_{3B}' and R_{4B}' have the meanings indicated previously, in order to obtain, after cyclization, the products of formula (II_{f}): 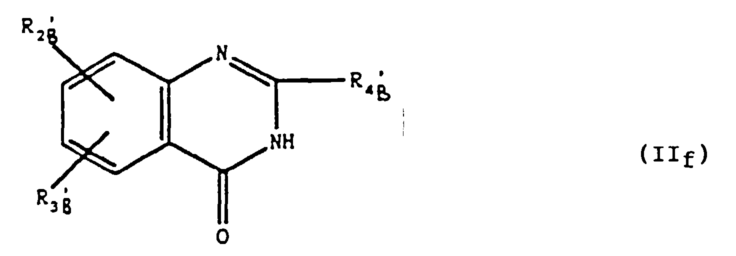 in which R_{2B}', R_{3B}' and R_{4B}' have the meanings indicated above,
which products of formula (Iᵢ) can represent products of formula (I_{B}) and products of formulae (II_{b}), (II_{c}), (II_{d}), (IIₑ) and (II_{f}) as defined above which can represent products of formula (I_{B}) in which at least one of A_{1B}, A_{2B}, A_{3B} and A_{4B} represents a methine radical carrying a hydroxyl radical, which are subjected, if desired and if necessary, to one or more of following reactions, in any order:
- a complete reduction reaction of the hydroxyl or oxo radical to a methine radical followed by an aromatization,
- on the products of formula (Iᵢ) in which one of R_{4B}', R_{4B}'' or R_{4B}''' represents a hydroxyl radical or on the products of formulae (II_{b}), (II_{c}), (II_{d}), (IIₑ) and (II_{f}),
**either** firstly, to a substitution reaction of the hydroxyl radical by a halogen atom followed by the action of a product of formula Rₚ₄ - M - Hal in which Rₚ₄ has the meaning indicated in claim 1 for R_{4B} in which the optional reactive functions are optionally protected by protective groups, M represents a metal atom chosen from magnesium, copper and zinc and Hal represents a halogen atom,
**or** to the action of a product of formula Rₚ₄ - Hal in which Hal represents a halogen atom,
in order to obtain the corresponding products of formula (I_{B}),
- a conversion reaction of an (= O) oxo function into a (= S) thioxo function,
- an elimination reaction of the protective groups which can be carried by the protected reactive functions,
- a salification reaction by a mineral or organic acid or a mineral or organic base to obtain the corresponding salt,
- an esterification reaction of an acid function,
- a saponification reaction of the ester function into an acid function,
- a conversion reaction of the alkyloxy function into a hydroxyl function,
- a conversion reaction of the cyano function into an acid function,
- a reduction reaction of the carboxy function to an alcohol function,
- a resolution reaction of the racemic forms,
the said products of formula (I_{B}) thus obtained being in all possible racemic, enantiomeric and diastereoisomeric isomer forms.

2. Process according to claim 1 for preparing products of formula (I_{B}) as defined in claim 1 and corresponding to formula (Iₐ): 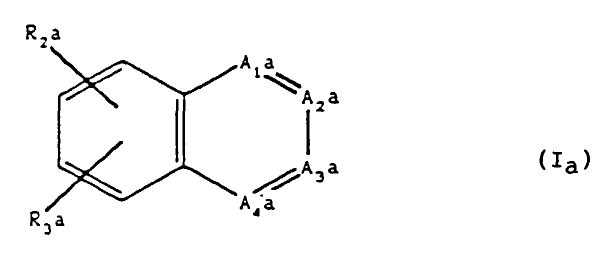 in which:
R₂ₐ and R₃ₐ, identical or different, are chosen from the group formed by:
- the hydrogen atom,
- halogen atoms,
- the hydroxyl radical,
- one of the following radicals: mercapto, cyano, nitro, formyl, benzoyl, acyl having at most 6 carbon atoms,
- carboxy radicals free, salified or esterified by a linear or branched alkyl radical containing at most 4 carbon atoms,
- linear or branched alkyl, alkyloxy and alkylthio radicals containing at most 6 carbon atoms, phenyl, naphthyl, benzyl and phenylthio radicals, all these radicals being optionally substituted one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole and isoxazole radicals,
- the following radicals: amino, mono- or dialkylamino, carbamoyl, pyrrolyl, morpholino, piperazinyl, pyrrolylmethyl, morpholinomethyl, piperazinylmethyl, pyrrolylcarbonyl, morpholinocarbonyl, pyrrolidinylcarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, the following radicals: hydroxyl, nitro, alkyl, alkyloxy or acyl containing at most 4 carbon atoms, trifluoromethyl, cyano, free, salified or esterified carboxy, tetrazole and isoxazole,
A₁ₐ, A₂ₐ, A₃ₐ and A₄ₐ are such that:
- one represents a nitrogen atom,
- another represents a nitrogen atom or the methine radical substituted by R_{4ab},
- another represents the methine radical substituted by R_{4ac},
- and the last one represents the methine radical substituted by the benzyl radical itself substituted by one or more radicals chosen from cyano radicals, free, salified and esterified carboxy radicals,
- R_{4ab} and R_{4ac}, identical or different, are chosen from the values of R₄ₐ,
such that R₄ₐ represents:
- the hydrogen atom, one of the following radicals: hydroxyl, cyano, carboxy free, salified or esterified by an alkyl radical containing at most 4 carbon atoms,
- the alkyl, alkenyl, alkyloxy, acyl or alkylthio radical, these radicals being linear or branched and containing at most 7 carbon atoms, all these radicals being optionally substituted by one or more radicals chosen from halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, it being understood that the products of formula (Iₐ) cannot represent the following products:
either R₂ₐ and R₃ₐ are chosen from halogen atoms, hydroxyl, alkyl and alkyloxy radicals and A₁ₐ, A₂ₐ, A₃ₐ and A₄ₐ are such that:
- one represents the nitrogen atom,
- another represents the methine radical or the nitrogen atom,
- another represents the methine radical,
- these two methine radicals being optionally substituted by a radical chosen from free or esterified carboxy radicals, benzoyl radicals, phenyl radicals optionally substituted by an alkyl radical containing at most two carbon atoms itself optionally substituted by a hydroxyl or acyloxy radical,
- and the last one represents the benzyl radical optionally substituted by one or more radicals chosen from halogen atoms, alkyl, alkyloxy, amino, nitro and acetyl radicals,
or R₂ₐ and R₃ₐ are identical and represent the methoxy radical, A₁ₐ represents the benzyl radical substituted by a chlorine atom, A₂ₐ represents the methine radical substituted by the isopropyl or isobutyl radical, A₃ₐ represents the nitrogen atom and A₄ₐ represents the methine radical, the said products of formula (Iₐ) being in all possible racemic, enantiomeric and diastereoisomeric isomer forms, as well as the addition salts with mineral and organic acids or mineral and organic bases of said products of formula (Iₐ), characterized in that the starting products and the reagents are chosen in such a way that the products of formula (Iₐ) as defined above are prepared.

3. Process according to claim 1 for preparing products of formula (I_{B}) in which:
R_{2B} and R_{3B} represent a hydrogen atom,
A_{1B}, A_{2B} and A_{3B} are such that one or two of them represent a nitrogen atom, and the others are identical or different and represent the = C - R_{4B} radical such that R_{4B} is chosen from the hydrogen atom, the n-butyl radical and the alkylthio radical containing at most 4 carbon atoms,
and A_{4B} represents the methine radical substituted by the benzyl radical optionally substituted by one or more radicals chosen from cyano, free, salified and esterified carboxy radicals,
the said products of formula (I_{B}) being in all possible racemic, enantiomeric and diastereoisomeric isomer forms, as well as the addition salts with mineral and organic acids or mineral and organic bases of said products of formula (I_{B}), characterized in that the starting products and the reagents are chosen in such a way that the products of formula (I_{B}) as defined above are prepared.

## Claims (Claims for the following Contracting State(s): GR)

1. Process for preparing products of formula (I_{B}): 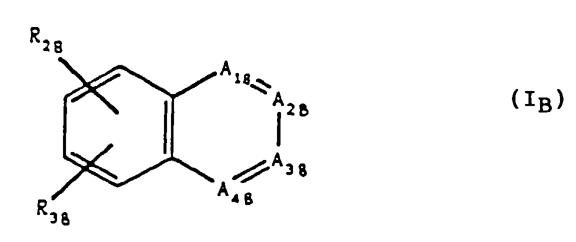 in which:
R_{2B} and R_{3B}, identical or different, are chosen from the group formed by:
- the hydrogen atom,
- halogen atoms,
- the hydroxyl radical,
- the mercapto, cyano, nitro, formyl, benzoyl, acyl radical having at most 6 carbon atoms,
- carboxy radicals free, salified or esterified by a linear or branched alkyl radical containing at most 4 carbon atoms,
- linear or branched alkyl, alkenyl, alkyloxy and alkylthio radicals containing at most 6 carbon atoms, phenyl, naphthyl, benzyl and phenylthio radicals, all these radicals being optionally substituted by one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole, isoxazole, pyrrolidinyl, pyrrolidinylcarbonyl radicals and the phenyl radical optionally substituted by one or more radicals chosen from halogen atoms, the hydroxyl radical, alkyl and alkoxy radicals containing at most 4 carbon atoms,
- the following radicals: amino, mono- or dialkylamino, carbamoyl, pyrrolyl, morpholino, piperazinyl, pyrrolylmethyl, morpholinomethyl, piperazinylmethyl, pyrrolylcarbonyl, morpholinocarbonyl, pyrrolidinylcarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, the following radicals: hydroxyl, nitro, alkyl, alkyloxy or acyl containing at most 4 carbon atoms, trifluoromethyl, cyano, free, salified or esterified carboxy, tetrazole and isoxazole,
A_{1B}, A_{2B}, A_{3B} and A_{4B} are such that:
- one represents a nitrogen atom,
- another represents a nitrogen atom or the methine radical substituted by R_{4b},
- another represents the methine radical substituted by R_{4c},
- and the last one represents the methine radical substituted by the benzyl radical itself substituted by one or more radicals chosen from cyano radicals, free, salified and esterified carboxy radicals,
- R_{4b} and R_{4c}, identical or different, are chosen from the values of R_{4B},
such that R_{4B} represents:
- the hydrogen atom, one of the following radicals: hydroxyl, cyano, carboxy free, salified or esterified by an alkyl radical containing at most 4 carbon atoms,
- the alkyl, alkenyl, alkyloxy, acyl or alkylthio radical, these radicals being linear or branched and containing at most 7 carbon atoms, all these radicals being optionally substituted by one or more radicals chosen from the halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, the said products of formula (I_{B}) being in all possible racemic, enantiomeric and diastereoisomeric isomer forms, as well as the addition salts with mineral and organic acids or mineral and organic bases of said products of formula (I_{B}),
characterized in that:
a) either the compound of formula (III): 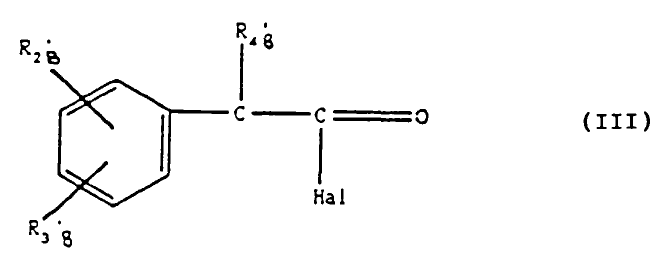 in which R_{2B'}, R_{3B'} and R_{4B'} have the meanings indicated above for R_{2B}, R_{3B} and R_{4B} respectively in which the optional reactive functions are optionally protected by protective groups and Hal represents a halogen atom, is subjected to a substitution reaction in order to obtain the product of formula (IV): 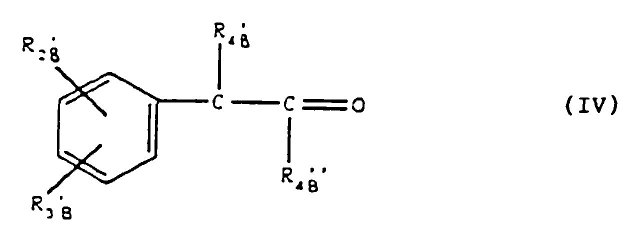 in which R_{2B'}, R_{3B'} and R_{4B'} have the meanings indicated above and R_{4B''}, identical to or different from R_{4B'}, has the meaning indicated above for R_{4B} in which the optional reactive functions are optionally protected by protective groups, which is reacted: with a compound of formula (V):
R_{4B'''}-C≡N (V)
in which R_{4B'''}, identical to or different from R_{4B'} or R_{4B''}, has the meaning indicated above for R_{4B} in which the optional reactive functions are optionally protected by protective groups, in order to obtain after cyclization a product of formula (Iᵢ): 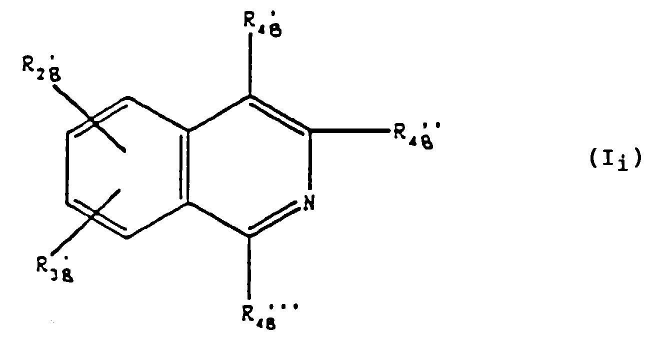 in which R_{2B'}, R_{3B'}, R_{4B'}, R_{4B''} and R_{4B'''} have the meanings indicated above,
b) or a product of formula (VI): 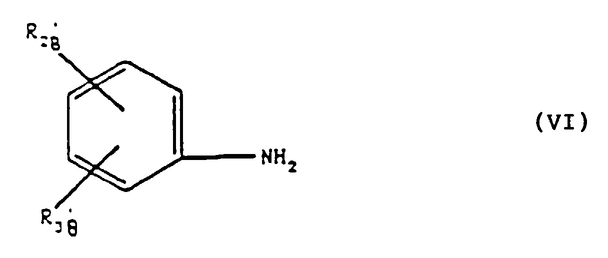 in which R_{2B'} and R_{3B'} have the meanings indicated above, is reacted with a product of formula (VII):
R_{4B'}-CH(CO₂alk)-CO-R_{4B''} (VII)
in which R_{4B'} and R_{4B''}, identical or different, have the meanings indicated above, and alk represents an alkyl radical containing at most 6 carbon atoms, in order to obtain the products of formula (VIII): 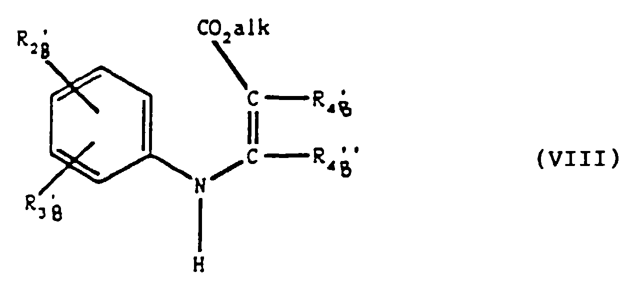 in which R_{2B'}, R_{3B'}, R_{4B'}, R_{4B''} and alk have the meanings indicated above, which are cyclized in order to obtain the products of formula (II_{b}): 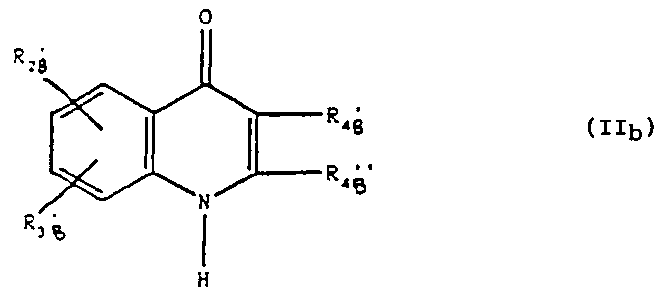 in which R_{2B'}, R_{3B'}, R_{4B'} and R_{4B''} have the meanings indicated above,
c) or a product of formula (IX): 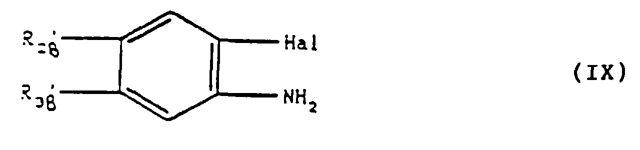 in which R_{2B'} and R_{3B'} have the meanings indicated above, is reacted with a product of formula (X):
R_{4B'''}-C≡CH (X)
in which R_{4B'''} has the meaning indicated above, in order to obtain the products of formula (XI): 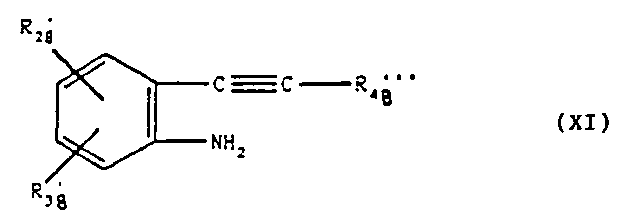 in which R_{2B'}, R_{3B'} and R_{4B'''} have the meanings indicated above, which is subjected to a cyclization reaction in the presence of a nitrogen donor such as sodium nitrite, in order to obtain a product of formula (II_{c}): 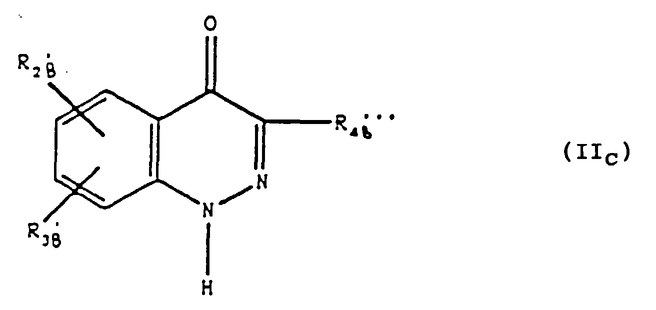 in which R_{2B'}, R_{3B'} and R_{4B'''} have the meanings indicated above,
d) or a product of formula (XII): 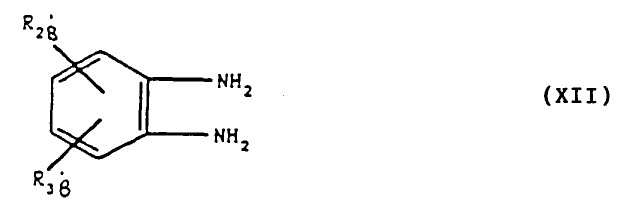 in which R_{2B}' and R_{3B}' have the meanings indicated above, is reacted with a product of formula (XIII):
R_{4B}'-CO-CO₂alk (XIII)
in which R_{4B}' has the meaning indicated above, and alk represents an alkyl radical containing at most 6 carbon atoms, in order to obtain, after cyclization, the products of formula (II_{d}): 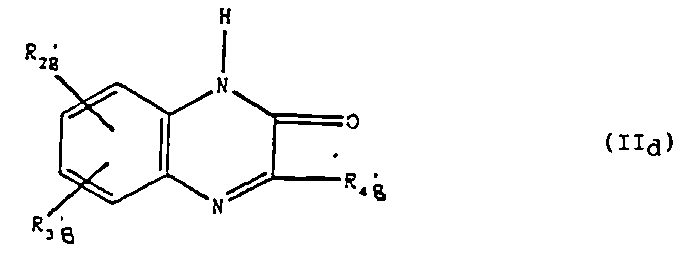 in which R_{2B}', R_{3B}' and R_{4B}' have the meanings indicated above,
e) or a product of formula (XIV): 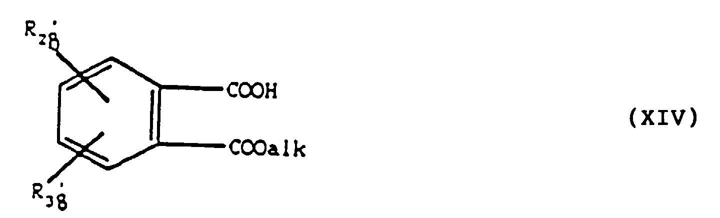 in which R_{2B}', R_{3B}' and alk have the meanings indicated above, after, if desired, a halogenation reaction of the free carboxy function, is subjected to an addition reaction on this carboxy function of a compound of formula R_{4B}'-H, R_{4B}' having the meaning indicated above, in order to obtain, after cyclization in the presence of hydrazine or a derivative, the products of formula (IIₑ): 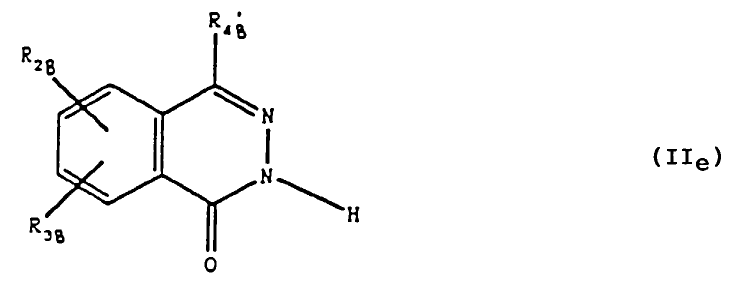 in which R_{2B}', R_{3B}' and R_{4B}' have the meanings indicated above,
f) or a product of formula (XV): 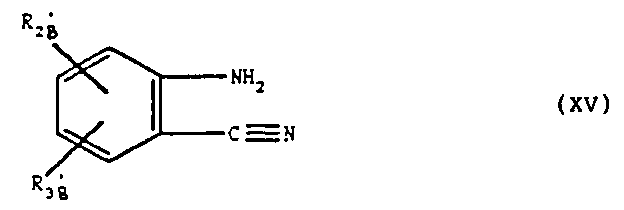 in which R_{2B}' and R_{3B}' have the meanings indicated above, is reacted with a product of formula (XVI):
R_{4B}'-CO-Cl (XVI)
in which R_{4B}' has the meaning indicated above, in order to obtain the product of formula (XVII): 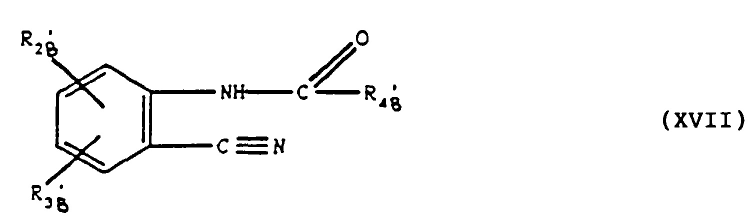 in which R_{2B}', R_{3B}' and R_{4B}' have the meanings indicated previously, in order to obtain, after cyclization, the products of formula (II_{f}): 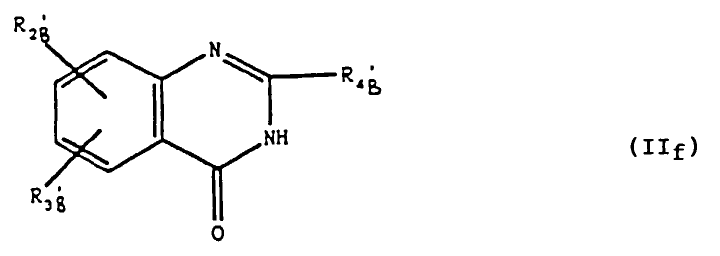 in which R_{2B}', R_{3B}' and R_{4B}' have the meanings indicated above,
which products of formula (Iᵢ) can represent products of formula (I_{B}) and products of formulae (II_{b}), (II_{c}), (II_{d}), (IIₑ) and (II_{f}) as defined above which can represent products of formula (I_{B}) in which at least one of A_{1B}, A_{2B}, A_{3B} and A_{4B} represents a methine radical carrying a hydroxyl radical, which are subjected, if desired and if necessary, to one or more of following reactions, in any order:
- a complete reduction reaction of the hydroxyl or oxo radical to a methine radical followed by an aromatization,
- on the products of formula (Iᵢ) in which one of R_{4B}', R_{4B}'' or R_{4B}''' represents a hydroxyl radical or on the products of formulae (II_{b}), (II_{c}), (II_{d}), (IIₑ) and (II_{f}),
**either** firstly, to a substitution reaction of the hydroxyl radical by a halogen atom followed by the action of a product of formula Rₚ₄ - M - Hal in which Rₚ₄ has the meaning indicated in claim 1 for R_{4B} in which the optional reactive functions are optionally protected by protective groups, M represents a metal atom chosen from magnesium, copper and zinc and Hal represents a halogen atom,
**or** to the action of a product of formula Rₚ₄ - Hal in which Hal represents a halogen atom,
in order to obtain the corresponding products of formula (I_{B}),
- a conversion reaction of an (= O) oxo function into a (= S) thioxo function,
- an elimination reaction of the protective groups which can be carried by the protected reactive functions,
- a salification reaction by a mineral or organic acid or a mineral or organic base to obtain the corresponding salt,
- an esterification reaction of an acid function,
- a saponification reaction of the ester function into an acid function,
- a conversion reaction of the alkyloxy function into a hydroxyl function,
- a conversion reaction of the cyano function into an acid function,
- a reduction reaction of the carboxy function to an alcohol function,
- a resolution reaction of the racemic forms,
the said products of formula (I_{B}) thus obtained being in all possible racemic, enantiomeric and diastereoisomeric isomer forms.

2. Process according to claim 1 for preparing products of formula (I_{B}) as defined in claim 1 and corresponding to formula (Iₐ): 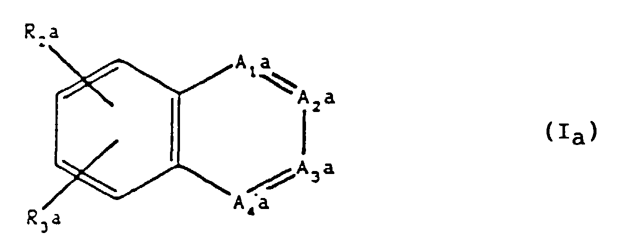 in which:
R₂ₐ and R₃ₐ, identical or different, are chosen from the group formed by:
- the hydrogen atom,
- halogen atoms,
- the hydroxyl radical,
- one of the following radicals: mercapto, cyano, nitro, formyl, benzoyl, acyl having at most 6 carbon atoms,
- carboxy radicals free, salified or esterified by a linear or branched alkyl radical containing at most 4 carbon atoms,
- linear or branched alkyl, alkyloxy and alkylthio radicals containing at most 6 carbon atoms, phenyl, naphthyl, benzyl and phenylthio radicals, all these radicals being optionally substituted by one or more identical or different radicals chosen from halogen atoms, the hydroxyl radical, alkyloxy radicals containing at most 4 carbon atoms, trifluoromethyl, cyano, acyl radicals, free, salified or esterified carboxy radicals, tetrazole and isoxazole radicals,
- the following radicals: amino, mono- or dialkylamino, carbamoyl, pyrrolyl, morpholino, piperazinyl, pyrrolylmethyl, morpholinomethyl, piperazinylmethyl, pyrrolylcarbonyl, morpholinocarbonyl, pyrrolidinylcarbonyl, piperazinylcarbonyl, all the piperazinyl radicals of these radicals being optionally substituted on the second nitrogen atom by an alkyl or phenyl radical, these alkyl and phenyl radicals themselves being optionally substituted by one or more radicals chosen from halogen atoms, the following radicals: hydroxyl, nitro, alkyl, alkyloxy or acyl containing at most 4 carbon atoms, trifluoromethyl, cyano, free, salified or esterified carboxy, tetrazole and isoxazole,
A₁ₐ, A₂ₐ, A₃ₐ and A₄ₐ are such that:
- one represents a nitrogen atom,
- another represents a nitrogen atom or the methine radical substituted by R_{4ab},
- another represents the methine radical substituted by R_{4ac},
- and the last one represents the methine radical substituted by the benzyl radical itself substituted by one or more radicals chosen from cyano radicals, free, salified and esterified carboxy radicals,
- R_{4ab} and R_{4ac}, identical or different, are chosen from the values of R₄ₐ,
such that R₄ₐ represents:
- the hydrogen atom, one of the following radicals: hydroxyl, cyano, carboxy free, salified or esterified by an alkyl radical containing at most 4 carbon atoms,
- an alkyl, alkenyl, alkyloxy, acyl or alkylthio radical, these radicals being linear or branched and containing at most 7 carbon atoms, all these radicals being optionally substituted by one or more radicals chosen from halogen atoms, the following radicals: hydroxyl, nitro, alkyl, alkyloxy or acyl containing at most 4 carbon atoms, trifluoromethyl, cyano, carboxy free, salified or esterified by an alkyl radical containing at most 4 carbon atoms, it being understood that the products of formula (Iₐ) cannot represent the following products:
either R₂ₐ and R₃ₐ are chosen from halogen atoms, hydroxyl, alkyl and alkyloxy radicals and A₁ₐ, A₂ₐ, A₃ₐ and A₄ₐ are such that:
- one represents the nitrogen atom,
- another represents the methine radical or the nitrogen atom,
- another represents the methine radical,
these two methine radicals being optionally substituted by a radical chosen from free or esterified carboxy radicals, benzoyl radicals, phenyl radicals optionally substituted by an alkyl radical containing at most two carbon atoms itself optionally substituted by a hydroxyl or acyloxy radical,
- and the last one represents the benzyl radical optionally substituted by one or more radicals chosen from halogen atoms, alkyl, alkyloxy, amino, nitro and acetyl radicals,
or R₂ₐ and R₃ₐ are identical and represent the methoxy radical, A₁ₐ represents the benzyl radical substituted by a chlorine atom, A₂ₐ represents the methine radical substituted by the isopropyl or isobutyl radical, A₃ₐ represents the nitrogen atom and A₄ₐ represents the methine radical, the said products of formula (Iₐ) being in all possible racemic, enantiomeric and diastereoisomeric isomer forms, as well as the addition salts with mineral and organic acids or mineral and organic bases of said products of formula (Iₐ), characterized in that the starting products and the reagents are chosen in such a way that the products of formula (Iₐ) as defined above are prepared.

3. Process according to claim 1 for preparing products of formula (I_{B}) in which:
R_{2B} and R_{3B} represent a hydrogen atom,
A_{1B}, A_{2B} and A_{3B} are such that one or two of them represent a nitrogen atom, and the others are identical or different and represent the = C - R_{4B} radical such that R_{4B} is chosen from the hydrogen atom, the n-butyl radical and the alkylthio radical containing at most 4 carbon atoms,
and A_{4B} represents the methine radical substituted by the benzyl radical optionally substituted by one or more radicals chosen from cyano, free, salified and esterified carboxy radicals,
the said products of formula (I_{B}) being in all possible racemic, enantiomeric and diastereoisomeric isomer forms, as well as the addition salts with mineral and organic acids or mineral and organic bases of said products of formula (I_{B}), characterized in that the starting products and the reagents are chosen in such a way that the products of formula (I_{B}) as defined above are prepared.

4. Preparation process for pharmaceutical compositions, characterized in that at least one of the products of formula (I_{B}) as defined in claim 1 in all possible racemic, enantiomeric and diastereoisomeric isomer forms or at least one of the addition salts with pharmaceutically acceptable mineral and organic acids or mineral and organic bases of said product of formula (I_{B}) is used as active ingredient in a form intended for this use.

5. Preparation process for pharmaceutical compositions, characterized in that at least one of the products of formula (Iₐ) as defined in one of claims 2 to 3 in all possible racemic, enantiomeric and diastereoisomeric isomer forms or at least one of the addition salts with pharmaceutically acceptable mineral and organic acids or mineral and organic bases of said products of formula (Iₐ) is used as active ingredient in a form intended for this use.

6. As new industrial products, the compounds of formulae (VIII), (II_{b}), (II_{c}), (II_{d}), (IIₑ) and (II_{f}) as defined in claim 1, with the exception of the following compounds:
a) the compounds of formula (VIII) in which R_{2'B} and R_{3'B} = H, R_{4'B} = CO₂Et, Me, Et, CF₃, H and R_{4''B} = CO₂Et;
as well as the compound:
ethyl 2-[1-[2-(trifluoromethyl) phenyl amino]pent-1-ylidene]propanedioate.
b) the compounds of formula (II_{b}) in which R_{4'B} = H, Hal or alkyl having 1 to 4 C and R_{4''B} = CF₃;
as well as the compound:
ethyl 2-butyl 4-hydroxy 8-trifluoromethyl 3-quinoline carboxylate.
c) the compounds of formula (II_{c}) in which one of R'_{2B} and R'_{3B} represents H and the other represents phenyl and R_{4'''B} represents free or esterified carboxy;
d) the compound of formula (IIₑ): 4-butyl-1-phthalazinone
e) the compounds of formula (II_{f}) in which R_{2'B} and R_{3'B} represent H, Hal, CF₃ or alkyl and R'_{4B} represents alkyl or CF₃.

## Patentansprüche (Patentansprüche für folgende(n) Vertragsstaat(en): AT, BE, CH, LI, DE, DK, FR, GB, IT, LU, NL, SE)

1. Produkte der Formel (I_{B}): 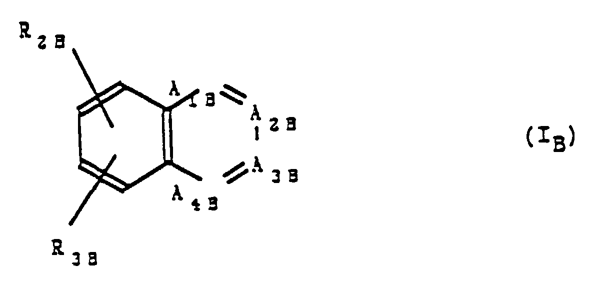 worin:
R_{2B} und R_{3B'} die identisch oder verschieden sind, ausgewählt sind aus der Gruppe gebildet durch:
- das Wasserstoffatom,
- die Halogenatome,
- der Hydroxylrest,
- der Rest Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Hohlenstoffatomen,
- die Reste Alkyl, Alkenyl, Alkyloxy und Alkylthio, linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, Alkyloxyresten mit höchstens 4 Kohlenstoffatomen, Trifluoromethyl, Cyano, Acyl, Carboxy frei, in Salzform oder verestert, Tetrazol, Isoxazol, Pyrrolidinyl, Pyrrolidinylcarbonyl und Phenyl gegebenenfalls substituiert durch einen oder mehrere Reste, ausgewählt unter den Halogenatomen, dem Hydroxylrest, den Alkyl- und Alkoxyresten mit höchstens 4 Kohlenstoffatomen,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Pyrrolidinylcarbonyl, Piperazinylcarbonyl, wobei alle Piperazinylreste dieser Reste gegebenenfalls substituiert sind am zweiten Stickstoffatom durch einen Alkyl- oder Phenylrest, wobei diese Alkyl- und Phenylreste ihrerseits gegebenenfalls substituiert sind durch einen oder mehrere Reste ausgewählt unter den Halogenatomen, den Hydroxyl-, Nitro-, Alkyl-, Alkyloxy- oder Acylresten mit höchstens 4 Kohlenstoffatomen, Trifluoromethyl, Cyano, Carboxy frei, in Salzform oder verestert, Tetrazol und Isoxazol,
A_{1B}, A_{2B}, A_{3B} und A_{4B} sind derart, daß:
- eines ein Stickstoffatom darstellt,
- ein anderes ein Stickstoffatom oder den Methinrest substituiert durch R_{4b} bedeutet,
- ein anderes den Methinrest substituiert durch R_{4c} darstellt,
- und das letzte den Methinrest substituiert durch den Benzylrest bedeutet, der seinerseits substituiert ist durch ein oder mehrere Reste ausgewählt unter den Resten Cyano, Carboxy frei, in Salzform oder verestert,
R_{4b} und R_{4c}, die identisch oder verschieden sind, sind ausgewählt unter den Werten von R_{4B}, derart, daß
R_{4B} bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen,
wobei diese Produkte der Formel (I_{B}) unter allen möglichen isomeren Formen, racemisch, enantiomer oder diastereoisomer, vorliegen, sowie die Additionssalze mit Mineral- und organischen Säuren oder mit Mineral- und organischen Basen dieser Produkte der Formel (I_{B}).

2. Produkte der Formel (I_{B}), wie in Anspruch 1 definiert, und entsprechend der Formel (Iₐ): 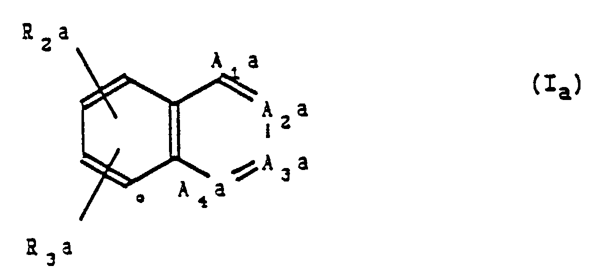 worin:
R₂ₐ und R₃ₐ, die identisch oder verschieden sind, ausgewählt sind aus der Gruppe gebildet durch:
- das Wasserstoffatom,
- die Halogenatome,
- den Hydroxylrest,
- den Rest Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Kohlenstoffatomen,
- die Reste Alkyl, Alkyloxy und Alkylthio linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, Alkyloxyresten mit höchstens 4 Kohlenstoffatomen, Trifluoromethyl, Cyano, Acyl, Carboxy frei, in Salzform oder verestert, Tetrazol und Isoxazol,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Piperazinylcarbonyl, wobei alle Piperazinylreste dieser Reste gegebenenfalls substituiert sind am zweiten Stickstoffatom durch einen Alkyl- oder Phenylrest, wobei diese Alkyl- und Phenylreste ihrerseits gegebenenfalls substituiert sind durch einen oder mehrere Reste ausgewählt unter den Halogenatomen, den Hydroxyl-, Nitro-, Alkyl-, Alkyloxy- oder Acylresten mit höchstens 4 Kohlenstoffatomen, Trifluoromethyl, Cyano, Carboxy frei, in Salzform oder verestert, Tetrazol und Isoxazol,
A₁ₐ, A₂ₐ, A₃ₐ und A₄ₐ sind derart, daß:
- eines ein Stickstoffatom darstellt,
- ein anderes ein Stickstoffatom oder den Methinrest substituiert durch R_{4ab} bedeutet,
- ein anderes den Methinrest substituiert durch R_{4ac} darstellt,
- und das letzte den Methinrest substituiert durch den Benzylrest bedeutet, der seinerseits substituiert ist durch ein oder mehrere Reste ausgewählt unter den Resten Cyano, Carboxy frei, in Salzform oder verestert,
R_{4ab} und R_{4ac}, die identisch oder verschieden sind, sind ausgewählt unter den Werten von R₄ₐ, derart, daß
R₄ₐ bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen
wobei diese Produkte der Formel (Iₐ) unter allen möglichen isomeren Formen, racemisch, enantiomer oder diastereoisomer, vorliegen, sowie die Additionssalze mit Mineral- und organischen Säuren oder mit Mineral- und organischen Basen dieser Produkte der Formel (Iₐ).

3. Produkte der Formel (I_{B}), wie in den Ansprüchen 1 und 2 definiert, worin:
R_{2B} und R_{3B} ein Wasserstoffatom bedeuten,
A_{1B}, A_{2B} und A_{3B} sind derart, daß eines oder zwei von ihnen ein Stickstoffatom bedeutet und die anderen, die identisch oder verschieden sind, den Rest = C - R_{4B} bedeuten, R_{4B} ausgewählt ist unter dem Wasserstoffatom, dem n-Butylrest und Alkylthio mit höchstens 4 Kohlenstoffatomen
und A_{4B} den Methinrest substituiert durch den Benzylrest bedeutet, der seinerseits substituiert ist durch einen oder mehrere Rest(e) ausgewählt unter den Resten cyano, Carboxy frei, in Salzform oder verestert,
wobei diese Produkte der Formel (I_{B}) unter allen möglichen isomeren Formen, racemisch, enantiomer und diastereoisomer, vorliegen, sowie die Additionssalze mit Mineral- und organischen Säuren, oder mit Mineral- und organischen Basen dieser Produkte der Formel (I_{B}).

4. Verfahren zur Herstellung der Produkte der Formel (I_{B}), wie in Anspruch 1 definiert, dadurch gekennzeichnet, daß man:
a) entweder die Verbindung der Formel (III) 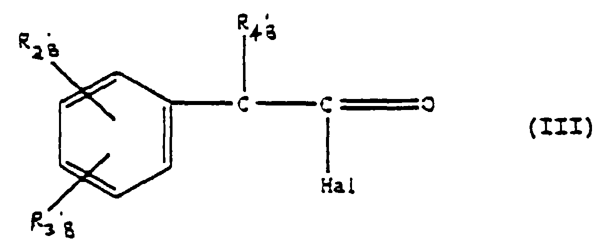 worin R_{2B}', R_{3B}' und R_{4B}' die in Anspruch 1 für R_{2B}, R_{3B} und R_{4B} angegebenen Bedeutungen haben, worin die eventuellen reaktiven Funktionen gegebenenfalls durch Schutzgruppen geschützt sind und Hal ein Halogenatom bedeutet,
einer Substitutionsreaktion unterwirft, um das Produkt der Formel (IV): 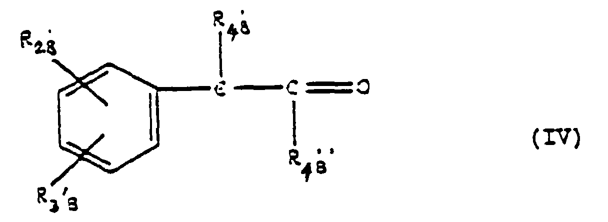 zu erhalten, worin R_{2B}', R_{3B}' und R_{4B}' die vorstehend angegebenen Bedeutungen haben, und R_{4B}'', identisch oder verschieden von R_{4B}', die in Anspruch 1 für R_{4B} angegebene Bedeutung hat, worin die eventuellen reaktiven Funktionen gegebenenfalls durch Schutzgruppen geschützt sind, daß man mit einer Verbindung der Formel (V):
R_{4B}'''-C≡N (V)
umsetzt, worin R_{4B}''', das identisch oder verschieden von R_{4B}' oder R_{4B}'' ist, die vorstehend in Anspruch 1 für R_{4B} angegebene Bedeutung hat, worin die eventuellen reaktiven Funktionen gegebenenfalls durch Schutzgruppen geschützt sind, um nach Cyclisierung ein Produkt der Formel (Iᵢ) 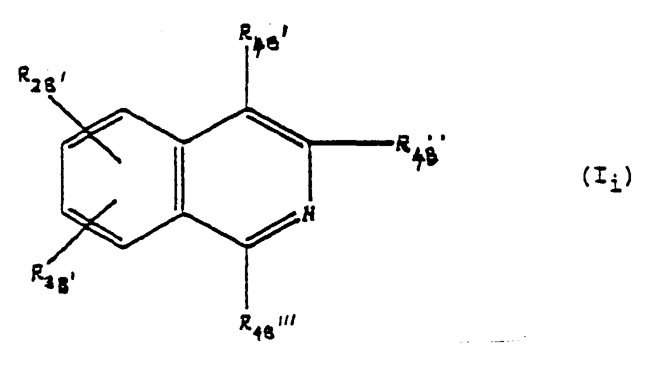 zu erhalten, worin R_{2B}', R_{3B}', R_{4B}', R_{4B}'' und R_{4B}''' die vorstehend angegebenen Bedeutungen haben,
b) oder daß man ein Produkt der Formel (VI): 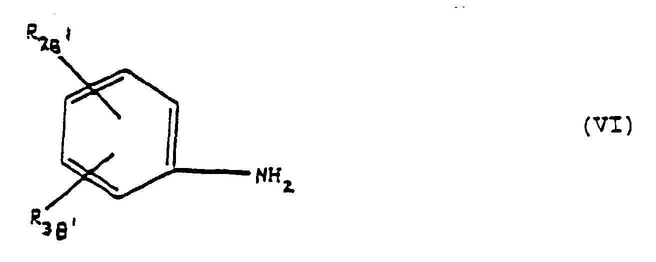 worin R_{2B}' und R_{3B}' die vorstehend angegebenen Bedeutungen haben, mit einem Produkt der Formel (VII):
R_{4B}'-CH(CO₂alk)-CO-R_{4B}'' (VII)
umsetzt, worin R_{4B}' und R_{4B}'', die identisch oder verschieden sind, die vorstehend angegebenen Bedeutungen haben, und alk einen Alkylrest mit höchstens 6 Kohlenstoffatomen darstellt, um Produkte der Formel (VIII): 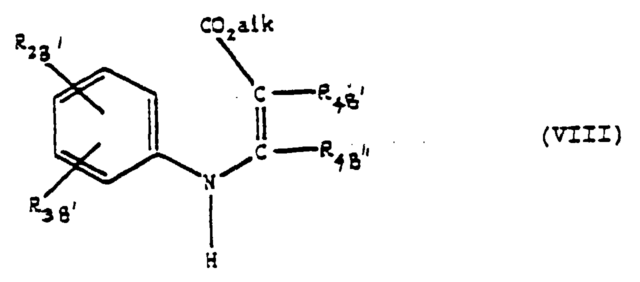 zu erhalten, worin R_{2B}', R_{3B}', R_{4B}', R_{4B}'' und alk die vorstehend angegebenen Bedeutungen haben, die man cyclisiert, um Produkte der Formel (II_{b}): 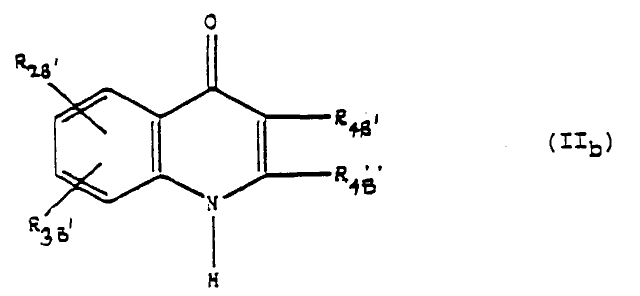 zu erhalten, worin R_{2B}', R_{3B}', R_{4B}' und R_{4B}'' die vorstehend angegebenen Bedeutungen haben,
c) oder daß man ein Produkt der Formel (IX) 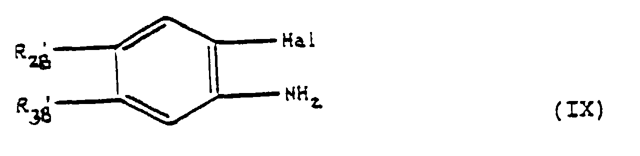 worin R_{2B}' und R_{3B}' die vorstehend angegebenen Bedeutungen haben, mit einem Produkt der Formel (X)
R_{4B}'''-C≡CH (X)
zur Reaktion bringt, worin R_{4B}''' die vorstehend angegebene Bedeutung hat, um Produkte der Formel (XI) 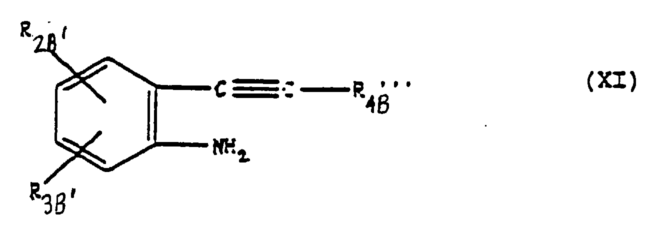 zu erhalten, worin R_{2B}', R_{3B}' und R_{4B}''' die vorstehend angegebenen Bedeutungen haben, die man einer Cyclisierungsreaktion in Gegenwart eines Stickstoffdonators wie Natriumnitrit unterwirft, um ein Produkt der Formel (II_{c}) 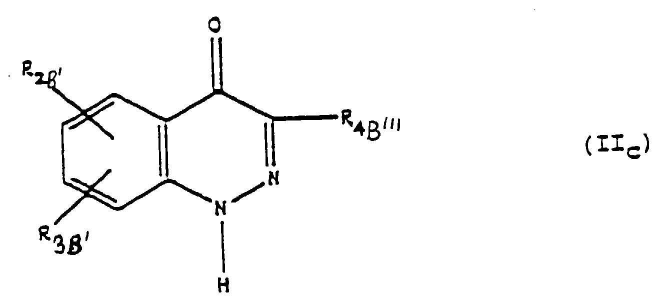 zu erhalten, worin R_{2B}', R_{3B}' und R_{4B}''' die vorstehend angegebenen Bedeutungen haben,
d) oder daß man ein Produkt der Formel (XII) 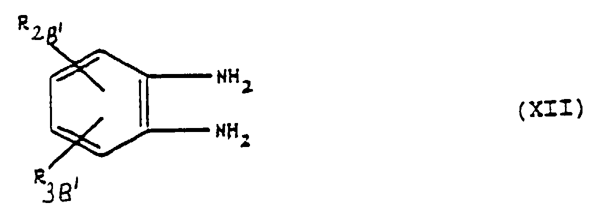 worin R_{2B}' und R_{3B}' die vorstehend angegebenen Bedeutungen haben, mit einem Produkt der Formel (XIII)
R_{4B}'-CO-CO₂alk (XIII)
zur Reaktion bringt, worin R_{4B}' die vorstehend angegebene Bedeutung hat, und alk einen Alkylrest mit höchstens 6 Kohlenstoffatomen darstellt, um nach Cyclisierung Produkte der Formel (II_{d}) 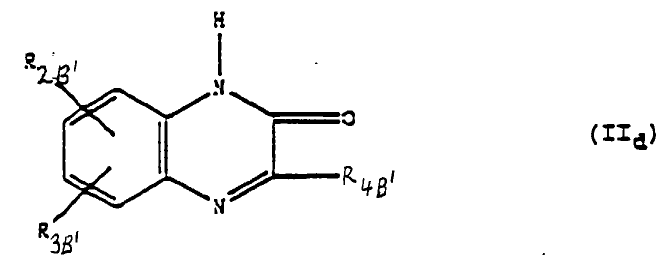 worin R_{2B}', R_{3B}' und R_{4B}' die vorstehend angegebenen Bedeutungen haben, zu erhalten,
e) oder daß man ein Produkt der Formel (XIV) 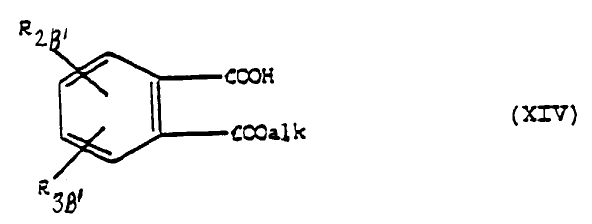 worin R_{2B}', R_{3B}' und alk die vorstehend angegebenen Bedeutungen haben, gewünschtenfalls nach einer Halogenierungsreaktion der freien Carboxygruppe, einer Additionsreaktion an dieser Carboxygruppe einer Verbindung der Formel R_{4B}'-H unterwirft, wobei R_{4B}' die vorstehend angegebene Bedeutung hat, um nach Cyclisierung in Gegenwart von Hydrazin oder einem Derivat Produkte der Formel (IIₑ) 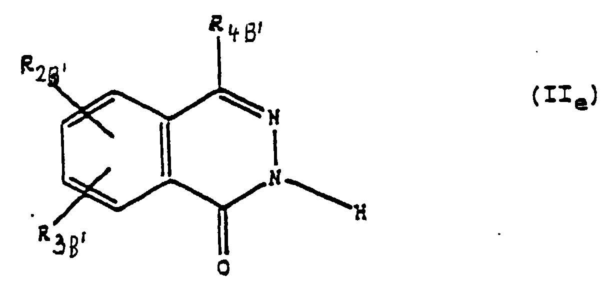 worin R_{2B}', R_{3B}' und R_{4B}' die vorstehend angegebenen Bedeutungen haben,
f) oder daß man ein Produkt der Formel (XV) 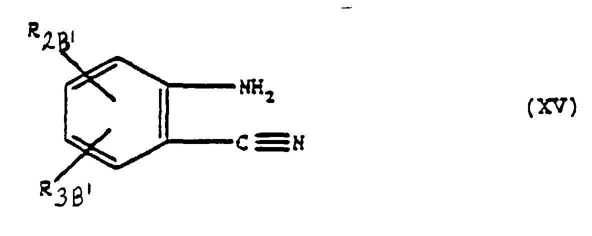 worin R_{2B}' und R_{3B}' die vorstehend angegebenen Bedeutungen haben, mit einem Produkt der Formel (XVI)
R_{4B}'-CO-Cl (XVI)
zur Umsetzung bringt, worin R_{4B}' die vorstehend angegebene Bedeutung hat, um das Produkt der Formel (XVII) 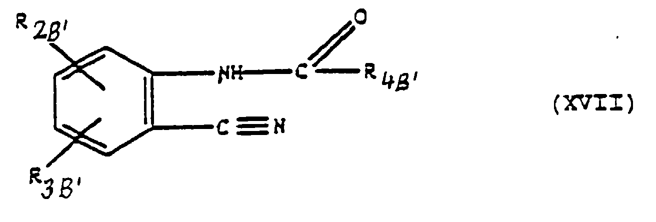 zu ergeben, worin R_{2B}', R_{3B}' und R_{4B}' die vorstehend angegebenen Bedeutungen haben, um nach Cyclisierung Produkte der Formel (II_{f}) 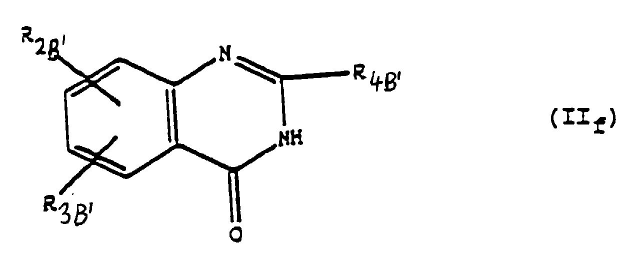 zu ergeben, worin R_{2B}', R_{3B}' und R_{4B}' die vorstehend angegebenen Bedeutungen haben,
Produkte der Formel (Iᵢ), welche Produkte der Formel (I_{B}) darstellen können, und Produkte der Formeln (II_{b}), (II_{c}), (II_{d}), (IIₑ) und (II_{f}), wie sie vorstehend definiert sind, welche Produkte der Formel (I_{B}) darstellen können, worin mindestens eines von A_{1B}, A_{2B}, A_{3B} und A_{4B} einen Methinrest bedeutet, der einen Hydroxylrest trägt, dass man gewünschtenfalls und falls notwendig einer oder mehreren der folgenden Reaktionen in irgendeiner Reihenfolge unterwirft:
- einer vollständigen Reduktionsreaktion des Hydroxyl- oder Oxorestes zum Methinrest gefolgt von einer Aromatisierung;
- an den Produkten der Formel (Iᵢ), worin eines von R_{4B}', R_{4B}'' oder R_{4B}''' einen Hydroxylrest bedeutet oder an den Produkten der Formeln (II_{b}), (II_{c}), (II_{d}), (IIₑ) und (II_{f}), entweder zunächst eine Substitutionsreaktion des Hydroxylrestes durch ein Halogenatom, gefolgt von der Einwirkung eines Produkts der Formel Rₚ₄ - M - Hal, worin Rₚ₄ die in Anspruch 1 für R_{4B} angegebene Bedeutung hat, worin die gegebenenfalls vorhandenen reaktiven Funktionen gegebenenfalls geschützt sind durch Schutzgruppen, M ein Metallatom ausgewählt unter Magnesium, Kupfer und Zink, darstellt, und Hai ein Halogenatom bedeutet,
oder der Einwirkung eines Produkts der Formel Rₚ₄ - Hal unterwirft, worin Hai ein Halogenatom bedeutet, um die entsprechenden Produkte der Formel (I_{B}) zu erhalten,
- einer Umwandlungsreaktion der Oxofunktion (= O) zur Thioxofunktion (= S) durchführt,
- einer Eliminierungsreaktion der Schutzgruppen, welche die geschützten reaktiven Funktionen tragen können, unterwirft,
- eine Salzbildungsreaktion mit einer Mineral- oder organischen Säure oder mit einer Mineral- oder organischen Base durchführt, um das entsprechende Salz zu erhalten,
- eine Veresterungsreaktion der Säurefunktion durchführt,
- eine Verseifungsreaktion der Esterfunktion zur Säurefunktion durchführt,
- eine Umwandlungsreaktion der Alkyloxyfunktion zur Hydroxylfunktion durchführt,
- eine Umwandlungsreaktion der Cyanofunktion zur Säurefunktion durchführt,
- eine Reduktionsreaktion der Carboxyfunktion zur Alkoholfunktion durchführt,
- eine Aufspaltungsreaktion der racemischen Formen durchführt,
wobei die so erhaltenen Produkte der Formel (I_{B}) unter allen möglichen isomeren Formen, racemischen, enantiomeren und diastereoisomeren, vorliegen.

5. Als Arzneimittel die Produkte, wie durch die Formel (I_{B}) von Anspruch 1 definiert, wobei diese Produkte der Formel (I_{B}) unter allen möglichen isomeren Formen, racemischen, enantiomeren und diastereoisomeren, vorliegen, sowie die Additionssalze mit Mineral- und organischen Säuren oder mit Mineral- und organischen Basen, die für die Produkte der Formel (I_{B}) pharmazeutisch annehmbar sind.

6. Als Arzneimittel die Produkte, wie durch die Formel (Iₐ) von Anspruch 2 definiert, sowie die Additionssalze mit Mineral- und organischen Säuren oder mit Mineral- und organischen Basen, die für die Produkte der Formel (Iₐ) pharmazeutisch annehmbar sind.

7. Pharmazeutische Zusammensetzungen, enthaltend als Wirkstoff mindestens eines der Arzneimittel, wie in einem der Ansprüche 5 und 6 definiert.

8. Als neue industrielle Produkte die Verbindungen der Formeln (VIII), (II_{b}), (II_{c}), (II_{d}), (IIₑ) und (II_{f}), wie in Anspruch 4 definiert, mit Ausnahme der folgenden Verbindungen
a) die Verbindungen der Formel (VIII), worin R_{2'B} und R_{3'B} = H, R_{4'B} = CO₂Et, Me, Et, CF₃, H und R_{4''B} = CO₂Et;
sowie die Verbindung
Ethyl-2-[1-[2-(trifluoromethyl)-phenylamino]-pent-1-yliden]propandioat,
b) die Verbindungen der Formel (II_{b}), worin R_{4'B} = H, Hal, Alkyl mit 1 bis 4 Kohlenstoffatomen und R_{4''B} = CF₃,
sowie die Verbindung
Ethyl-2-butyl-4-hydroxy-8-trifluoromethyl-3-chinolincarboxylat,
c) die Verbindungen der Formel (II_{c}), worin R'_{2B} und R'_{3B} eines Wasserstoff und das andere Phenyl bedeuten, und R₄'''_{B} bedeutet Carboxy frei oder verestert,
d) die Verbindung der Formel (IIₑ): 4-Butyl-1-phthalazinon,
e) die Verbindungen der Formel (II_{f}), worin R_{2'B} und R_{3'B} H, Hal, CF₃ oder Alkyl bedeuten, und R'_{4B} Alkyl oder CF₃ bedeutet.

9. Verwendung der Produkte der Formel (I_{B}), wie in Anspruch 1 definiert, wobei diese Produkte der Formel (I_{B}) unter allen möglichen isomeren Formen, racemischen, enantiomeren und diastereoisomeren, vorliegen, sowie die Säureadditionssalze mit Mineral- und organischen Säuren oder mit Mineral- und organischen Basen dieser Produkte der Formel (I_{B}) zur Herstellung von Arzneimitteln, die bestimmt sind entweder zur Behandlung der arteriellen Hypertension, von Herzinsuffizienzen, Niereninsuffizienzen und bei der Vorbeugung von postangioplastischen Restenosen,
oder zur Behandlung von gewissen gastrointestinalen oder gynäkologischen Störungen.

## Patentansprüche (Patentansprüche für folgende(n) Vertragsstaat(en): ES)

1. Verfahren zur Herstellung von Produkten der Formel (I_{B}): 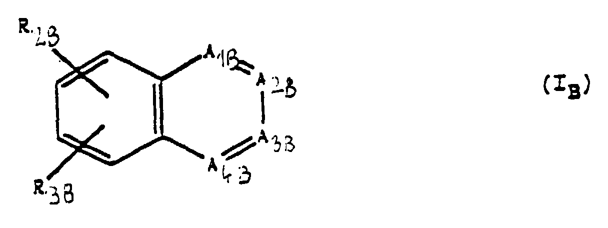 worin:
R_{2B} und R_{3B}, die identisch oder verschieden sind, ausgewählt sind aus der Gruppe gebildet durch:
- das Wasserstoffatom,
- die Halogenatome,
- den Hydroxylrest,
- den Rest Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Kohlenstoffatomen,
- die Reste Alkyl, Alkenyl, Alkyloxy und Alkylthio linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, den Resten Alkyloxy mit höchstens 4 Kohlenstoffatomen, Trifluoromethyl, Cyano, Acyl, Carboxy frei, in Salzform oder verestert, Tetrazol, Isoxazol, Pyrrolidinyl, Pyrrolidinylcarbonyl und Phenyl gegebenenfalls substituiert durch einen oder mehrere Reste, ausgewählt unter den Halogenatomen, dem Hydroxylrest, den Resten Alkyl und Alkoxy mit höchstens 4 Kohlenstoffatomen,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Pyrrolidinylcarbonyl, Piperazinylcarbonyl, wobei alle Piperazinylreste dieser Reste gegebenenfalls substituiert sind am zweiten Stickstoffatom durch einen Alkyl- oder Phenylrest, wobei diese Alkyl- und Phenylreste ihrerseits gegebenenfalls substituiert sind durch einen oder mehrere Reste ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert, Tetrazol und Isoxazol,
A_{1B}, A_{2B}, A_{3B} und A_{4B} sind derart, daß:
- eines ein Stickstoffatom darstellt,
- ein anderes bedeutet ein Stickstoffatom oder den Methinrest substituiert durch R_{4b},
- ein anderes bedeutet den Methinrest substituiert durch R_{4c},
- und das letzte bedeutet den Methinrest substituiert durch den Benzylrest, der seinerseits substituiert ist durch einen oder mehrere Reste ausgewählt unter den Resten Cyano, Carboxy frei, in Salzform oder verestert,
R_{4b} und R_{4c}, die identisch oder verschieden sind, sind ausgewählt unter den Werten von R_{4B}, derart,
so daß R_{4B} bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen,
wobei diese Produkte der Formel (I_{B}) unter allen möglichen isomeren Formen, racemisch, enantiomer und diastereoisomer, vorliegen, sowie die Additionssalze mit Mineral- und organischen Säuren oder mit Mineral- und organischen Basen dieser Produkte der Formel (I_{B}),
dadurch gekennzeichnet, daß man:
a) entweder die Verbindung der Formel (III): 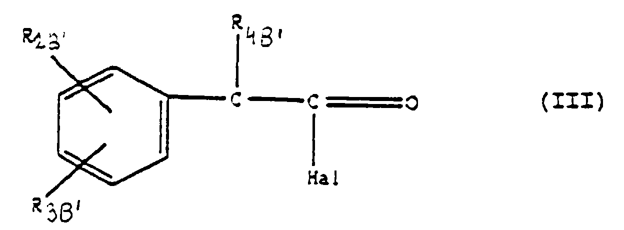 worin R_{2B}', R_{3B}' und R_{4B}' die vorstehend angegebenen Bedeutungen für R_{2B}, R_{3B} und R_{4B} haben, worin die eventuellen reaktiven Funktionen gegebenenfalls durch Schutzgruppen geschützt sind und Hal ein Halogenatom bedeutet,
einer Substitutionsreaktion unterwirft, um das Produkt der Formel (IV): 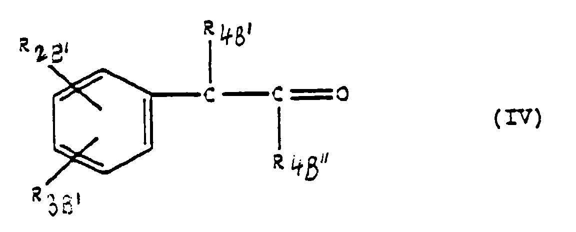 zu erhalten, worin R_{2B}', R_{3B}' und R_{4B}' die vorstehend angegebenen Bedeutungen haben, und R_{4B}'', identisch oder verschieden von R_{4B}', die vorstehend für R_{4B} angegebene Bedeutung hat, worin die eventuellen reaktiven Funktionen gegebenenfalls durch Schutzgruppen geschützt sind, das man mit einer Verbindung der Formel (V):
R_{4B}'''-C≡N (V)
zur Reaktion bringt, worin R_{4B}''', das identisch oder verschieden von R_{4B}' oder R_{4B}'' ist, die vorstehend für R_{4B} angegebene Bedeutung hat, worin die eventuellen reaktiven Funktionen gegebenenfalls durch Schutzgruppen geschützt sind, um nach Cyclisierung ein Produkt der Formel (Iᵢ) 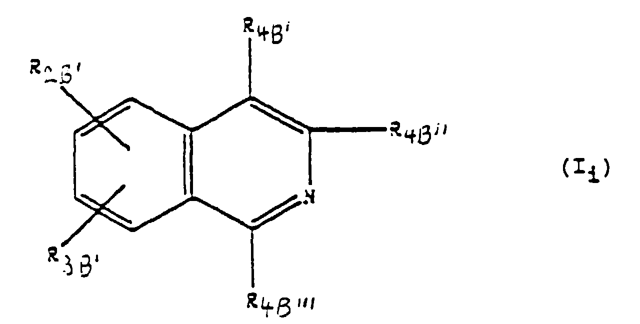 zu erhalten, worin R_{2B}', R_{3B}', R_{4B}', R_{4B}'' und R_{4B}''' die vorstehend angegebenen Bedeutungen haben,
b) oder man bringt ein Produkt der Formel (VI): 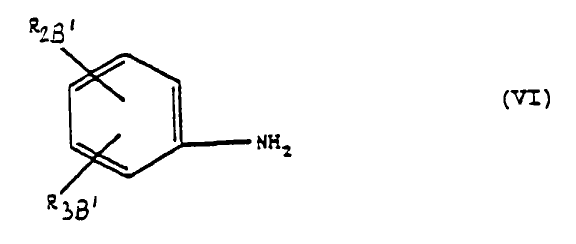 worin R_{2B}' und R_{3B}' die vorstehend angegebenen Bedeutungen haben, zur Reaktion mit einem Produkt der Formel (VII):
R_{4B}'-CH(CO₂alk)-CO-R_{4B}'' (VII)
worin R_{4B}' und R_{4B}'', die identisch oder verschieden sind, die vorstehend angegebenen Bedeutungen haben, und alk einen Alkylrest mit höchstens 6 Kohlenstoffatomen darstellt, um Produkte der Formel (VIII): 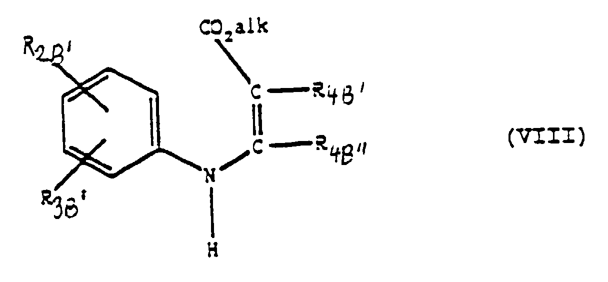 zu erhalten, worin R_{2B}', R_{3B}', R_{4B}', R_{4B}'' und alk die vorstehend angegebenen Bedeutungen haben, die man cyclisiert, um Produkte der Formel (II_{b}): 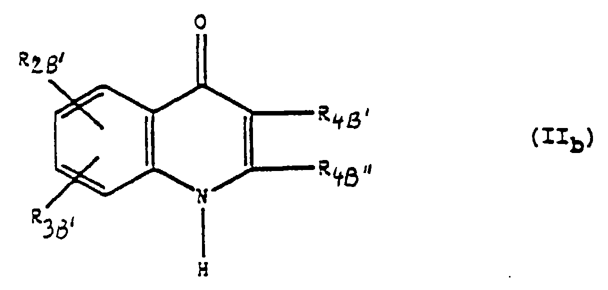 zu erhalten, worin R_{2B}', R_{3B}', R_{4B}' und R_{4B}'' die vorstehend angegebenen Bedeutungen haben,
c) oder man bringt ein Produkt der Formel (IX) 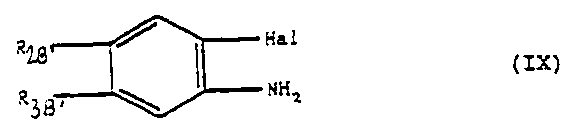 worin R_{2B}' und R_{3B}' die vorstehend angegebenen Bedeutungen haben, mit einem Produkt der Formel (X)
R_{4B}'''-C≡CH (X)
worin R_{4B}''' die vorstehend angegebenen Bedeutung hat, zur Reaktion, um Produkte der Formel (XI) 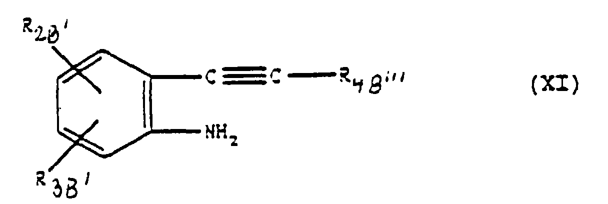 zu erhalten, worin R_{2B}', R_{3B}' und R_{4B}''' die vorstehend angegebenen Bedeutungen haben, die man einer Cyclisierungsreaktion in Gegenwart eines Stickstoffdonators, wie Natriumnitrit, unterwirft, um ein Produkt der Formel (II_{c}) 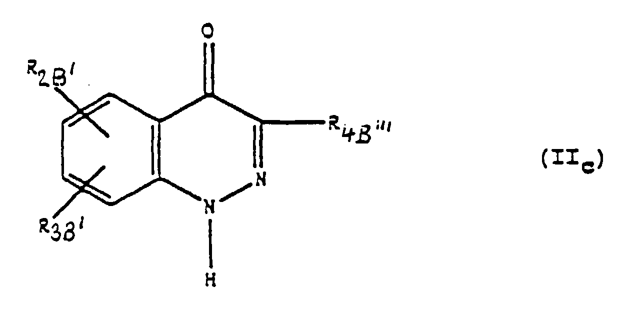 zu erhalten, worin R_{2B}', R_{3B}' und R_{4B}''' die vorstehend angegebenen Bedeutungen haben,
d) oder man bringt ein Produkt der Formel (XII) 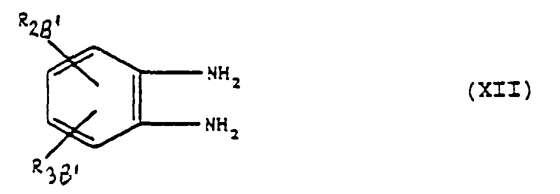 worin R_{2B}' und R_{3B}' die vorstehend angegebenen Bedeutungen haben, mit einem Produkt der Formel (XIII)
R_{4B}'-CO-CO₂alk (XIII)
zur Reaktion, worin R_{4B}' die vorstehend angegebene Bedeutung hat, und alk einen Alkylrest mit höchstens 6 Kohlenstoffatomen darstellt, um nach Cyclisierung der Produkte der Formel (II_{d}) 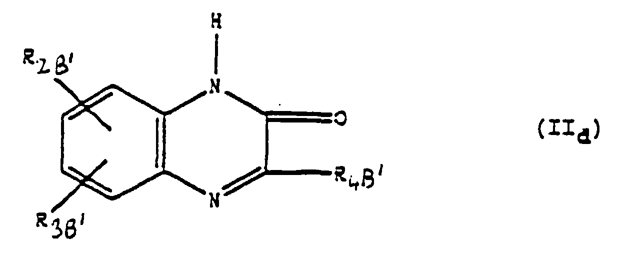 zu erhalten, worin R_{2B}', R_{3B}' und R_{4B}' die vorstehend angegebenen Bedeutungen haben,
e) oder man unterwirft ein Produkt der Formel (XIV) 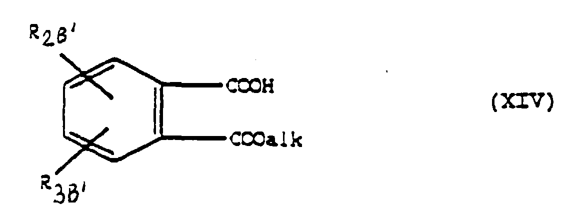 worin R_{2B}', R_{3B}' und alk die vorstehend angegebenen Bedeutungen haben, gewünschtenfalls nach einer Halogenierungsreaktion der freien Carboxyfunktion, einer Additionsreaktion auf dieser Carboxyfunktion einer Verbindung der Formel R_{4B}'-H, wobei R_{4B}' die vorstehend angegebene Bedeutung hat, um nach Cyclisierung in Gegenwart von Hydrazin oder einem Derivat Produkte der Formel (IIₑ) 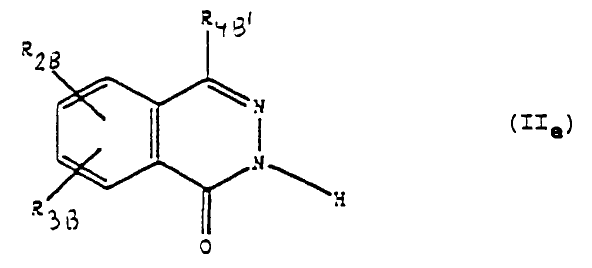 zu erhalten, worin R_{2B}', R_{3B}' und R_{4B}' die vorstehend angegebenen Bedeutungen haben,
f) oder man bringt ein Produkt der Formel (XV) 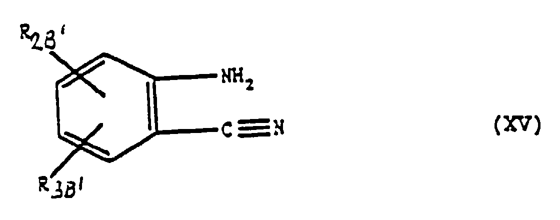 worin R_{2B}' und R_{3B}, die vorstehend angegebenen Bedeutungen haben, mit einem Produkt der Formel (XVI)
R_{4B}'-CO-Cl (XVI)
worin R_{4B}' die vorstehend angegebene Bedeutung hat, zur Reaktion, um das Produkt der Formel (XVII) 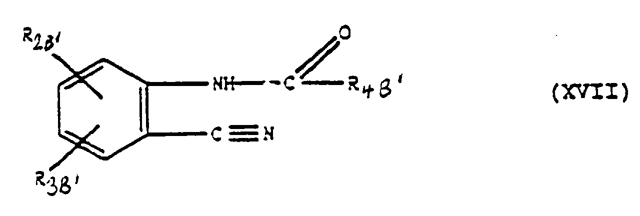 zu ergeben, worin R_{2B}', R_{3B}' und R_{4B}' die vorstehend angegebenen Bedeutungen haben, um nach Cyclisierung Produkte der Formel (II_{f}) 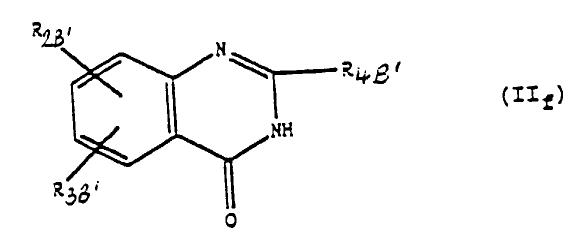 zu ergeben, worin R_{2B}', R_{3B}' und R_{4B}' die vorstehend angegebenen Bedeutungen haben,
Produkte der Formel (Iᵢ), welche Produkte der Formel (I_{B}) darstellen können, und Produkte der Formeln (II_{b}), (II_{c}), (II_{d}), (IIₑ) und (II_{f}), wie vorstehend definiert, welche Produkte der Formel (I_{B}) darstellen können, worin mindestens eines von A_{1B}, A_{2B}, A_{3B} und A_{4B} einen Methinrest darstellt, der einen Hydroxylrest trägt, die man gewünschtenfalls und falls notwendig einer oder mehreren der folgenden Reaktionen in irgendeiner Reihenfolge unterwirft:
- einer vollständigen Reduktionsreaktion des Hydroxyl- oder Oxorestes zum Methinrest gefolgt von einer Aromatisierung,
- auf die Produkte der Formel (Iᵢ), worin eines von R_{4B}', R_{4B}'' oder R_{4B}''' einen Hydroxylrest bedeutet, oder auf die Produkte der Formeln (II_{b}), (II_{c}), (II_{d}), (IIₑ) und (II_{f}), wendet man entweder zunächst eine Substitutionsreaktion des Hydroxylrestes durch ein Halogenatom, gefolgt von der Einwirkung eines Produkts der Formel Rₚ₄ - M - Hal, an, worin Rₚ₄ die in Anspruch 1 für R_{4B} angegebene Bedeutung hat, worin die gegebenenfalls vorhandenen reaktiven Funktionen gegebenenfalls geschützt sind durch Schutzgruppen, M ein Metallatom, ausgewählt unter Magnesium, Kupfer und Zink, darstellt, und Hal ein Halogenatom bedeutet,
oder man unterwirft der Einwirkung eines Produkts der Formel Rₚ₄ Hal, worin Hal ein Halogenatom bedeutet,
um die entsprechenden Produkte der Formel (I_{B}) zu erhalten,
- eine Umwandlungsreaktion der Oxofunktion (= O) zur Thioxofunktion (= S) durchführt,
- einer Eliminierungsreaktion der Schutzgruppen, welche die geschützten reaktiven Funktionen tragen können, unterwirft,
- einer Salzbildungsreaktion mit einer Mineral- oder organischen Säure oder mit einer Mineral- oder organischen Base, um das entsprechende Salz zu erhalten,
- einer Veresterungsreaktion der Säurefunktion,
- einer Verseifungsreaktion der Esterfunktion zur Säurefunktion,
- einer Umwandlungsreaktion der Alkyloxyfunktion zur Hydroxylfunktion,
- einer Umwandlungsreaktion der Cyanofunktion zur Säurefunktion,
- einer Reduktionsreaktion der Carboxyfunktion zur Alkoholfunktion,
- einer Aufspaltungsreaktion der racemischen Formen,
wobei diese so erhaltenen Produkte der Formel (I_{B}) unter allen möglichen isomeren Formen, racemischen, enantiomeren und diastereoisomeren, vorliegen.

2. Verfahren gemäß Anspruch 1 zur Herstellung der Produkte der Formel (I_{B}), wie in Anspruch 1 definiert, und entsprechend der Formel (Iₐ): 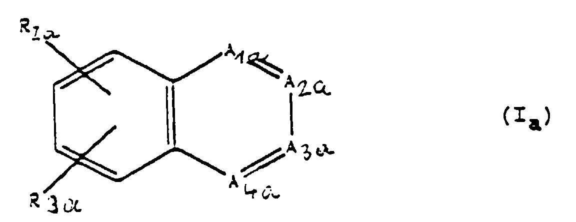 worin:
R₂ₐ und R₃ₐ, die identisch oder verschieden sind, ausgewählt sind aus der Gruppe gebildet durch:
- das Wasserstoffatom,
- die Halogenatome,
- den Hydroxylrest,
- die Reste Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Kohlenstoffatomen,
- die Reste Alkyl, Alkyloxy und Alkylthio linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, den Alkyloxyresten mit höchstens 4 Kohlenstoffatomen, Trifluoromethyl, Cyano, Acyl, Carboxy frei, in Salzform oder verestert, Tetrazol und Isoxazol,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Piperazinylcarbonyl, wobei alle Piperazinylreste dieser Reste gegebenenfalls am zweiten Stickstoffatom substituiert sind durch einen Alkyl- oder Phenylrest, wobei diese Alkyl- und Phenylreste ihrerseits gegebenenfalls substituiert sind durch einen oder mehrere Reste ausgewählt unter den Halogenatomen, den Hydroxyl-, Nitro-, Alkyl-, Alkyloxy- oder Acylresten mit höchstens 4 Kohlenstoffatomen, Trifluoromethyl, Cyano, Carboxy frei, in Salzform oder verestert, Tetrazol und Isoxazol,
A₁ₐ, A₂ₐ , A₃ₐ und A₄ₐ sind derart, daß eines ein Stickstoffatom bedeutet, ein anderes ein Stickstoffatom oder den Methinrest substituiert durch R_{4ab} darstellt, ein anderes den Methinrest substituiert durch R_{4ac} darstellt, und das letzte den Methinrest substituiert durch den Benzylrest bedeutet, der seinerseits substituiert ist durch einen oder mehrere Reste ausgewählt unter den Resten Cyano, Carboxy, frei, in Salzform oder verestert,
R_{4ab} und R_{4ac}, die identisch oder verschieden sind, sind ausgewählt unter den Werten von R₄ₐ, derart, daß R₄ₐ bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen,
wobei diese Produkte der Formel (Iₐ) nicht die folgenden Produkte bedeuten können:
entweder R₂ₐ und R₃ₐ sind ausgewählt unter den Halogenatomen, den Resten Hydroxyl, Alkyl und Alkyloxy und A₁ₐ, A₂ₐ, A₃ₐ und A₄ₐ sind derart, daß:
- eines ein Stickstoffatom darstellt,
- ein anderes den Methinrest oder das Stickstoffatom bedeutet,
- ein anderes den Methinrest bedeutet, wobei diese Methinreste gegebenenfalls substituiert sind durch einen Rest ausgewählt unter den Carboxyresten frei oder verestert, Benzoyl, Phenyl gegebenenfalls substituiert durch einen Alkylrest mit höchstens 2 Kohlenstoffatomen, seinerseits gegebenenfalls substituiert durch einen Hydroxyl- oder Acyloxyrest,
- und das letzte bedeutet den Benzylrest gegebenenfalls substituiert durch einen oder mehrere Reste ausgewählt unter den Halogenatomen, den Resten Alkyl, Alkyloxy, Amino, Nitro und Acetyl,
oder aber R₂ₐ und R₃ₐ, die identisch sind, bedeuten den Methoxyrest, A₁ₐ bedeutet den Benzylrest substituiert durch ein Chloratom, A₂ₐ bedeutet den Methinrest substituiert durch den Isopropyl- oder Isobutylrest, A₃ₐ bedeutet das Stickstoffatom, und A₄ₐ bedeutet den Methinrest,
wobei diese Produkte der Formel (Iₐ) unter allen möglichen isomeren Formen, racemischen, enantiomeren und diastereoisomeren, vorliegen, sowie die Additionssalze mit Mineral- und organischen Säuren oder mit Mineral- und organischen Basen dieser Produkte der Formel (Iₐ), dadurch gekennzeichnet, daß man die Ausgangsprodukte und die Reaktionsteilnehmer derart auswählt, daß man die Produkte der Formel (Iₐ), wie vorstehend definiert, herstellt.

3. Verfahren gemäß Anspruch 1 zur Herstellung von Produkten der Formel (I_{B}), worin:
R_{2B} und R_{3B} ein Wasserstoffatom darstellen,
A_{1B}, A_{2B} und A_{3B} sind derart, daß eines oder zwei davon ein Stickstoffatom bedeutet und die anderen, die identisch oder verschieden sind, den Rest = 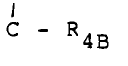 bedeuten, derart, daß R_{4B} ausgewählt ist unter den Wasserstoffatomen, dem n-Butylrest und Alkylthio mit höchstens 4 Kohlenstoffatomen,
und A_{4B} bedeutet den Methinrest substituiert durch den Benzylrest gegebenenfalls substituiert durch einen oder mehrere Reste ausgewählt unter den Resten Cyano, Carboxy frei, in Salzform oder verestert,
wobei diese Produkte der Formel (I_{B}) unter allen möglichen isomeren Formen, racemischen, enantiomeren oder diastereoisomeren, vorliegen, sowie die Additionssalze mit Mineral- und organischen Säuren oder mit Mineral- und organischen Basen dieser Produkte der Formel (I_{B}), dadurch gekennzeichnet, daß man die Ausgangsprodukte und die Reaktionsteilnehmer derart auswählt, daß man die Produkte der Formel (I_{B}), wie vorstehend definiert, herstellt.

## Patentansprüche (Patentansprüche für folgende(n) Vertragsstaat(en): GR)

1. Verfahren zur Herstellung von Produkten der Formel (I_{B}): 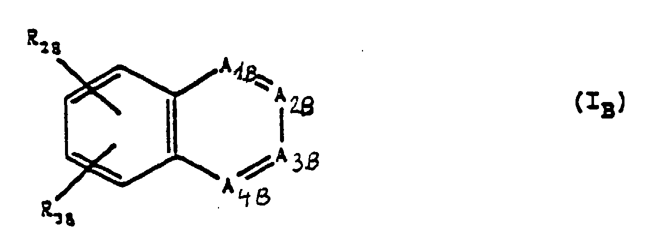 worin:
R_{2B} und R_{3B}, die identisch oder verschieden sind, ausgewählt sind aus der Gruppe gebildet durch:
- das Wasserstoffatom,
- die Halogenatome,
- den Hydroxylrest,
- den Rest Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Kohlenstoffatomen,
- die Reste Alkyl, Alkenyl, Alkyloxy und Alkylthio linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, den Resten Alkyloxy mit höchstens 4 Kohlenstoffatomen, Trifluoromethyl, Cyano, Acyl, Carboxy frei, in Salzform oder verestert, Tetrazol, Isoxazol, Pyrrolidinyl, Pyrrolidinylcarbonyl und Phenyl gegebenenfalls substituiert durch einen oder mehrere Reste, ausgewählt unter den Halogenatomen, dem Hydroxylrest, den Resten Alkyl und Alkoxy mit höchstens 4 Kohlenstoffatomen,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Pyrrolidinylcarbonyl, Piperazinylcarbonyl, wobei alle Piperazinylreste dieser Reste gegebenenfalls am zweiten Stickstoffatom substituiert sind durch einen Alkyl- oder Phenylrest, wobei diese Alkyl- und Phenylreste ihrerseits gegebenenfalls substituiert sind durch einen oder mehrere Reste ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert, Tetrazol und Isoxazol,
A_{1B}, A_{2B}, A_{3B} und A_{4B} sind derart, daß:
- das eine ein Stickstoffatom darstellt,
- ein anderes ein Stickstoffatom oder den Methinrest substituiert durch R_{4b} darstellt,
- ein anderes den Methinrest substituiert durch R_{4c} darstellt,
- und das letzte bedeutet den Methinrest substituiert durch den Benzylrest, der seinerseits substituiert ist durch einen oder mehrere Reste ausgewählt unter den Resten Cyano, Carboxy frei, in Salzform oder verestert,
R_{4b} und R_{4c}, die identisch oder verschieden sind, sind ausgewählt unter den Werten von R_{4B}, derart,
daß R_{4B} bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen,
wobei diese Produkte der Formel (I_{B}) unter allen möglichen isomeren Formen, racemisch, enantiomer oder diastereoisomer, vorliegen, sowie die Additionssalze mit Mineral- und organischen Säuren oder mit Mineral- und organischen Basen dieser Produkte der Formel (I_{B}),
dadurch gekennzeichnet, daß man:
a) entweder die Verbindung der Formel (III): 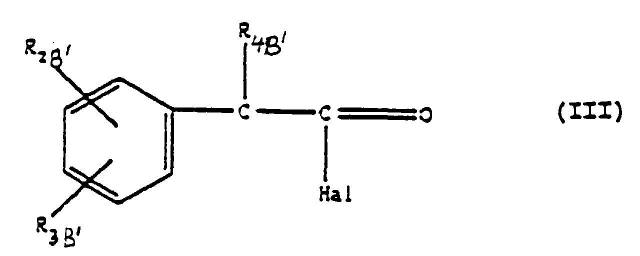 worin R_{2B}', R_{3B}' und R_{4B}' die vorstehend angegebenen Bedeutungen für R_{2B}, R_{3B} und R_{4B} haben, worin die eventuellen reaktiven Funktionen gegebenenfalls durch Schutzgruppen geschützt sind und Hal ein Halogenatom bedeutet,
einer Substitutionsreaktion unterwirft, um das Produkt der Formel (IV): 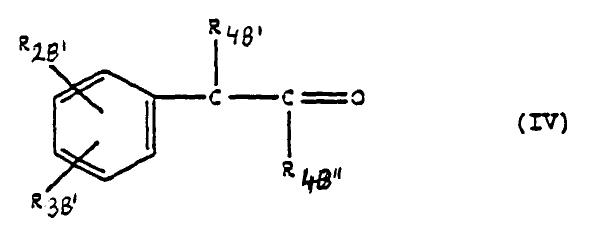 zu erhalten, worin R_{2B}', R_{3B}' und R_{4B}' die vorstehend angegebenen Bedeutungen haben, und R_{4B}'', identisch oder verschieden von R_{4B}', die vorstehend für R_{4B} angegebene Bedeutung hat, worin die eventuellen reaktiven Funktionen gegebenenfalls durch Schutzgruppen geschützt sind, das man mit einer Verbindung der Formel (V):
R_{4B}'''-C≡N (V)
zur Reaktion bringt, worin R_{4B}''', das identisch oder verschieden von R_{4B}' oder R_{4B}'' ist, die vorstehend für R_{4B} angegebene Bedeutung hat, worin die eventuellen reaktiven Funktionen gegebenenfalls durch Schutzgruppen geschützt sind, um nach Cyclisierung ein Produkt der Formel (Iᵢ) 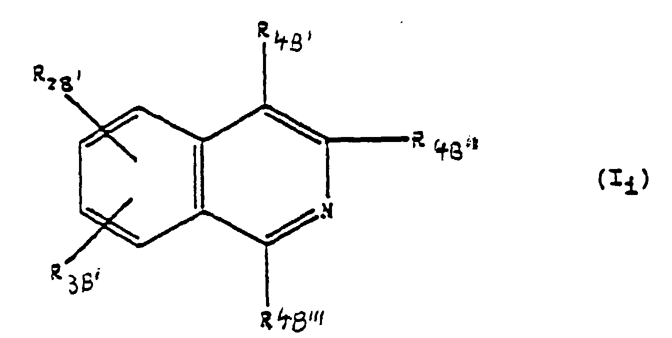 zu erhalten, worin R_{2B}', R_{3B}', R_{4B}', R_{4B}'' und R_{4B}''' die vorstehend angegebenen Bedeutungen haben,
b) oder man bringt ein Produkt der Formel (VI): 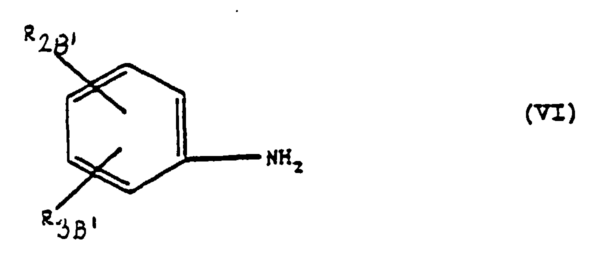 worin R_{2B}' und R_{3B}' die vorstehend angegebenen Bedeutungen haben, zur Reaktion mit einem Produkt der Formel (VII):
R_{4B}'-CH(CO₂alk)-CO-R_{4B}'' (VII)
worin R_{4B}' und R_{4B}'', die identisch oder verschieden sind, die vorstehend angegebenen Bedeutungen haben, und alk einen Alkylrest mit höchstens 6 Kohlenstoffatomen darstellt, um Produkte der Formel (VIII): 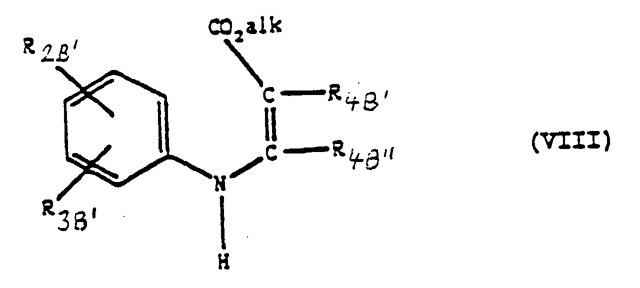 zu erhalten, worin R_{2B}', R_{3B}', R_{4B}', R_{4B}'' und alk die vorstehend angegebenen Bedeutungen haben, die man cyclisiert, um Produkte der Formel (II_{b}): 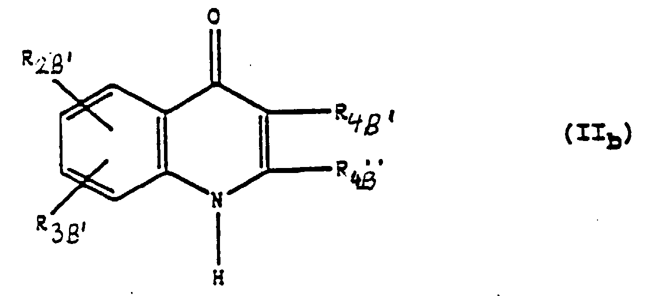 zu erhalten, worin R_{2B}', R_{3B}', R_{4B}' und R_{4B}'' die vorstehend angegebenen Bedeutungen haben,
c) oder man bringt ein Produkt der Formel (IX) 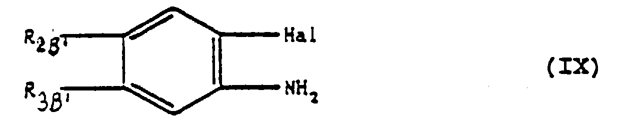 worin R_{2B}' und R_{3B}' die vorstehend angegebenen Bedeutungen haben, mit einem Produkt der Formel (X)
R_{4B}'''-C≡CH (X)
worin R_{4B}''' die vorstehend angegebene Bedeutung hat, zur Reaktion, um Produkte der Formel (XI) 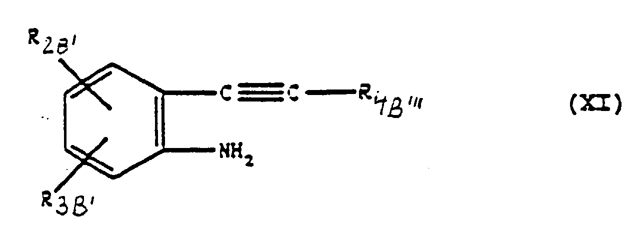 zu erhalten, worin R_{2B}', R_{3B}' und R_{4B}''' die vorstehend angegebenen Bedeutungen haben, die man einer Cyclisierungsreaktion in Gegenwart eines Stickstoffdonators, wie Natriumnitrit, unterwirft, um ein Produkt der Formel (II_{c}) 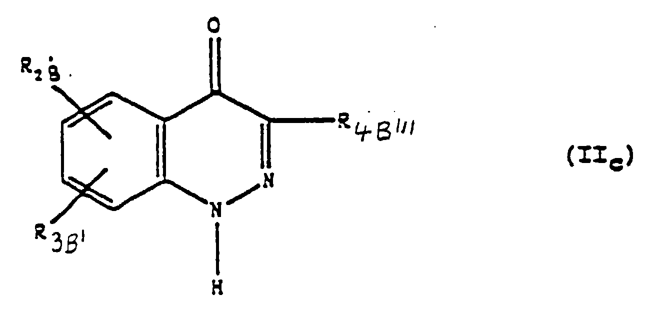 zu erhalten, worin R_{2B}', R_{3B}' und R_{4B}''' die vorstehend angegebenen Bedeutungen haben,
d) oder man bringt ein Produkt der Formel (XII) 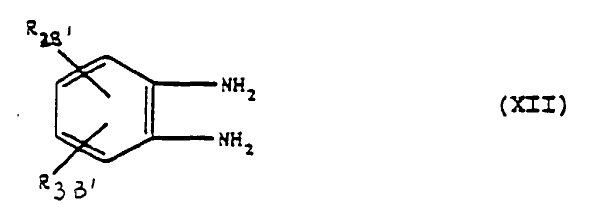 worin R_{2B}' und R_{3B}' die vorstehend angegebenen Bedeutungen haben, mit einem Produkt der Formel (XIII)
R_{4B}'-CO-CO₂alk (XIII)
worin R_{4B}' die vorstehend angegebene Bedeutung hat, und alk einen Alkylrest mit höchstens 6 Kohlenstoffatomen darstellt, zur Reaktion, um nach Cyclisierung Produkte der Formel (II_{d}) 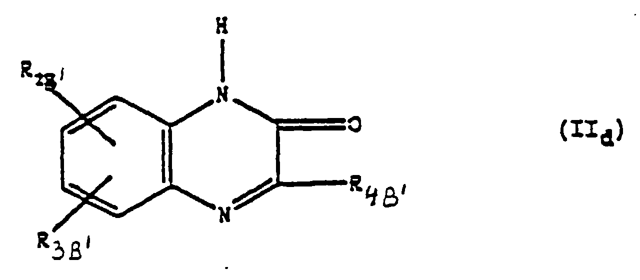 zu erhalten, worin R_{2B}', R_{3B}' und R_{4B}' die vorstehend angegebenen Bedeutungen haben,
e) oder man unterwirft ein Produkt der Formel (XIV) 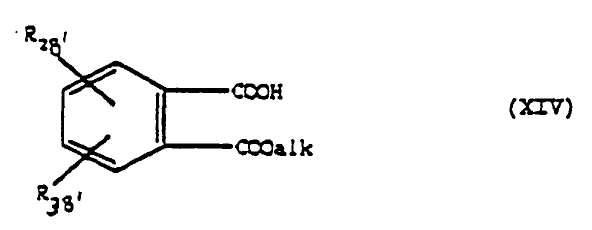 worin R_{2B}', R_{3B}' und alk die vorstehend angegebenen Bedeutungen haben, gewünschtenfalls nach einer Halogenierungsreaktion der freien Carboxyfunktion, einer Additionsreaktion auf dieser Carboxyfunktion einer Verbindung der Formel R_{4B}'-H, wobei R_{4B}' die vorstehend angegebene Bedeutung hat, um nach Cyclisierung in Gegenwart von Hydrazin oder einem Derivat Produkte der Formel (IIₑ) 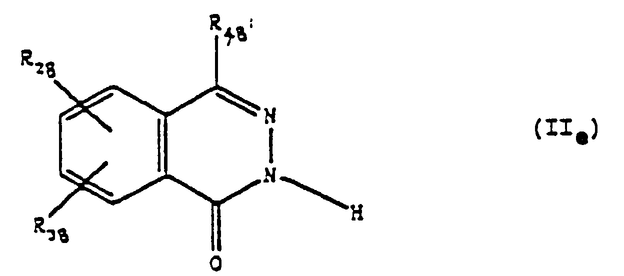 zu erhalten, worin R_{2B}', R_{3B}' und R_{4B}' die vorstehend angegebenen Bedeutungen haben,
f) oder man bringt ein Produkt der Formel (XV) 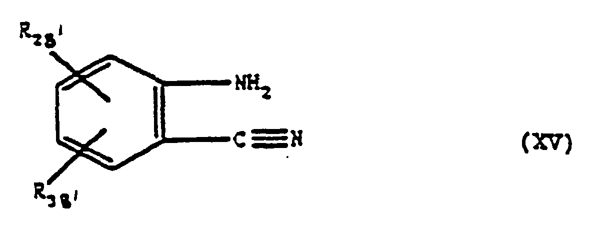 worin R_{2B}' und R_{3B}' die vorstehend angegebenen Bedeutungen haben, mit einem Produkt der Formel (XVI)
R_{4B}'-CO-Cl (XVI)
zur Reaktion, worin R_{4B}' die vorstehend angegebene Bedeutung hat, um das Produkt der Formel (XVII) 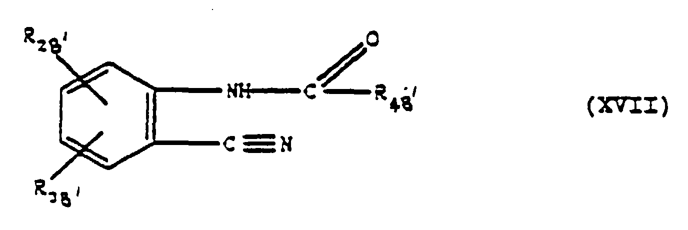 zu ergeben, worin R_{2B}', R_{3B}' und R_{4B}' die vorstehend angegebenen Bedeutungen haben, um nach Cyclisierung Produkte der Formel (II_{f}) 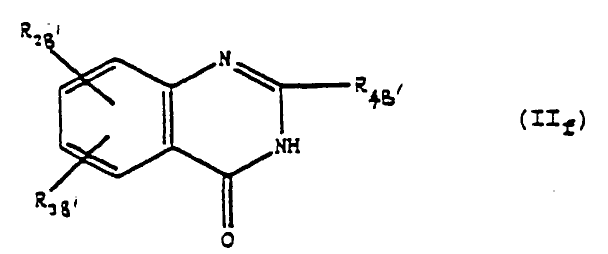 zu erhalten, worin R_{2B}', R_{3B}' und R_{4B}' die vorstehend angegebenen Bedeutungen haben,
Produkte der Formel (Iᵢ), welche Produkte der Formel (I_{B}) und Produkte der Formeln (II_{b}), (II_{c}), (II_{d}), (IIₑ) und (II_{f}), wie vorstehend definiert, darstellen können, und die Produkte der Formel (I_{B}) darstellen können, worin mindestens eines von A_{1B}, A_{2B}, A_{3B} und A_{4B} einen Methinrest darstellt, der einen Hydroxylrest trägt, die man gewünschtenfalls und falls notwendig einer oder mehreren der folgenden Reaktionen in irgendeiner Reihenfolge unterwirft:
- einer vollständigen Reduktionsreaktion des Hydroxyl- oder Oxorestes zum Methinrest gefolgt von einer Aromatisierung,
- auf die Produkte der Formel (Iᵢ), worin eines von R_{4B}', R_{4B}'' oder R_{4B}''' einen Hydroxylrest bedeutet, oder auf die Produkte der Formeln (II_{b}), (II_{c}), (II_{d}), (IIₑ) und (II_{f}) man entweder zunächst eine Substitutionsreaktion des Hydroxylrestes durch ein Halogenatom anwendet, gefolgt von der Einwirkung eines Produkts der Formel Rₚ₄ - M - Hal, worin Rₚ₄ die in Anspruch 1 für R_{4B} angegebene Bedeutung hat, worin die gegebenenfalls vorhandenen reaktiven Funktionen gegebenenfalls geschützt sind durch Schutzgruppen, M ein Metallatom, ausgewählt unter Magnesium, Kupfer und Zink, darstellt, und Hai ein Halogenatom bedeutet,
oder der Einwirkung eines Produkts der Formel Rₚ₄ Hal unterwirft, worin Hai ein Halogenatom bedeutet,
um die entsprechenden Produkte der Formel (I_{B}) zu erhalten,
- einer Umwandlungsreaktion der Oxofunktion (= O) zur Thioxofunktion (= S) unterwirft,
- einer Eliminierungsreaktion der Schutzgruppen, welche die geschützten reaktiven Funktionen tragen können,
- einer Salzbildungsreaktion mit einer Mineral- oder organischen Säure oder mit einer Mineral- oder organischen Base, um das entsprechende Salz zu erhalten,
- einer Veresterungsreaktion der Säurefunktion,
- einer Verseifungsreaktion der Esterfunktion zur Säurefunktion,
- einer Umwandlungsreaktion der Alkyloxyfunktion in eine Hydroxylfunktion,
- einer Umwandlungsreaktion der Cyanofunktion zur Säurefunktion,
- einer Reduktionsreaktion der Carboxyfunktion zur Alkoholfunktion,
- einer Aufspaltungsreaktion der racemischen Formen,
wobei die so erhaltenen Produkte der Formel (I_{B}) unter allen möglichen isomeren Formen, racemisch, enantiomer und diastereoisomer, vorliegen.

2. Verfahren gemäß Anspruch 1 zur Herstellung der Produkte der Formel (I_{B}), wie in Anspruch 1 definiert, und entsprechend der Formel (Iₐ): 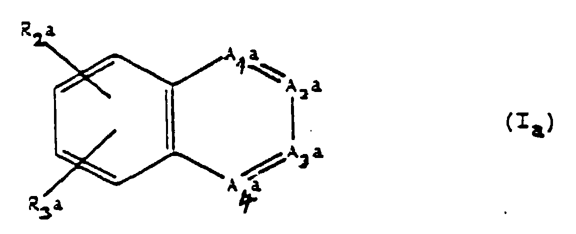 worin:
R₂ₐ und R₃ₐ, die identisch oder verschieden sind, ausgewählt sind aus der Gruppe gebildet durch:
- das Wasserstoffatom,
- die Halogenatome,
- den Hydroxylrest,
- die Reste Mercapto, Cyano, Nitro, Formyl, Benzoyl, Acyl mit höchstens 6 Kohlenstoffatomen,
- die Carboxyreste frei, in Salzform oder verestert durch einen linearen oder verzweigten Alkylrest mit höchstens 4 Kohlenstoffatomen,
- die Reste Alkyl, Alkyloxy und Alkylthio linear oder verzweigt mit höchstens 6 Kohlenstoffatomen, Phenyl, Naphthyl, Benzyl und Phenylthio, wobei alle diese Reste gegebenenfalls substituiert sind durch einen oder mehrere Reste, die identisch oder verschieden sein können, ausgewählt unter den Halogenatomen, dem Hydroxylrest, den Alkyloxyresten mit höchstens 4 Kohlenstoffatomen, Trifluoromethyl, Cyano, Acyl, Carboxy frei, in Salzform oder verestert, Tetrazol und Isoxazol,
- die Reste Amino, Mono- oder Dialkylamino, Carbamoyl, Pyrrolyl, Morpholino, Piperazinyl, Pyrrolylmethyl, Morpholinomethyl, Piperazinylmethyl, Pyrrolylcarbonyl, Morpholinocarbonyl, Piperazinylcarbonyl, wobei alle Piperazinylreste dieser Reste gegebenenfalls am zweiten Stickstoffatom substituiert sind durch einen Alkyl- oder Phenylrest, wobei diese Alkyl- und Phenylreste ihrerseits gegebenenfalls substituiert sind durch einen oder mehrere Reste ausgewählt unter den Halogenatomen, den Hydroxyl-, Nitro-, Alkyl-, Alkyloxy- oder Acylresten mit höchstens 4 Kohlenstoffatomen, Trifluoromethyl, Cyano, Carboxy frei, in Salzform oder verestert, Tetrazol und Isoxazol,
A₁ₐ, A₂ₐ, A₃ₐ und A₄ₐ sind derart, daß:
- eines ein Stickstoffatom darstellt,
- ein anderes ein Stickstoffatom oder den Methinrest, substituiert durch R_{4ab}, darstellt,
- ein anderes den Methinrest, substituiert durch R_{4ac}, darstellt, und
- der letzte den Methinrest, substituiert durch den Benzylrest, bedeutet, der seinerseits substituiert ist durch einen oder mehrere Reste ausgewählt unter den Resten Cyano, Carboxy, frei, in Salzform oder verestert,
R_{4ab} und R_{4ac}, die identisch oder verschieden sind, sind ausgewählt unter den Werten von R₄ₐ, derart,
daß R₄ₐ bedeutet:
- das Wasserstoffatom, den Rest Hydroxyl, Cyano, Carboxy frei, in Salzform oder verestert durch einen Alkylrest mit höchstens 4 Kohlenstoffatomen,
- den Rest Alkyl, Alkenyl, Alkyloxy, Acyl oder Alkylthio, wobei diese Reste linear oder verzweigt sind und höchstens 7 Kohlenstoffatome enthalten, wobei alle diese Reste gegebenenfalls substituiert sind durch einen oder mehrere Reste, ausgewählt unter den Halogenatomen, den Resten Hydroxyl, Nitro, Alkyl, Alkyloxy oder Acyl mit höchstens 4 Kohlenstoffatomen, Trifluoromethyl, Cyano, Carboxy frei, in Salzform oder verestert durch einen Alkylrest mit höchstens 4 Kohlenstoffatomen,
wobei diese Produkte der Formel (Iₐ) nicht die folgenden Produkte bedeuten können:
entweder R₂ₐ und R₃ₐ sind ausgewählt unter den Halogenatomen, den Resten Hydroxyl, Alkyl und Alkyloxy und A₁ₐ, A₂ₐ, A₃ₐ und A₄ₐ sind derart, daß:
- eines ein Stickstoffatom darstellt,
- ein anderes den Methinrest oder das Stickstoffatom bedeutet,
- ein anderes den Methinrest bedeutet,
wobei diese zwei Methinreste gegebenenfalls substituiert sind durch einen Rest, ausgewählt aus den Resten Carboxy, frei oder verestert, Benzoyl, Phenyl gegebenenfalls substituiert durch einen Alkylrest mit höchstens 2 Kohlenstoffatomen, der seinerseits gegebenenfalls substituiert ist durch einen Hydroxyl- oder Acyloxyrest,
- und das letzte bedeutet den Benzylrest gegebenenfalls substituiert durch einen oder mehrere Reste ausgewählt unter den Halogenatomen, den Resten Alkyl, Alkyloxy, Amino, Nitro und Acetyl,
oder R₂ₐ und R₃ₐ, die identisch sind, bedeuten den Methoxyrest, A₁ₐ bedeutet den Benzylrest substituiert durch ein Chloratom, A₂ₐ bedeutet den Methinrest substituiert durch den Isopropyl- oder Isobutylrest, A₃ₐ bedeutet das Stickstoffatom, und A₄ₐ bedeutet den Methinrest,
wobei diese Produkte der Formel (Iₐ) unter allen möglichen isomeren Formen, racemisch, enantiomer und diastereoisomer, vorliegen, sowie die Additionssalze mit Mineral- und organischen Säuren oder mit Mineral- und organischen Basen dieser Produkte der Formel (Iₐ), dadurch gekennzeichnet, daß man die Ausgangsprodukte und die Reaktionsteilnehmer derart auswählt, daß man die Produkte der Formel (Iₐ), wie vorstehend definiert, herstellt.

3. Verfahren gemäß Anspruch 1 zur Herstellung von Produkten der Formel (I_{B}), worin:
R_{2B} und R_{3B} ein Wasserstoffatom darstellen,
A_{1B}, A_{2B} und A_{3B} sind derart, daß eines oder zwei davon ein Stickstoffatom bedeutet und die anderen, die identisch oder verschieden sind, den Rest = C - R_{4B} bedeuten, derart, daß R_{4B} ausgewählt ist unter dem Wasserstoffatom, dem N-Butylrest und Alkylthio mit höchstens 4 Kohienstoffatomen,
und A_{4B} bedeutet den Methinrest substituiert durch den Benzylrest gegebenenfalls substituiert durch einen oder mehrere Reste ausgewählt unter den Resten Cyano, Carboxy frei, in Salzform oder verestert,
wobei diese Produkte der Formel (I_{B}) unter allen möglichen isomeren Formen, racemisch, enantiomer oder diastereoisomer, vorliegen, sowie die Additionssalze mit Mineral- und organischen Säuren oder mit Mineral- und organischen Basen dieser Produkte der Formel (I_{B}), dadurch gekennzeichnet, daß man die Ausgangsprodukte und die Reaktionsteilnehmer derart auswählt, daß man die Produkte der Formel (I_{B}), wie vorstehend definiert, herstellt.

4. Verfahren zur Herstellung von pharmazeutischen Zusammensetzungen, dadurch gekennzeichnet, daß man als Wirkstoff mindestens eines der Produkte der Formel (I_{B}) wie in Anspruch 1 definiert unter allen möglichen isomeren Formen, racemisch, enantiomer und diastereoisomer, oder mindestens eines der Additionssalze mit Mineral- und organischen Säuren oder mit Mineral- und organischen Basen, die pharmazeutisch annehmbar sind, dieser Produkte der Formel (I_{B}) unter einer Form, die für diesen Verwendungszweck geeignet ist, verwendet.

5. Verfahren zur Herstellung von pharmazeutischen Zusammensetzungen, dadurch gekennzeichnet, daß man als Wirkstoff mindestens eines der Produkte der Formel (Iₐ), wie in einem der Ansprüche 2 bis 3 definiert, unter allen möglichen isomeren Formen, racemisch, enantiomer und diastereoisomer, oder mindestens eines der Additionssalze mit Mineral- und organischen Säuren oder mit Mineral- und organischen Basen, die pharmazeutisch annehmbar sind, dieser Produkte der Formel (Iₐ) in einer für diesen Verwendungszweck geeigneten Form einsetzt.

6. Als neue industrielle Produkte die Verbindungen der Formeln (VIII), (II_{b}), (II_{c}), (II_{d}), (IIₑ) und (II_{f}), wie in Anspruch 1 definiert, mit Ausnahme der folgenden Verbindungen:
a) die Verbindungen der Formel (VIII), worin R_{2'B} und R_{3'B} = H, R_{4'B} = CO₂Et, Me, Et, CF₃, H, und R_{4''B} = CO₂Et ist;
sowie die Verbindung:
Ethyl-2-[1-[2-(trifluoromethyl)-phenylamino]-pent-1-yliden]propandioat;
b) die Verbindungen der Formel (II_{b}), worin R_{4'B} = H, Hal oder Alkyl mit 1 bis 4 Kohlenstoffatomen, und R_{4''B} = CF₃ ist;
sowie die Verbindung:
Ethyl-2-butyl-4-hydroxy-8-trifluoromethyl-3-chinoleincarboxylat;
c) die Verbindungen der Formel (II_{c}), worin R'_{2B} und R'_{3B} das eine Wasserstoff und das andere Phenyl bedeuten und R_{4'''B} freies oder verestertes Carboxy ist;
d) die Verbindung der Formel (IIₑ):
4-Butyl-1-phthalazinon;
e) die Verbindungen der Formel (II_{f}), worin R_{2'B} und R_{3'B} Wasserstoff, Hal, CF₃ oder Alkyl bedeuten, und R'_{4B} Alkyl oder CF₃ darstellt.
